# EUROPEAN PATENT SPECIFICATION

(11) **EP 3 507 298 B1**
(45) Date of publication and mention of the grant of the patent: **30.08.2023**
(21) Application number: 17847642.0
(22) Date of filing: 01.09.2017
(51) Int. Cl.: C12Q 1/6886

(54) **DETECTING HEPATOCELLULAR CARCINOMA**
ERKENNUNG EINES LEBERZELLKARZINOMS
DÉTECTION D'UN CARCINOME HÉPATOCELLULAIRE

(30) Priority: 02.09.2016 US 201662383165 P
(43) Date of publication of application: 10.07.2019
(73) Proprietor: Mayo Foundation for Medical Education and Research, Rochester, MN 55905 (US); Exact Sciences Corporation, Madison, Wisconsin 53719 (US)
(72) Inventor: TAYLOR, William R., Rochester, Minnesota 55905 (US); AHLQUIST, David A., Rochester, Minnesota 55905 (US); MAHONEY, Douglas W., Rochester, Minnesota 55905 (US); KISIEL, John B., Rochester, Minnesota 55905 (US); YAB, Tracy C., Rochester, Minnesota 55905 (US); ALLAWI, Hatim T., Madison, Wisconsin 53719 (US); GIAKOUMOPOULOS, Maria, Madison, Wisconsin 53719 (US); LIDGARD, Graham P., Madison, Wisconsin 53719 (US)
(74) Representative: Forresters IP LLP
(86) International application number: PCT/US2017/049915
(87) International publication number: WO 2018/045322

(56) References cited:
- EP-A1- 2 899 274
- WO-A1-2016/097120
- CN-A- 104 762 301
- US-A1- 2003 143 606
- US-A1- 2008 039 413
- US-A1- 2011 256 538
- US-A1- 2011 287 968
- US-A1- 2014 162 894
- US-A1- 2014 358 448
- US-A1- 2016 090 634
- BRADLEY W ANDERSON ET AL: "Aberrant Methylation During Gastric Carcinogenesis: Patterns of Acquisition Using Novel Methylated DNA Markers From Whole Methylome Discovery", GASTROENTEROLOGY, vol. 150, no. 4, 1 April 2016 (2016-04-01) , pages S863-S864, XP055737623, DOI: https://doi.org/10.1016/S0016-5085(16)3290 9-2
- SOHACHI NANJO ET AL: "Identification of gastric cancer risk markers that are informative in individuals with past.infection", GASTRIC CANCER, SPRINGER-VERLAG, TO, vol. 15, no. 4, 12 January 2012 (2012-01-12), pages 382-388, XP035128377, ISSN: 1436-3305, DOI: 10.1007/S10120-011-0126-1
- QIU XUEPING ET AL: "Hypermethylation ofACP1,BMP4, andTSPYL5in Hepatocellular Carcinoma and Their Potential Clinical Significance", DIGESTIVE DISEASES AND SCIENCES, SPRINGER NEW YORK LLC, US, vol. 61, no. 1, 19 September 2015 (2015-09-19), pages 149-157, XP035905801, ISSN: 0163-2116, DOI: 10.1007/S10620-015-3878-3 [retrieved on 2015-09-19]
- WENQING HUANG ET AL: "Analysis of DNA Methylation in Plasma for Monitoring Hepatocarcinogenesis", GENETIC TESTING AND MOLECULAR BIOMARKERS, vol. 19, no. 6, 1 June 2015 (2015-06-01), pages 295-302, XP055737817, United States ISSN: 1945-0265, DOI: 10.1089/gtmb.2014.0292
- JING SHEN ET AL: "Integrative epigenomic and genomic filtering for methylation markers in hepatocellular carcinomas", BMC MEDICAL GENOMICS, vol. 8, no. 1, 10 June 2015 (2015-06-10), XP055770789, DOI: 10.1186/s12920-015-0105-1
- TIAN-ZHOU ZHA ET AL: "Overexpression of HOXA1 correlates with poor prognosis in patients with hepatocellular carcinoma", TUMOR BIOLOGY, vol. 33, no. 6, 4 August 2012 (2012-08-04) , pages 2125-2134, XP055771443, CH ISSN: 1010-4283, DOI: 10.1007/s13277-012-0472-6
- TSOU JEFFREY A ET AL: "Identification of a panel of sensitive and specific DNA methylation markers for lung adenocarcinoma", MOLECULAR CANCER, BIOMED CENTRAL, LONDON, GB, vol. 6, no. 1, 29 October 2007 (2007-10-29), page 70, XP021036914, ISSN: 1476-4598
- QIU ET AL.: 'Hypermethylation of ACP1, BMP4, and TSPYL5 in Hepatocellular Carcinoma and Their Potential Clinical Significance' DIGESTIVE DISEASES AND SCIENCES vol. 61, no. 1, 19 September 2015, pages 149 - 157, XP035905801

## Description

### FIELD OF INVENTION

Provided herein is technology for hepatocellular carcinoma screening and particularly, but not exclusively, to methods, compositions, and related uses for detecting the presence of hepatocellular carcinoma.

### BACKGROUND

Hepatocellular carcinoma (HCC) is a primary malignancy of the liver and occurs predominantly in patients with underlying chronic liver disease and cirrhosis. The cell(s) of origin are believed to be the hepatic stem cells (see, Alison MR. Stem Cell Rev. 2005. 1(3):253-60). Tumors progress with local expansion, intrahepatic spread, and distant metastases.

HCC is now the third leading cause of cancer deaths worldwide, with over 500,000 people affected. The incidence of HCC is highest in Asia and Africa, where the endemic high prevalence of hepatitis B and hepatitis C strongly predisposes to the development of chronic liver disease and subsequent development of HCC.

The presentation of HCC has evolved significantly over the past few decades. Whereas in the past, HCC generally presented at an advanced stage with right-upper-quadrant pain, weight loss, and signs of decompensated liver disease, it is now increasingly recognized at a much earlier stage as a consequence of the routine screening of patients with known cirrhosis, using cross-sectional imaging studies and serum alpha-fetoprotein (AFP) measurements.

US 2011/256538 A1 relates to a method for detecting the presence or absence of a cancer in an individual, by determining the level of methylation of the sense strand of a selected regulatory region of the tumor suppressor gene adenomatous polyposis coli (APC). Hypermethylation has been described in the HOXA1 gene in cholangiocarcinoma (US 2016/090634 A1) and adenocarcinoma (Tsou et al., Molecular Cancer, Biomed Central, vol. 6, no. 1, 29 October 2007, pages 70).

The threat of HCC is expected to continue to grow in the coming years (see, Llovet JM, et al., Liver Transpl. 2004 Feb. 10(2 Suppl 1):S115-20). Accordingly, there is a great need for early detection of HCC to improve the survival rate of these patients.

### SUMMARY

Hepatocellular carcinoma (HCC) is the 2nd most fatal cancer worldwide. Survival improves with early stage detection, and accurate noninvasive screening tools are needed. There is an imperative for innovation that will deliver accurate, affordable, and safe screening tools for the pre-symptomatic detection of the earliest stage of HCC.

The present invention addresses this need. Indeed, the present invention provides novel methylated DNA markers that discriminate HCC from normal controls (controls with and without cirrhosis).

Methylated DNA has been studied as a potential class of biomarkers in the tissues of most tumor types. In many instances, DNA methyltransferases add a methyl group to DNA at cytosine-phosphate-guanine (CpG) island sites as an epigenetic control of gene expression. In a biologically attractive mechanism, acquired methylation events in promoter regions of tumor suppressor genes are thought to silence expression, thus contributing to oncogenesis. DNA methylation may be a more chemically and biologically stable diagnostic tool than RNA or protein expression (Laird (2010) Nat Rev Genet 11: 191-203). Furthermore, in other cancers like sporadic colon cancer, methylation markers offer excellent specificity and are more broadly informative and sensitive than are individual DNA mutations (see, Zou et al (2007) Cancer Epidemiol Biomarkers Prev 16: 2686-96).

Analysis of CpG islands has yielded important findings when applied to animal models and human cell lines. For example, Zhang and colleagues found that amplicons from different parts of the same CpG island may have different levels of methylation (Zhang et al. (2009) PLoS Genet 5: e1000438). Further, methylation levels were distributed bi-modally between highly methylated and unmethylated sequences, further supporting the binary switch-like pattern of DNA methyltransferase activity (Zhang et al. (2009) PLoS Genet 5: e1000438). Analysis of murine tissues in vivo and cell lines in vitro demonstrated that only about 0.3% of high CpG density promoters (HCP, defined as having >7% CpG sequence within a 300 base pair region) were methylated, whereas areas of low CpG density (defined as having <5% CpG sequence within a 300 base pair region) tended to be frequently methylated in a dynamic tissue-specific pattern (Meissner et al. (2008) Nature 454: 766-70). HCPs include promoters for ubiquitous housekeeping genes and highly regulated developmental genes. Among the HCP sites methylated at >50% were several established markers such as Wnt 2, NDRG2, SFRP2, and BMP3 (Meissner et al. (2008) Nature 454: 766-70).

Experiments conducted during the course of developing embodiments for the present invention compared the methylation state of DNA markers from plasma of subjects having HCC to the methylation state of the same DNA markers from control subjects (e.g., subjects having cirrhosis or normal). Such experiments identified and validated methylated DNA marker candidates that discriminate HCC from such control groups.

Accordingly, disclosed herein is technology for HCC screening (e.g., surveilling) and particularly, but not exclusively, to methods, compositions, and related uses for detecting the presence of HCC.

Markers and/or panels of markers were identified capable of detecting HCC (see, Examples I, II and III) (ACP1, BDH1, Chr12.133, CLEC11A, DAB2IP, DBNL, EMX1, EFNB2, HOXA1, LRRC4, SPINT2, TSPYL5, CCNJ 3707, CCNJ 3124, PFKP, SCRN1, and ECE1).

Therefore the invention describes a method for characterizing a biological sample comprising: measuring a methylation level of a CpG site for the gene HOXA1 and one or more genes selected from either EMX1, DAB2IP, TSPYL5, ACP1, BDH1, Chr12.133, CLEC11A, DBNL, EFNB2, LRRC4, SPINT2, CCNJ 3707, CCNJ 3124, PFKP, SCRN1, and ECE1 in a biological sample of a human individual through treating genomic DNA in the biological sample with bisulfite; amplifying the bisulfite-treated genomic DNA using a set of primers for the genes; and determining the methylation level of the CpG site by methylation-specific PCR, quantitative methylation-specific PCR, methylation-sensitive DNA restriction enzyme analysis, quantitative bisulfite pyrosequencing, or bisulfite genomic sequencing PCR, and comparing the methylation level to a methylation level of a corresponding set of genes in control samples without HCC; and determining that the individual has HCC when the methylation level measured in the genes is higher than the methylation level measured in the respective control samples.

As disclosed herein, the technology provides a number of methylated DNA markers and subsets thereof (e.g., sets of 2, 3, 4, 5, 6, 7, 8, 9, 10, 11, 12 markers) with high discrimination for detecting the presence of HCC in subjects. Experiments applied a selection filter to candidate markers to identify markers that provide a high signal to noise ratio and a low background level to provide high specificity, e.g., when assaying media (e.g., plasma) for purposes of screening or diagnosis (e.g., HCC screening or diagnosis).

In some aspects of the disclosure, the technology is related to assessing the presence of and methylation state of one or more of the markers identified herein in a biological sample (e.g., a plasma sample). These markers comprise one or more differentially methylated regions (DMR) as discussed herein, e.g., as provided in Tables 1 and 4. Methylation state is assessed in embodiments of the technology. As such, the technology provided herein is not restricted in the method by which a gene's methylation state is measured. For example, in some aspects the methylation state is measured by a genome scanning method. For example, one method involves restriction landmark genomic scanning (see, Kawai et al. (1994) Mol. Cell. Biol. 14: 7421-7427) and another example involves methylation-sensitive arbitrarily primed PCR (see, Gonzalgo et al. (1997) Cancer Res. 57: 594-599). In some aspects, changes in methylation patterns at specific CpG sites are monitored by digestion of genomic DNA with methylation-sensitive restriction enzymes followed by Southern analysis of the regions of interest (digestion-Southern method). In some embodiments of the invention, analyzing changes in methylation patterns involves a PCR-based process that involves digestion of genomic DNA with methylation-sensitive restriction enzymes prior to PCR amplification (see, Singer-Sam et al. (1990) Nucl. Acids Res. 18: 687). In addition, other techniques have been reported that utilize bisulfite treatment of DNA as a starting point for methylation analysis. These include methylation-specific PCR (MSP) (see, Herman et al. (1992) Proc. Natl. Acad. Sci. USA 93: 9821-9826) and restriction enzyme digestion of PCR products amplified from bisulfite-converted DNA (see, Sadri and Hornsby (1996) Nucl. Acids Res. 24: 5058-5059; and Xiong and Laird (1997) Nucl. Acids Res. 25: 2532-2534). PCR techniques have been developed for detection of gene mutations (see, Kuppuswamy et al. (1991) Proc. Natl. Acad. Sci. USA 88: 1143-1147) and quantification of allelic-specific expression (Szabo and Mann (1995) Genes Dev. 9: 3097-3108; and Singer-Sam et al. (1992) PCR Methods Appl. 1: 160-163). Such techniques use internal primers, which anneal to a PCR-generated template and terminate immediately 5' of the single nucleotide to be assayed. Methods using a "quantitative Ms-SNuPE assay" as described in U.S. Pat. No. 7,037,650 are used in some embodiments.

Upon evaluating a methylation state, the methylation state is often expressed as the fraction or percentage of individual strands of DNA that is methylated at a particular site (e.g., at a single nucleotide, at a particular region or locus, at a longer sequence of interest, e.g., up to a ~100-bp, 200-bp, 500-bp, 1000-bp subsequence of a DNA or longer) relative to the total population of DNA in the sample comprising that particular site. Traditionally, the amount of the unmethylated nucleic acid is determined by PCR using calibrators. Then, a known amount of DNA is bisulfite treated and the resulting methylation-specific sequence is determined using either a real-time PCR or other exponential amplification, e.g., a QuARTS assay (e.g., as provided by U.S. Pat. Nos. 8,361,720, 8,715,937, and 8,916,344).

For example, in some embodiments methods comprise generating a standard curve for the unmethylated target by using external standards. The standard curve is constructed from at least two points and relates the real-time Ct value for unmethylated DNA to known quantitative standards. Then, a second standard curve for the methylated target is constructed from at least two points and external standards. This second standard curve relates the Ct for methylated DNA to known quantitative standards. Next, the test sample Ct values are determined for the methylated and unmethylated populations and the genomic equivalents of DNA are calculated from the standard curves produced by the first two steps. The percentage of methylation at the site of interest is calculated from the amount of methylated DNAs relative to the total amount of DNAs in the population, e.g., (number of methylated DNAs) / (the number of methylated DNAs + number of unmethylated DNAs) × 100.

Also disclosed herein are compositions and kits for practicing the methods. For example, in some aspects, reagents (e.g., primers, probes) specific for one or more markers are provided alone or in sets (e.g., sets of primers pairs for amplifying a plurality of markers). Additional reagents for conducting a detection assay may also be provided (e.g., enzymes, buffers, positive and negative controls for conducting QuARTS, PCR, sequencing, bisulfite, or other assays). In some aspects, the kits containing one or more reagent necessary, sufficient, or useful for conducting a method are provided. Also provided are reactions mixtures containing the reagents. Further provided are master mix reagent sets containing a plurality of reagents that may be added to each other and/or to a test sample to complete a reaction mixture.

In some aspects, the technology described herein is associated with a programmable machine designed to perform a sequence of arithmetic or logical operations as provided by the methods described herein. For example, some aspects of the technology are associated with (e.g., implemented in) computer software and/or computer hardware. In one aspect, the technology relates to a computer comprising a form of memory, an element for performing arithmetic and logical operations, and a processing element (e.g., a microprocessor) for executing a series of instructions (e.g., a method as provided herein) to read, manipulate, and store data. In some aspects, a microprocessor is part of a system for determining a methylation state (e.g., of one or more DMR, e.g., DMR 1-400 as provided in Tables 1 and 4); comparing methylation states (e.g., of one or more DMR, e.g., DMR 1-400 as provided in Tables 1 and 4); generating standard curves; determining a Ct value; calculating a fraction, frequency, or percentage of methylation (e.g., of one or more DMR, e.g., DMR 1-400 as provided in Tables 1 and 4); identifying a CpG island; determining a specificity and/or sensitivity of an assay or marker; calculating an ROC curve and an associated AUC; sequence analysis; all as described herein or is known in the art.

In some aspects, a microprocessor or computer uses methylation state data in an algorithm to predict a site of a cancer.

In some aspects, a software or hardware component receives the results of multiple assays and determines a single value result to report to a user that indicates a cancer risk based on the results of the multiple assays (e.g., determining the methylation state of multiple DMR, e.g., as provided in Tables 1 and 4). Related aspects calculate a risk factor based on a mathematical combination (e.g., a weighted combination, a linear combination) of the results from multiple assays, e.g., determining the methylation states of multiple markers (such as multiple DMR, e.g., as provided in Tables 1 and 4). In some aspects, the methylation state of a DMR defines a dimension and may have values in a multidimensional space and the coordinate defined by the methylation states of multiple DMR is a result, e.g., to report to a user.

Some aspects comprise a storage medium and memory components. Memory components (e.g., volatile and/or nonvolatile memory) find use in storing instructions (e.g., an aspect of a process as provided herein) and/or data (e.g., a work piece such as methylation measurements, sequences, and statistical descriptions associated therewith). Some aspects relate to systems also comprising one or more of a CPU, a graphics card, and a user interface (e.g., comprising an output device such as display and an input device such as a keyboard).

Programmable machines associated with the technology comprise conventional extant technologies and technologies in development or yet to be developed (e.g., a quantum computer, a chemical computer, a DNA computer, an optical computer, a spintronics based computer, etc.).

In some aspects, the technology comprises a wired (e.g., metallic cable, fiber optic) or wireless transmission medium for transmitting data. For example, some aspects relate to data transmission over a network (e.g., a local area network (LAN), a wide area network (WAN), an ad-hoc network, the internet, etc.). In some aspects, programmable machines are present on such a network as peers and in some aspects the programmable machines have a client/server relationship.

In some aspects, data are stored on a computer-readable storage medium such as a hard disk, flash memory, optical media, a floppy disk, etc.

In some aspects, the technology provided herein is associated with a plurality of programmable devices that operate in concert to perform a method as described herein. For example, in some aspects, a plurality of computers (e.g., connected by a network) may work in parallel to collect and process data, e.g., in an implementation of cluster computing or grid computing or some other distributed computer architecture that relies on complete computers (with onboard CPUs, storage, power supplies, network interfaces, etc.) connected to a network (private, public, or the internet) by a conventional network interface, such as Ethernet, fiber optic, or by a wireless network technology.

For example, some aspects provide a computer that includes a computer-readable medium. The aspect includes a random access memory (RAM) coupled to a processor. The processor executes computer-executable program instructions stored in memory. Such processors may include a microprocessor, an ASIC, a state machine, or other processor, and can be any of a number of computer processors, such as processors from Intel Corporation of Santa Clara, California and Motorola Corporation of Schaumburg, Illinois. Such processors include, or may be in communication with, media, for example computer-readable media, which stores instructions that, when executed by the processor, cause the processor to perform the steps described herein.

Aspects of computer-readable media include, but are not limited to, an electronic, optical, magnetic, or other storage or transmission device capable of providing a processor with computer-readable instructions. Other examples of suitable media include, but are not limited to, a floppy disk, CD-ROM, DVD, magnetic disk, memory chip, ROM, RAM, an ASIC, a configured processor, all optical media, all magnetic tape or other magnetic media, or any other medium from which a computer processor can read instructions. Also, various other forms of computer-readable media may transmit or carry instructions to a computer, including a router, private or public network, or other transmission device or channel, both wired and wireless. The instructions may comprise code from any suitable computerprogramming language, including, for example, C, C++, C#, Visual Basic, Java, Python, Perl, and JavaScript.

Computers are connected in some aspects to a network. Computers may also include a number of external or internal devices such as a mouse, a CD-ROM, DVD, a keyboard, a display, or other input or output devices. Examples of computers are personal computers, digital assistants, personal digital assistants, cellular phones, mobile phones, smart phones, pagers, digital tablets, laptop computers, internet appliances, and other processor-based devices. In general, the computers related to aspects of the technology provided herein may be any type of processor-based platform that operates on any operating system, such as Microsoft Windows, Linux, UNIX, Mac OS X, etc., capable of supporting one or more programs comprising the technology provided herein. Some aspects comprise a personal computer executing other application programs (e.g., applications). The applications can be contained in memory and can include, for example, a word processing application, a spreadsheet application, an email application, an instant messenger application, a presentation application, an Internet browser application, a calendar/organizer application, and any other application capable of being executed by a client device.

All such components, computers, and systems described herein as associated with the technology may be logical or virtual.

Provided herein is technology related to a method of screening for HCC in a sample obtained from a subject, the method comprising assaying a methylation state of a marker in a sample obtained from a subject; and identifying the subject as having HCC when the methylation state of the marker is different than a methylation state of the marker assayed in a subject that does not have HCC (e.g., a subject that does not have HCC) (e.g., a subject that does not have HCC but does have liver cirrhosis), wherein the marker comprises one or more bases in a differentially methylated region (DMR) selected from ACP1, BDH1, Chr12.133, CLEC11A, DAB2IP, DBNL, EMX1, EFNB2, HOXA1, LRRC4, SPINT2, TSPYL5, CCNJ_3707, CCNJ_3124, PFKP, SCRN1, and ECE1 as provided in Tables 1 and 4.

The technology is not limited in the methylation state assessed. In some embodiments assessing the methylation state of the marker in the sample comprises determining the methylation state of one base. In some embodiments, assaying the methylation state of the marker in the sample comprises determining the extent of methylation at a plurality of bases. Moreover, in some embodiments the methylation state of the marker comprises an increased methylation of the marker relative to a normal methylation state of the marker. In some embodiments, the methylation state of the marker comprises a decreased methylation of the marker relative to a normal methylation state of the marker. In some embodiments the methylation state of the marker comprises a different pattern of methylation of the marker relative to a normal methylation state of the marker.

Furthermore, in some embodiments the marker is a region of 100 or fewer bases, the marker is a region of 500 or fewer bases, the marker is a region of 1000 or fewer bases, the marker is a region of 5000 or fewer bases, or, in some embodiments, the marker is one base. In some embodiments the marker is in a high CpG density promoter.

The technology is not limited by sample type. For example, in some aspects of the disclosure the sample is a blood sample (e.g., plasma, serum, whole blood), a stool sample, a tissue sample (e.g., stomach tissue, pancreatic tissue, bile duct / liver tissue, pancreatic juice, and colorectal tissue), an excretion, or a urine sample.

Furthermore, the technology is not limited in the method used to determine methylation state. In some aspects the assaying comprises using methylation specific polymerase chain reaction, nucleic acid sequencing, mass spectrometry, methylation specific nuclease, mass-based separation, or target capture. In some aspects, the assaying comprises use of a methylation specific oligonucleotide. In some aspects, the technology uses massively parallel sequencing (e.g., next-generation sequencing) to determine methylation state, e.g., sequencing-by-synthesis, real-time (e.g., single-molecule) sequencing, bead emulsion sequencing, nanopore sequencing, etc.

The technology provides reagents for detecting a DMR, e.g., in some aspects are provided a set of oligonucleotides comprising the sequences provided by SEQ ID NO: 1-94 (Tables 2 and 5). In some aspects are provided an oligonucleotide comprising a sequence complementary to a chromosomal region having a base in a DMR, e.g., an oligonucleotide sensitive to methylation state of a DMR.

The technology provides various panels of markers, e.g., in some aspects the marker comprises a chromosomal region having an annotation that is provided in Tables 1 or 3, and that comprises the marker. In addition, aspects provide a method of analyzing a DMR from Tables 1 and/or 4 that one or more of DMR Nos. 1-400.

Kit aspects are provided in the disclosure, e.g., a kit comprising a bisulfite reagent; and a control nucleic acid comprising a sequence from a DMR selected from a group consisting of DMR 1-400 (from Tables 1 and 4) and having a methylation state associated with a subject who does not have HCC (e.g., a subject that does not have HCC and does not have liver cirrhosis) (e.g., a subject that does not have HCC but does have liver cirrhosis). Kit aspects are provided, e.g., a kit comprising a bisulfite reagent; and a control nucleic acid comprising a sequence from a DMR selected from a group consisting of DMR 1-400 (from Tables 1 and 4) and having a methylation state associated with a subject who does not have HCC.

Some kit aspects comprise a sample collector for obtaining a sample from a subject (e.g., a stool sample); reagents for isolating a nucleic acid from the sample; a bisulfite reagent; and an oligonucleotide as described herein.

The technology is related to aspects of compositions (e.g., reaction mixtures). In some aspects are provided a composition comprising a nucleic acid comprising a DMR and a bisulfite reagent. Some aspects provide a composition comprising a nucleic acid comprising a DMR and an oligonucleotide as described herein. Some aspects provide a composition comprising a nucleic acid comprising a DMR and a methylation-sensitive restriction enzyme. Some aspects provide a composition comprising a nucleic acid comprising a DMR and a polymerase.

Additional related method embodiments are provided for screening for HCC in a sample obtained from a subject (e.g., a plasma sample), e.g., a method comprising determining a methylation state of a marker in the sample comprising a base in a DMR that is one or more of DMR 1-400 (from Tables 1 and 4); comparing the methylation state of the marker from the subject sample to a methylation state of the marker from a normal control sample from a subject who does not have HCC; and determining a confidence interval and/or a p value of the difference in the methylation state of the subject sample and the normal control sample.

In some embodiments, the confidence interval is 90%, 95%, 97.5%, 98%, 99%, 99.5%, 99.9% or 99.99% and the p value is 0.1, 0.05, 0.025, 0.02, 0.01, 0.005, 0.001, or 0.0001. Some embodiments of methods provide steps of reacting a nucleic acid comprising a DMR with a bisulfite reagent to produce a bisulfite-reacted nucleic acid; sequencing the bisulfite-reacted nucleic acid to provide a nucleotide sequence of the bisulfite-reacted nucleic acid; comparing the nucleotide sequence of the bisulfite-reacted nucleic acid with a nucleotide sequence of a nucleic acid comprising the DMR from a subject who does not have a cancer to identify differences in the two sequences; and identifying the subject as having a neoplasm when a difference is present.

Systems for screening for HCC in a sample obtained from a subject are provided by the technology. Exemplary embodiments of systems include, e.g., a system for screening for HCC in a sample obtained from a subject, the system comprising an analysis component configured to determine the methylation state of a sample, a software component configured to compare the methylation state of the sample with a control sample or a reference sample methylation state recorded in a database, and an alert component configured to alert a user of a HCC-associated methylation state (e.g., a methylation state for no HCC; a methylation state for HCC). An alert is determined in some aspects by a software component that receives the results from multiple assays (e.g., determining the methylation states of multiple markers, e.g., DMR, e.g., as provided in Tables 1 and 4) and calculating a value or result to report based on the multiple results. Some aspects provide a database of weighted parameters associated with each DMR provided herein for use in calculating a value or result and/or an alert to report to a user (e.g., such as a physician, nurse, clinician, etc.). In some aspects all results from multiple assays are reported and in some aspects one or more results are used to provide a score, value, or result based on a composite of one or more results from multiple assays that is indicative of a HCC risk in a subject.

In some aspects of systems, a sample comprises a nucleic acid comprising a DMR. In some aspects the system further comprises a component for isolating a nucleic acid, a component for collecting a sample such as a component for collecting a plasma sample. In some aspects, the system comprises nucleic acid sequences comprising a DMR. In some aspects the database comprises nucleic acid sequences from subjects who do not have HCC. Also provided are nucleic acids, e.g., a set of nucleic acids, each nucleic acid having a sequence comprising a DMR. In some aspects the set of nucleic acids wherein each nucleic acid has a sequence from a subject who does not have HCC. Related system aspects comprise a set of nucleic acids as described and a database of nucleic acid sequences associated with the set of nucleic acids. Some aspects further comprise a bisulfite reagent. And, some aspects further comprise a nucleic acid sequencer.

In certain embodiments, methods for detecting HCC in a sample obtained from a subject (e.g., a plasma sample) are provided, comprising a) obtaining a sample comprising DNA from a subject; b) treating the obtained DNA with a reagent which selectively modifies unmethylated cytosine residues in the obtained DNA to produce modified residues but which does not modify methylated cytosine residues; c) determining the methylation level of one or more DNA methylation markers in the DNA having undergone the treating of step b), wherein one or more DNA methylation markers comprises a base in a differentially methylated region (DMR) as provided by DMR 1-400 (from Tables 1 and 4), d) comparing the determined methylation level of the one or more DNA methylation markers with methylation level references for the one or more DNA methylation markers for subjects who do not have HCC; and e) identifying the subject as having HCC when differences are present.

In some embodiments, a determination of elevated methylation in one or more of the DNA methylation markers comprises a determination of altered methylation within a region selected from the group consisting of a CpG island and a CpG island shore.

In some embodiments, a determination of elevated methylation within the CpG island or CpG shore comprises elevated methylation within a coding region or a regulatory region of the DNA methylation marker.

In some embodiments, the determining the methylation level of one or more DNA methylation markers in the DNA having undergone the treating of step b) comprises determining the methylation score and/or the methylation frequency of the one or more DNA methylation markers. In some embodiments, the treating of step b) is accomplished through bisulfite modification of the obtained DNA.

In some embodiments, the determining the methylation level of one or more DNA methylation markers in the DNA having undergone the treating of step b) is achieved by a technique selected from the group consisting of methylation-specific PCR, quantitative methylation-specific PCR, methylation-sensitive DNA restriction enzyme analysis, quantitative bisulfite pyrosequencing, and bisulfite genomic sequencing PCR.

Additional embodiments will be apparent to persons skilled in the relevant art based on the teachings contained herein.

### BRIEF DESCRIPTION OF THE DRAWINGS

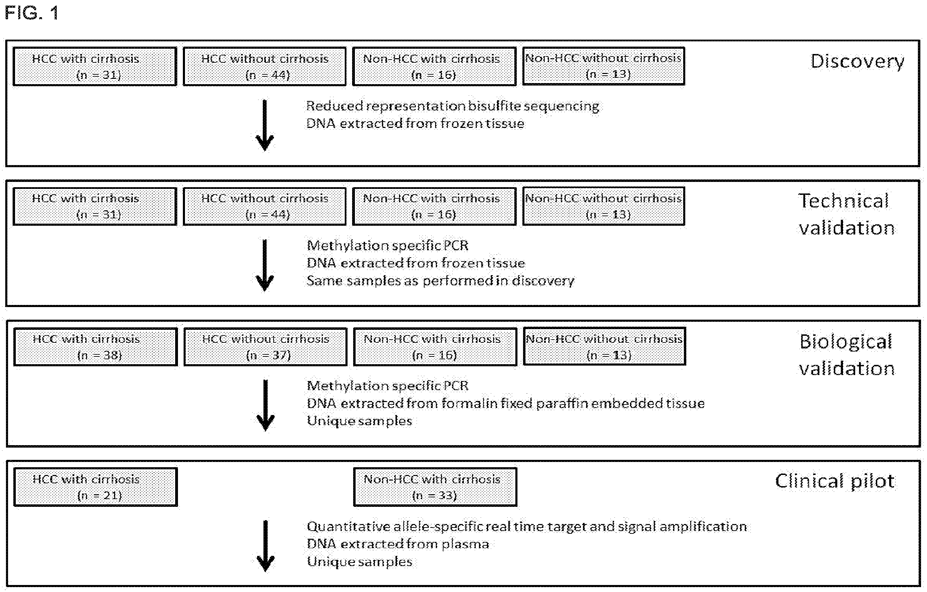

FIG. 1: A depiction of the four phases involved in the identification of 311 differentially methylated regions (DMRs) for discriminating HCC DNA samples from DNA derived from normal controls (e.g., non-HCC individuals with or without liver cirrhosis).

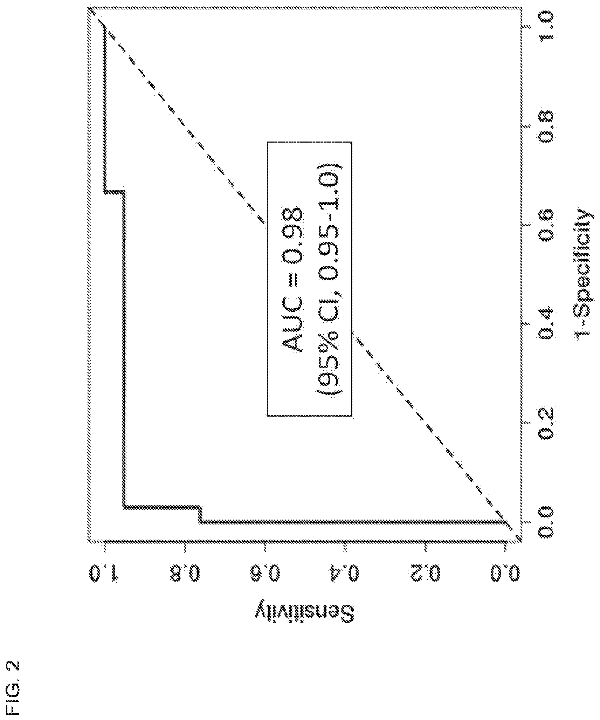

FIG. 2: A complementary 3-marker combination (EMX1, LRRC4 and BDH1) identified 20/21 HCC and 32/33 controls in plasma; 1 HCC had low levels of BDH1 and 1 control had elevated LRRC4. This panel was 95% sensitive (95% CI, 74%-100%) for HCC at 97% specificity (95% CI, 82%-100%) and achieved an AUC of 0.98 (see, Fig. 2).

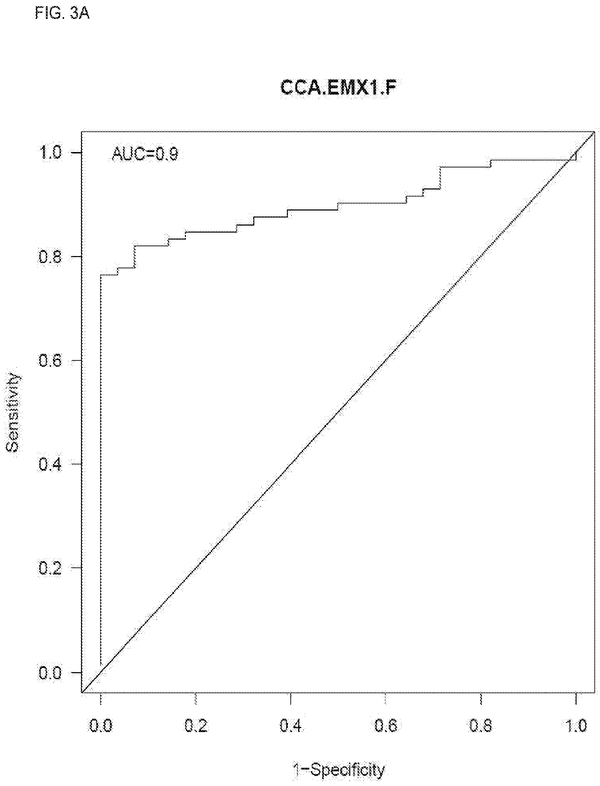

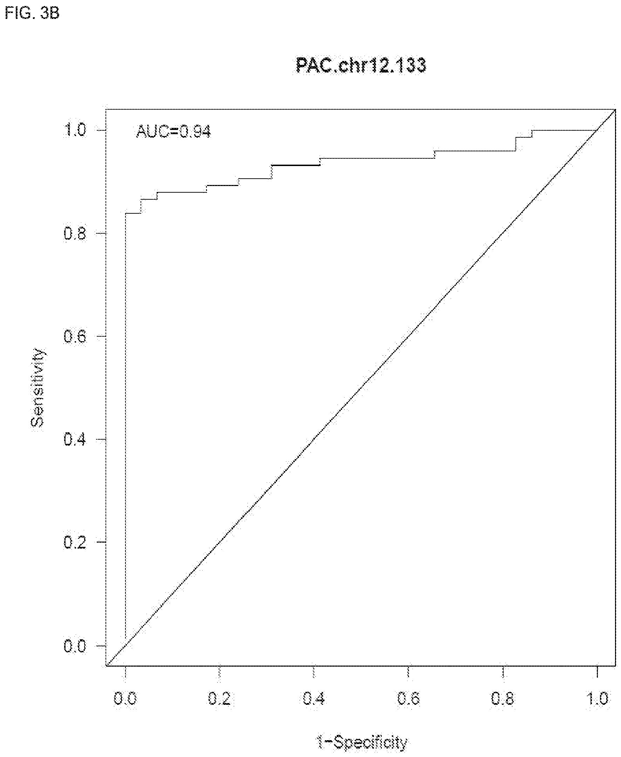

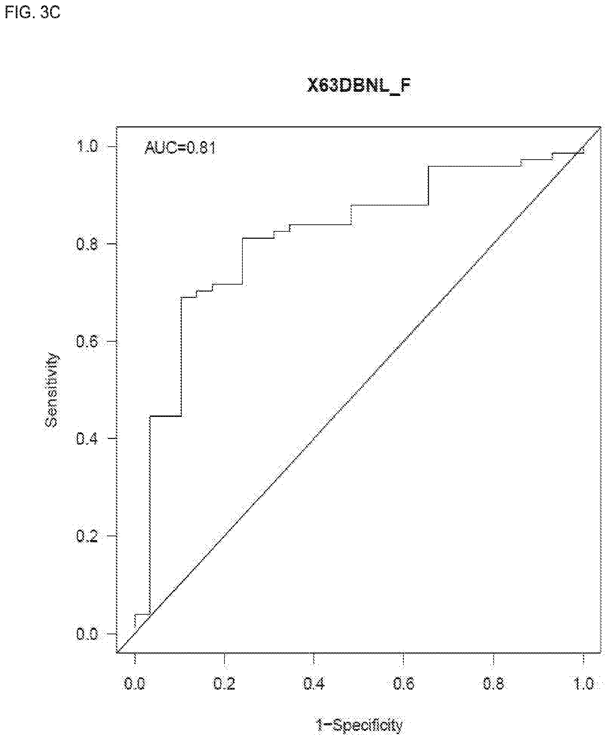

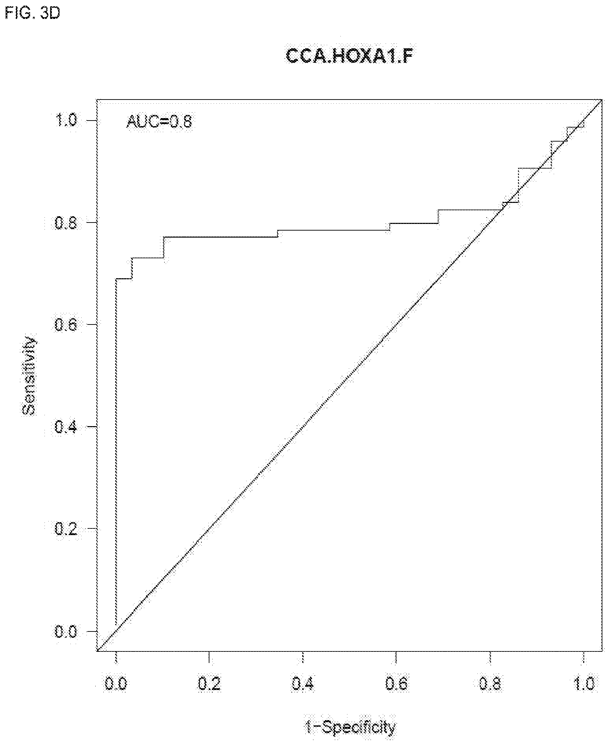

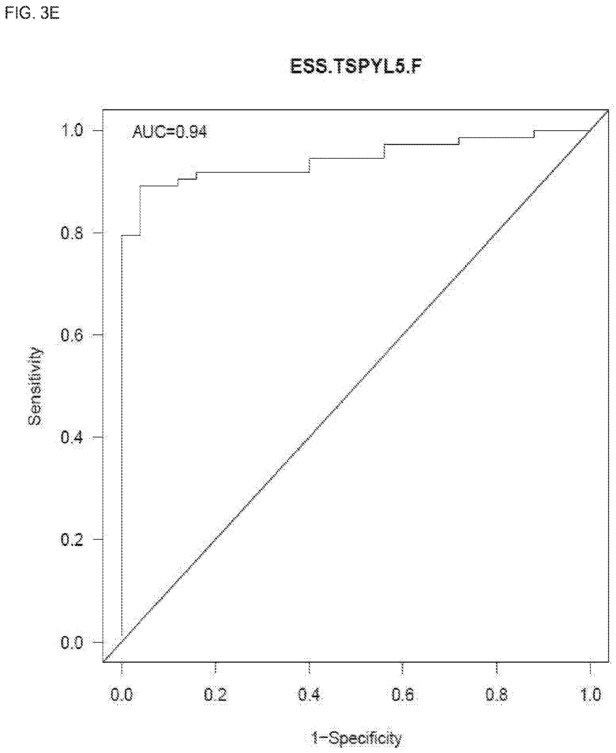

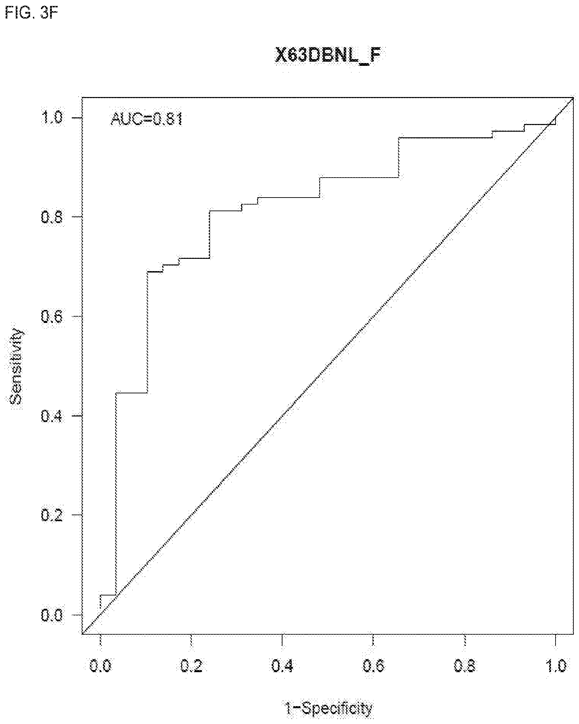

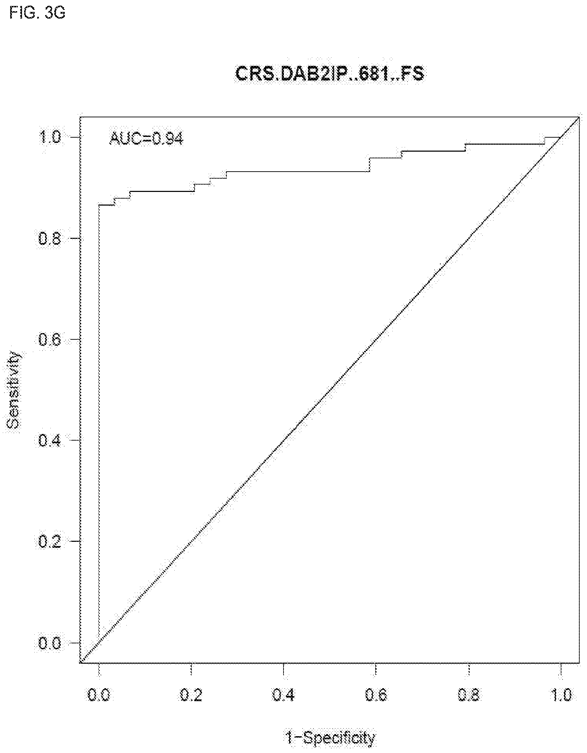

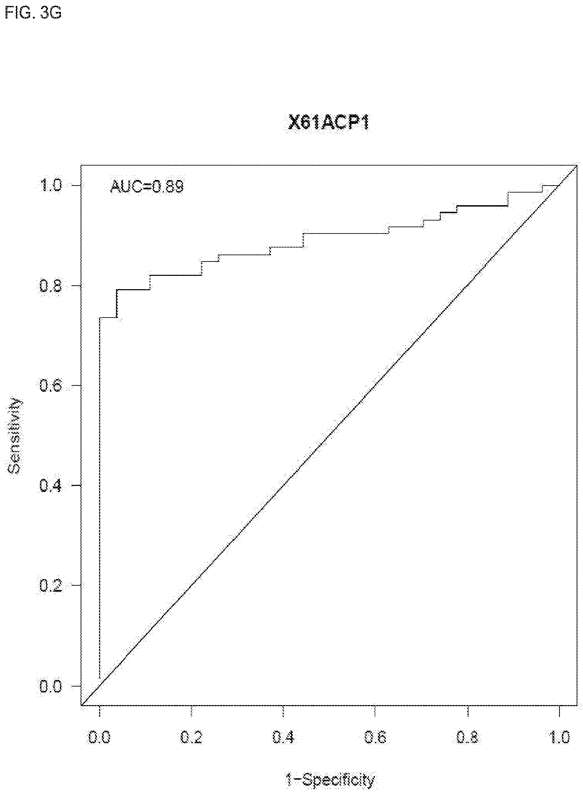

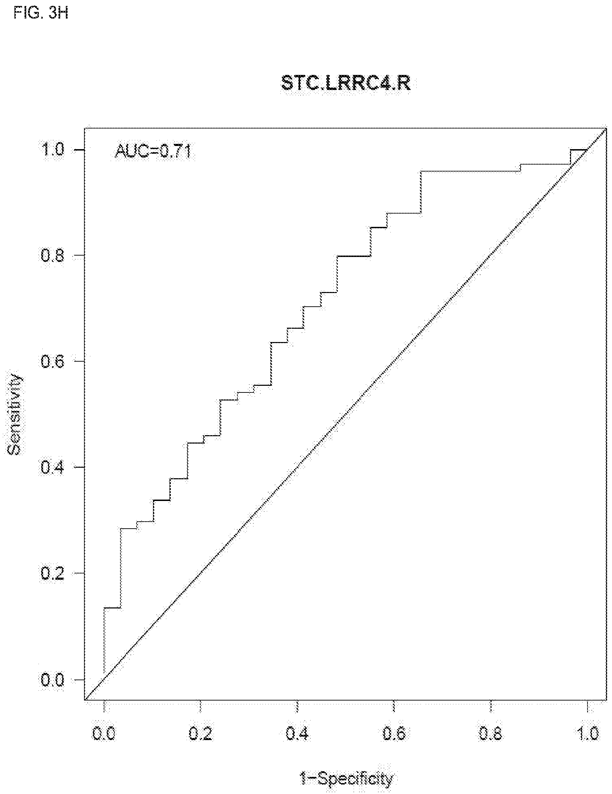

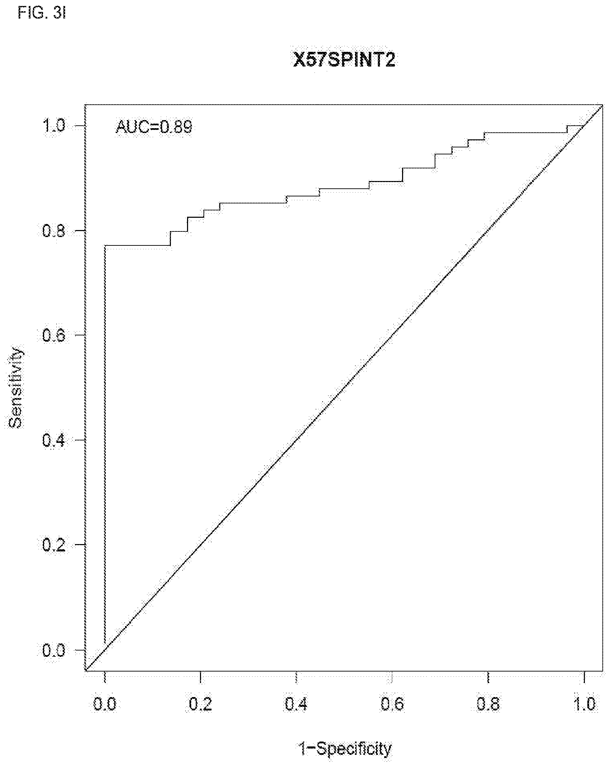

FIG. 3A-I: Area under the receiver operating characteristic curve information for ACP1, Chr12.133, CLEC11A, DAB2IP, DBNL, EMX1, HOXA1, LRRC4, SPINT2, and TSPYL5.

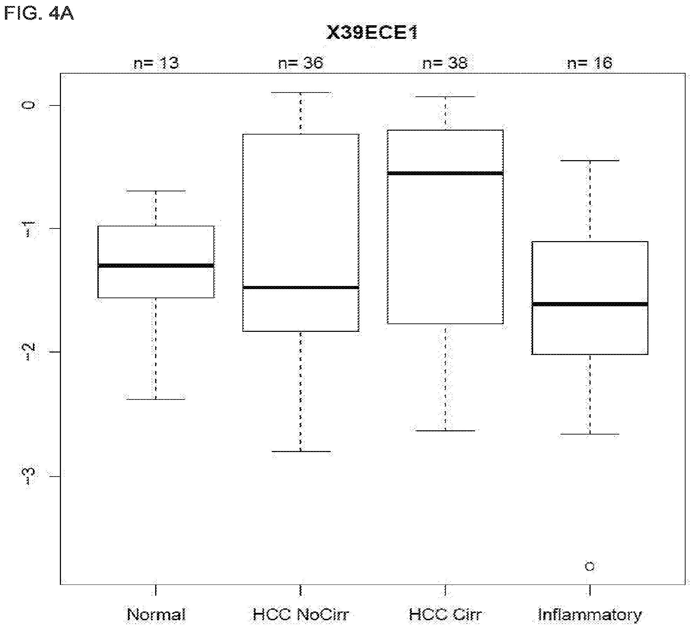

FIG. 4A-CC: Box plots (log scale) of 27 gastric cancer markers (29 with fit point analyses added) from biological tissue validation data. Samples are arranged with normal liver on the far left, followed by HCC w/o cirrhosis, HCC w/ cirrhosis, and cirrhosis controls (Inflammatory). The vertical axis is fractional methylation (normalized to β-actin strands).

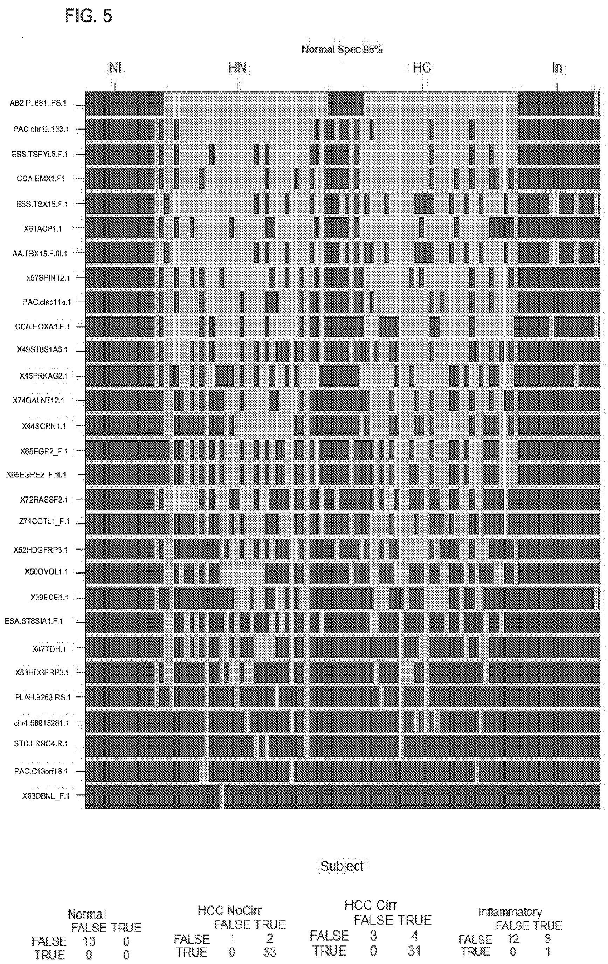

FIG. 5: Performance of 27 HCC cancer markers in 75 HCC tissue samples and 29 controls (16 cirrhosis, 13 normal liver) in a matrix format at 95% normal specificity. Markers are listed vertically and samples horizontally. Samples are arranged with normal liver (NI) on the far left, followed by HCC w/o cirrhosis (HN), HCC w/ cirrhosis (HC), and cirrhosis controls (In). Positive hits are in light grey and misses in dark grey. This plot allows markers to be assessed in a complementary fashion. Note: 2 markers, TBX15 and EGR2, were analyzed a second time using a fit points method on the qMSP data and are included here.

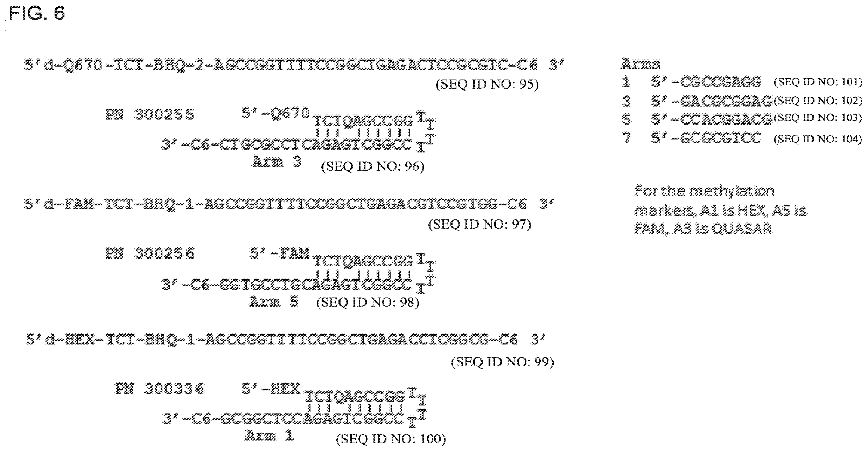

FIG. 6 provides the oligonucleotide sequences for the FRET cassettes, used in the detection of methylated DNA signatures by QuARTs (quantitative allele-specific real-time target and signal amplification) assay. Each FRET sequence includes a fluorophore and quencher which can be multiplexed together into 3 separate assays.

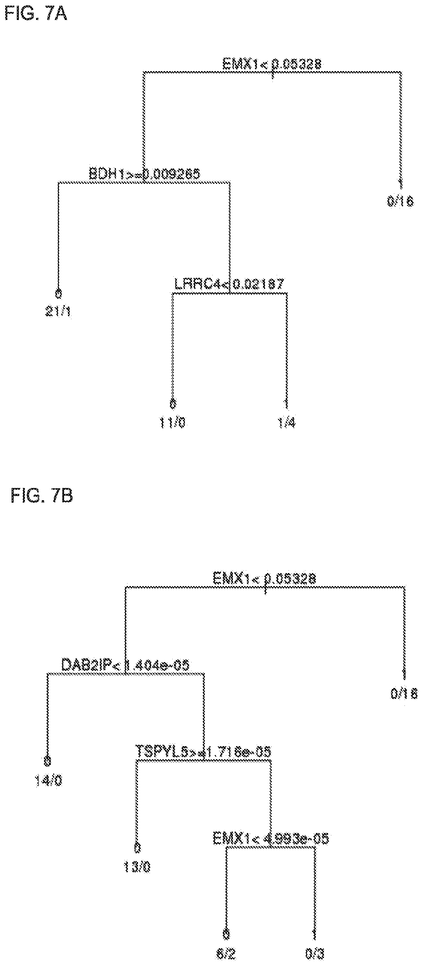

FIG. 7A-D: A) rPart binary tree with cut-offs for top 3 marker combination (EMX1, BDH1, LRRC4). The tree is built by the following process: first the single variable (marker) is found which best splits the data into two groups. The data is separated, and then this process is applied separately to each subgroup, and so on recursively until the subgroups either reach a minimum size or until no further improvement can be made. Control sample numbers which meet or do not meet cut-offs are represented in the numerator position and case samples in the denominator position. Here, the combination identified 20 of 21 HCC and 32 of 33 controls in plasma. B) rPart binary tree with cut-offs for top 3 marker combination (EMX1, DAB2IP, TSPYL5). C) rPart binary tree with cut-offs for top 3 marker combination (EMX1, HOXA1, ACP1). D) rPart binary tree with cut-offs for top 3 marker combination (EMX1, EFNB2, SPINT2).

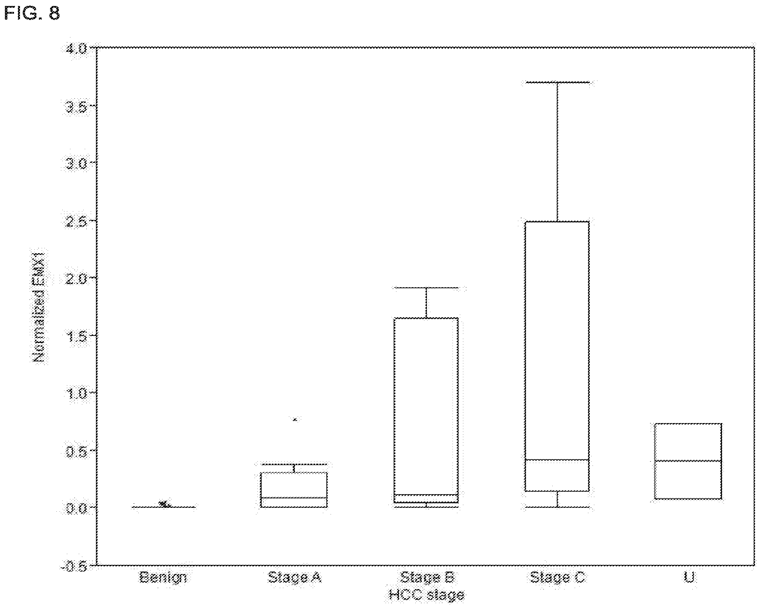

FIG. 8: Bar chart demonstrating HCC sensitivity in plasma by stage A-C at 100% specificity using methylation marker EMX1. Samples classified as U were undefined for stage.

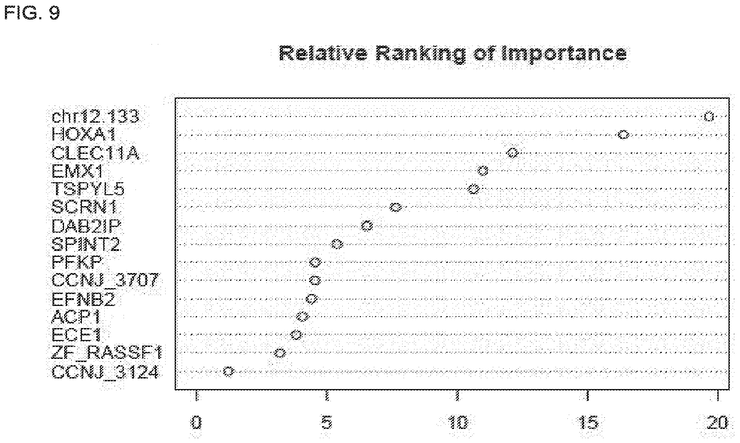

FIG. 9: Graph depicting the relative importance of each of the methylation markers considered in this analysis (ACP1, BDH1, Chr12.133, CLEC11A, DAB2IP, DBNL, EMX1, EFNB2, HOXA1, LRRC4, SPINT2, TSPYL5, CCNJ 3707, CCNJ 3124, PFKP, SCRN1, and ECE1). The cross validated estimate of sensitivity and specificity for the entire panel of markers was 75% and 96%, respectively.

### DETAILED DESCRIPTION

Provided herein is technology for hepatocellular carcinoma screening and particularly, but not exclusively, to methods, compositions, and related uses for detecting the presence of hepatocellular carcinoma.

As the technology is described herein, the section headings used are for organizational purposes only and are not to be construed as limiting the subject matter in any way.

In this detailed description of the various embodiments, for purposes of explanation, numerous specific details are set forth to provide a thorough understanding of the embodiments disclosed. One skilled in the art will appreciate, however, that these various embodiments may be practiced with or without these specific details. In other instances, structures and devices are shown in block diagram form. Furthermore, one skilled in the art can readily appreciate that the specific sequences in which methods are presented and performed are illustrative and it is contemplated that the sequences can be varied and still remain within the spirit and scope of the various embodiments disclosed herein.

Unless defined otherwise, all technical and scientific terms used herein have the same meaning as is commonly understood by one of ordinary skill in the art to which the various embodiments described herein belongs. When definitions of terms in incorporated references appear to differ from the definitions provided in the present teachings, the definition provided in the present teachings shall control.

### Definitions

To facilitate an understanding of the present technology, a number of terms and phrases are defined below. Additional definitions are set forth throughout the detailed description.

Throughout the specification and claims, the following terms take the meanings explicitly associated herein, unless the context clearly dictates otherwise. The phrase "in one embodiment" as used herein does not necessarily refer to the same embodiment, though it may. Furthermore, the phrase "in another embodiment" as used herein does not necessarily refer to a different embodiment, although it may. Thus, as described below, various embodiments of the invention may be readily combined, without departing from the scope or spirit of the invention.

In addition, as used herein, the term "or" is an inclusive "or" operator and is equivalent to the term "and/or" unless the context clearly dictates otherwise. The term "based on" is not exclusive and allows for being based on additional factors not described, unless the context clearly dictates otherwise. In addition, throughout the specification, the meaning of "a", "an", and "the" include plural references. The meaning of "in" includes "in" and "on."

As used herein, a "nucleic acid" or "nucleic acid molecule" generally refers to any ribonucleic acid or deoxyribonucleic acid, which may be unmodified or modified DNA or RNA. "Nucleic acids" include, without limitation, single- and double-stranded nucleic acids. As used herein, the term "nucleic acid" also includes DNA as described above that contains one or more modified bases. Thus, DNA with a backbone modified for stability or for other reasons is a "nucleic acid". The term "nucleic acid" as it is used herein embraces such chemically, enzymatically, or metabolically modified forms of nucleic acids, as well as the chemical forms of DNA characteristic of viruses and cells, including for example, simple and complex cells.

The terms "oligonucleotide" or "polynucleotide" or "nucleotide" or "nucleic acid" refer to a molecule having two or more deoxyribonucleotides or ribonucleotides, preferably more than three, and usually more than ten. The exact size will depend on many factors, which in turn depends on the ultimate function or use of the oligonucleotide. The oligonucleotide may be generated in any manner, including chemical synthesis, DNA replication, reverse transcription, or a combination thereof. Typical deoxyribonucleotides for DNA are thymine, adenine, cytosine, and guanine. Typical ribonucleotides for RNA are uracil, adenine, cytosine, and guanine.

As used herein, the terms "locus" or "region" of a nucleic acid refer to a subregion of a nucleic acid, e.g., a gene on a chromosome, a single nucleotide, a CpG island, etc.

The terms "complementary" and "complementarity" refer to nucleotides (e.g., 1 nucleotide) or polynucleotides (e.g., a sequence of nucleotides) related by the base-pairing rules. For example, the sequence 5'-A-G-T-3' is complementary to the sequence 3'-T-C-A-5'. Complementarity may be "partial," in which only some of the nucleic acids' bases are matched according to the base pairing rules. Or, there may be "complete" or "total" complementarity between the nucleic acids. The degree of complementarity between nucleic acid strands effects the efficiency and strength of hybridization between nucleic acid strands. This is of particular importance in amplification reactions and in detection methods that depend upon binding between nucleic acids.

The term "gene" refers to a nucleic acid (e.g., DNA or RNA) sequence that comprises coding sequences necessary for the production of an RNA, or of a polypeptide or its precursor. A functional polypeptide can be encoded by a full length coding sequence or by any portion of the coding sequence as long as the desired activity or functional properties (e.g., enzymatic activity, ligand binding, signal transduction, etc.) of the polypeptide are retained. The term "portion" when used in reference to a gene refers to fragments of that gene. The fragments may range in size from a few nucleotides to the entire gene sequence minus one nucleotide. Thus, "a nucleotide comprising at least a portion of a gene" may comprise fragments of the gene or the entire gene.

The term "gene" also encompasses the coding regions of a structural gene and includes sequences located adjacent to the coding region on both the 5' and 3' ends, e.g., for a distance of about 1 kb on either end, such that the gene corresponds to the length of the fulllength mRNA (e.g., comprising coding, regulatory, structural and other sequences). The sequences that are located 5' of the coding region and that are present on the mRNA are referred to as 5' non-translated or untranslated sequences. The sequences that are located 3' or downstream of the coding region and that are present on the mRNA are referred to as 3' non-translated or 3' untranslated sequences. The term "gene" encompasses both cDNA and genomic forms of a gene. In some organisms (e.g., eukaryotes), a genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

In addition to containing introns, genomic forms of a gene may also include sequences located on both the 5' and 3' ends of the sequences that are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers that control or influence the transcription of the gene. The 3' flanking region may contain sequences that direct the termination of transcription, posttranscriptional cleavage, and polyadenylation.

The term "allele" refers to a variation of a gene; the variations include but are not limited to variants and mutants, polymorphic loci, and single nucleotide polymorphic loci, frameshift, and splice mutations. An allele may occur naturally in a population or it might arise during the lifetime of any particular individual of the population.

Thus, the terms "variant" and "mutant" when used in reference to a nucleotide sequence refer to a nucleic acid sequence that differs by one or more nucleotides from another, usually related, nucleotide acid sequence. A "variation" is a difference between two different nucleotide sequences; typically, one sequence is a reference sequence.

"Amplification" is a special case of nucleic acid replication involving template specificity. It is to be contrasted with non-specific template replication (e.g., replication that is template-dependent but not dependent on a specific template). Template specificity is here distinguished from fidelity of replication (e.g., synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

Amplification of nucleic acids generally refers to the production of multiple copies of a polynucleotide, or a portion of the polynucleotide, typically starting from a small amount of the polynucleotide (e.g., a single polynucleotide molecule, 10 to 100 copies of a polynucleotide molecule, which may or may not be exactly the same), where the amplification products or amplicons are generally detectable. Amplification of polynucleotides encompasses a variety of chemical and enzymatic processes. The generation of multiple DNA copies from one or a few copies of a target or template DNA molecule during a polymerase chain reaction (PCR) or a ligase chain reaction (LCR; see, e.g., U.S. Patent No. 5,494,810) are forms of amplification. Additional types of amplification include, but are not limited to, allele-specific PCR (see, e.g., U.S. Patent No. 5,639,611), assembly PCR (see, e.g., U.S. Patent No. 5,965,408), helicase-dependent amplification (see, e.g., U.S. Patent No. 7,662,594), Hot-start PCR (see, e.g., U.S. Patent Nos. 5,773,258 and 5,338,671), intersequence-specfic PCR, inverse PCR (see, e.g., Triglia, et alet al. (1988) Nucleic Acids Res., 16:8186), ligation-mediated PCR (see, e.g., Guilfoyle, R. et al., Nucleic Acids Research, 25:1854-1858 (1997); U.S. Patent No. 5,508,169), methylation-specific PCR (see, e.g., Herman, et al., (1996) PNAS 93(13) 9821-9826), miniprimer PCR, multiplex ligationdependent probe amplification (see, e.g., Schouten, et al., (2002) Nucleic Acids Research 30(12): e57), multiplex PCR (see, e.g., Chamberlain, et al., (1988) Nucleic Acids Research 16(23) 11141-11156; Ballabio, et al., (1990) Human Genetics 84(6) 571-573; Hayden, et al., (2008) BMC Genetics 9:80), nested PCR, overlap-extension PCR (see, e.g., Higuchi, et al., (1988) Nucleic Acids Research 16(15) 7351-7367), real time PCR (see, e.g., Higuchi, et alet al., (1992) Biotechnology 10:413-417; Higuchi, et al., (1993) Biotechnology 11:1026-1030), reverse transcription PCR (see, e.g., Bustin, S.A. (2000) J. Molecular Endocrinology 25:169-193), solid phase PCR, thermal asymmetric interlaced PCR, and Touchdown PCR (see, e.g., Don, et al., Nucleic Acids Research (1991) 19(14) 4008; Roux, K. (1994) Biotechniques 16(5) 812-814; Hecker, et al., (1996) Biotechniques 20(3) 478-485). Polynucleotide amplification also can be accomplished using digital PCR (see, e.g., Kalinina, et al., Nucleic Acids Research. 25; 1999-2004, (1997); Vogelstein and Kinzler, Proc Natl Acad Sci USA. 96; 9236-41, (1999); International Patent Publication No. WO05023091A2; US Patent Application Publication No. 20070202525).

The term "polymerase chain reaction" ("PCR") refers to the method of K.B. Mullis U.S. Patent Nos. 4,683,195, 4,683,202, and 4,965,188, that describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing, and polymerase extension can be repeated many times (i.e., denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" ("PCR"). Because the desired amplified segments of the target sequence become the predominant sequences (in terms of concentration) in the mixture, they are said to be "PCR amplified" and are "PCR products" or "amplicons."

Template specificity is achieved in most amplification techniques by the choice of enzyme. Amplification enzymes are enzymes that, under conditions they are used, will process only specific sequences of nucleic acid in a heterogeneous mixture of nucleic acid. For example, in the case of Q-beta replicase, MDV-1 RNA is the specific template for the replicase (Kacian et al., Proc. Natl. Acad. Sci. USA, 69:3038 [1972]). Other nucleic acid will not be replicated by this amplification enzyme. Similarly, in the case of T7 RNA polymerase, this amplification enzyme has a stringent specificity for its own promoters (Chamberlin et al, Nature, 228:227 [1970]). In the case of T4 DNA ligase, the enzyme will not ligate the two oligonucleotides or polynucleotides, where there is a mismatch between the oligonucleotide or polynucleotide substrate and the template at the ligation junction (Wu and Wallace (1989) Genomics 4:560). Finally, thermostable template-dependant DNA polymerases (e.g., Taq and Pfu DNA polymerases), by virtue of their ability to function at high temperature, are found to display high specificity for the sequences bounded and thus defined by the primers; the high temperature results in thermodynamic conditions that favor primer hybridization with the target sequences and not hybridization with non-target sequences (H. A. Erlich (ed.), PCR Technology, Stockton Press [1989]).

As used herein, the term "nucleic acid detection assay" refers to any method of determining the nucleotide composition of a nucleic acid of interest. Nucleic acid detection assay include but are not limited to, DNA sequencing methods, probe hybridization methods, structure specific cleavage assays (e.g., the INVADER assay, Hologic, Inc.) and are described, e.g., in U.S. Patent Nos. 5,846,717, 5,985,557, 5,994,069, 6,001,567, 6,090,543, and 6,872,816; Lyamichev et al., Nat. Biotech., 17:292 (1999), Hall et al., PNAS, USA, 97:8272 (2000), and US 2009/0253142); enzyme mismatch cleavage methods (e.g., Variagenics, U.S. Pat. Nos. 6,110,684, 5,958,692, 5,851,770); polymerase chain reaction; branched hybridization methods (e.g., Chiron, U.S. Pat. Nos. 5,849,481, 5,710,264, 5,124,246, and 5,624,802); rolling circle replication (e.g., U.S. Pat. Nos. 6,210,884, 6,183,960 and 6,235,502); NASBA (e.g., U.S. Pat. No. 5,409,818); molecular beacon technology (e.g., U.S. Pat. No. 6,150,097); E-sensor technology (Motorola, U.S. Pat. Nos. 6,248,229, 6,221,583, 6,013,170, and 6,063,573); cycling probe technology (e.g., U.S. Pat. Nos. 5,403,711, 5,011,769, and 5,660,988); Dade Behring signal amplification methods (e.g., U.S. Pat. Nos. 6,121,001, 6,110,677, 5,914,230, 5,882,867, and 5,792,614); ligase chain reaction (e.g., Barnay Proc. Natl. Acad. Sci USA 88, 189-93 (1991)); and sandwich hybridization methods (e.g., U.S. Pat. No. 5,288,609).

The term "amplifiable nucleic acid" refers to a nucleic acid that may be amplified by any amplification method. It is contemplated that "amplifiable nucleic acid" will usually comprise "sample template."

The term "sample template" refers to nucleic acid originating from a sample that is analyzed for the presence of "target" (defined below). In contrast, "background template" is used in reference to nucleic acid other than sample template that may or may not be present in a sample. Background template is most often inadvertent. It may be the result of carryover or it may be due to the presence of nucleic acid contaminants sought to be purified away from the sample. For example, nucleic acids from organisms other than those to be detected may be present as background in a test sample.

The term "primer" refers to an oligonucleotide, whether occurring naturally as in a purified restriction digest or produced synthetically, that is capable of acting as a point of initiation of synthesis when placed under conditions in which synthesis of a primer extension product that is complementary to a nucleic acid strand is induced, (e.g., in the presence of nucleotides and an inducing agent such as a DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer, and the use of the method.

The term "probe" refers to an oligonucleotide (e.g., a sequence of nucleotides), whether occurring naturally as in a purified restriction digest or produced synthetically, recombinantly, or by PCR amplification, that is capable of hybridizing to another oligonucleotide of interest. A probe may be single-stranded or double-stranded. Probes are useful in the detection, identification, and isolation of particular gene sequences (e.g., a "capture probe"). It is contemplated that any probe used in the present invention may, in some embodiments, be labeled with any "reporter molecule," so that is detectable in any detection system, including, but not limited to enzyme (e.g., ELISA, as well as enzyme-based histochemical assays), fluorescent, radioactive, and luminescent systems. It is not intended that the present invention be limited to any particular detection system or label.

As used herein, "methylation" refers to cytosine methylation at positions C5 or N4 of cytosine, the N6 position of adenine, or other types of nucleic acid methylation. In vitro amplified DNA is usually unmethylated because typical in vitro DNA amplification methods do not retain the methylation pattern of the amplification template. However, "unmethylated DNA" or "methylated DNA" can also refer to amplified DNA whose original template was unmethylated or methylated, respectively.

Accordingly, as used herein a "methylated nucleotide" or a "methylated nucleotide base" refers to the presence of a methyl moiety on a nucleotide base, where the methyl moiety is not present in a recognized typical nucleotide base. For example, cytosine does not contain a methyl moiety on its pyrimidine ring, but 5-methylcytosine contains a methyl moiety at position 5 of its pyrimidine ring. Therefore, cytosine is not a methylated nucleotide and 5-methylcytosine is a methylated nucleotide. In another example, thymine contains a methyl moiety at position 5 of its pyrimidine ring; however, for purposes herein, thymine is not considered a methylated nucleotide when present in DNA since thymine is a typical nucleotide base of DNA.

As used herein, a "methylated nucleic acid molecule" refers to a nucleic acid molecule that contains one or more methylated nucleotides.

As used herein, a "methylation state", "methylation profile", and "methylation status" of a nucleic acid molecule refers to the presence of absence of one or more methylated nucleotide bases in the nucleic acid molecule. For example, a nucleic acid molecule containing a methylated cytosine is considered methylated (e.g., the methylation state of the nucleic acid molecule is methylated). A nucleic acid molecule that does not contain any methylated nucleotides is considered unmethylated.

The methylation state of a particular nucleic acid sequence (e.g., a gene marker or DNA region as described herein) can indicate the methylation state of every base in the sequence or can indicate the methylation state of a subset of the bases (e.g., of one or more cytosines) within the sequence, or can indicate information regarding regional methylation density within the sequence with or without providing precise information of the locations within the sequence the methylation occurs.

The methylation state of a nucleotide locus in a nucleic acid molecule refers to the presence or absence of a methylated nucleotide at a particular locus in the nucleic acid molecule. For example, the methylation state of a cytosine at the 7th nucleotide in a nucleic acid molecule is methylated when the nucleotide present at the 7th nucleotide in the nucleic acid molecule is 5-methylcytosine. Similarly, the methylation state of a cytosine at the 7th nucleotide in a nucleic acid molecule is unmethylated when the nucleotide present at the 7th nucleotide in the nucleic acid molecule is cytosine (and not 5-methylcytosine).

The methylation status can optionally be represented or indicated by a "methylation value" (e.g., representing a methylation frequency, fraction, ratio, percent, etc.) A methylation value can be generated, for example, by quantifying the amount of intact nucleic acid present following restriction digestion with a methylation dependent restriction enzyme or by comparing amplification profiles after bisulfite reaction or by comparing sequences of bisulfite-treated and untreated nucleic acids. Accordingly, a value, e.g., a methylation value, represents the methylation status and can thus be used as a quantitative indicator of methylation status across multiple copies of a locus. This is of particular use when it is desirable to compare the methylation status of a sequence in a sample to a threshold or reference value.

As used herein, "methylation frequency" or "methylation percent (%)" refer to the number of instances in which a molecule or locus is methylated relative to the number of instances the molecule or locus is unmethylated.

As such, the methylation state describes the state of methylation of a nucleic acid (e.g., a genomic sequence). In addition, the methylation state refers to the characteristics of a nucleic acid segment at a particular genomic locus relevant to methylation. Such characteristics include, but are not limited to, whether any of the cytosine (C) residues within this DNA sequence are methylated, the location of methylated C residue(s), the frequency or percentage of methylated C throughout any particular region of a nucleic acid, and allelic differences in methylation due to, e.g., difference in the origin of the alleles. The terms "methylation state", "methylation profile", and "methylation status" also refer to the relative concentration, absolute concentration, or pattern of methylated C or unmethylated C throughout any particular region of a nucleic acid in a biological sample. For example, if the cytosine (C) residue(s) within a nucleic acid sequence are methylated it may be referred to as "hypermethylated" or having "increased methylation", whereas if the cytosine (C) residue(s) within a DNA sequence are not methylated it may be referred to as "hypomethylated" or having "decreased methylation". Likewise, if the cytosine (C) residue(s) within a nucleic acid sequence are methylated as compared to another nucleic acid sequence (e.g., from a different region or from a different individual, etc.) that sequence is considered hypermethylated or having increased methylation compared to the other nucleic acid sequence. Alternatively, if the cytosine (C) residue(s) within a DNA sequence are not methylated as compared to another nucleic acid sequence (e.g., from a different region or from a different individual, etc.) that sequence is considered hypomethylated or having decreased methylation compared to the other nucleic acid sequence. Additionally, the term "methylation pattern" as used herein refers to the collective sites of methylated and unmethylated nucleotides over a region of a nucleic acid. Two nucleic acids may have the same or similar methylation frequency or methylation percent but have different methylation patterns when the number of methylated and unmethylated nucleotides are the same or similar throughout the region but the locations of methylated and unmethylated nucleotides are different. Sequences are said to be "differentially methylated" or as having a "difference in methylation" or having a "different methylation state" when they differ in the extent (e.g., one has increased or decreased methylation relative to the other), frequency, or pattern of methylation. The term "differential methylation" refers to a difference in the level or pattern of nucleic acid methylation in a cancer positive sample as compared with the level or pattern of nucleic acid methylation in a cancer negative sample. It may also refer to the difference in levels or patterns between patients that have recurrence of cancer after surgery versus patients who not have recurrence. Differential methylation and specific levels or patterns of DNA methylation are prognostic and predictive biomarkers, e.g., once the correct cut-off or predictive characteristics have been defined.

Methylation state frequency can be used to describe a population of individuals or a sample from a single individual. For example, a nucleotide locus having a methylation state frequency of 50% is methylated in 50% of instances and unmethylated in 50% of instances. Such a frequency can be used, for example, to describe the degree to which a nucleotide locus or nucleic acid region is methylated in a population of individuals or a collection of nucleic acids. Thus, when methylation in a first population or pool of nucleic acid molecules is different from methylation in a second population or pool of nucleic acid molecules, the methylation state frequency of the first population or pool will be different from the methylation state frequency of the second population or pool. Such a frequency also can be used, for example, to describe the degree to which a nucleotide locus or nucleic acid region is methylated in a single individual. For example, such a frequency can be used to describe the degree to which a group of cells from a tissue sample are methylated or unmethylated at a nucleotide locus or nucleic acid region.

As used herein a "nucleotide locus" refers to the location of a nucleotide in a nucleic acid molecule. A nucleotide locus of a methylated nucleotide refers to the location of a methylated nucleotide in a nucleic acid molecule.

Typically, methylation of human DNA occurs on a dinucleotide sequence including an adjacent guanine and cytosine where the cytosine is located 5' of the guanine (also termed CpG dinucleotide sequences). Most cytosines within the CpG dinucleotides are methylated in the human genome, however some remain unmethylated in specific CpG dinucleotide rich genomic regions, known as CpG islands (see, e.g, Antequera et al. (1990) Cell 62: 503-514).

As used herein, a "CpG island" refers to a G:C-rich region of genomic DNA containing an increased number of CpG dinucleotides relative to total genomic DNA. A CpG island can be at least 100, 200, or more base pairs in length, where the G:C content of the region is at least 50% and the ratio of observed CpG frequency over expected frequency is 0.6; in some instances, a CpG island can be at least 500 base pairs in length, where the G:C content of the region is at least 55%) and the ratio of observed CpG frequency over expected frequency is 0.65. The observed CpG frequency over expected frequency can be calculated according to the method provided in Gardiner-Garden et al (1987) J. Mol. Biol. 196: 261-281. For example, the observed CpG frequency over expected frequency can be calculated according to the formula R = (A × B) / (C × D), where R is the ratio of observed CpG frequency over expected frequency, A is the number of CpG dinucleotides in an analyzed sequence, B is the total number of nucleotides in the analyzed sequence, C is the total number of C nucleotides in the analyzed sequence, and D is the total number of G nucleotides in the analyzed sequence. Methylation state is typically determined in CpG islands, e.g., at promoter regions. It will be appreciated though that other sequences in the human genome are prone to DNA methylation such as CpA and CpT (see, e.g., Ramsahoye (2000) Proc. Natl. Acad. Sci. USA 97: 5237-5242; Salmon and Kaye (1970) Biochim. Biophys. Acta. 204: 340-351; Grafstrom (1985) Nucleic Acids Res. 13: 2827-2842; Nyce (1986) Nucleic Acids Res. 14: 4353-4367; Woodcock (1987) Biochem. Biophys. Res. Commun. 145: 888-894).

As used herein, a reagent that modifies a nucleotide of the nucleic acid molecule as a function of the methylation state of the nucleic acid molecule, or a methylation-specific reagent, refers to a compound or composition or other agent that can change the nucleotide sequence of a nucleic acid molecule in a manner that reflects the methylation state of the nucleic acid molecule. Methods of treating a nucleic acid molecule with such a reagent can include contacting the nucleic acid molecule with the reagent, coupled with additional steps, if desired, to accomplish the desired change of nucleotide sequence. Such a change in the nucleic acid molecule's nucleotide sequence can result in a nucleic acid molecule in which each methylated nucleotide is modified to a different nucleotide. Such a change in the nucleic acid nucleotide sequence can result in a nucleic acid molecule in which each unmethylated nucleotide is modified to a different nucleotide. Such a change in the nucleic acid nucleotide sequence can result in a nucleic acid molecule in which each of a selected nucleotide which is unmethylated (e.g., each unmethylated cytosine) is modified to a different nucleotide. Use of such a reagent to change the nucleic acid nucleotide sequence can result in a nucleic acid molecule in which each nucleotide that is a methylated nucleotide (e.g., each methylated cytosine) is modified to a different nucleotide. As used herein, use of a reagent that modifies a selected nucleotide refers to a reagent that modifies one nucleotide of the four typically occurring nucleotides in a nucleic acid molecule (C, G, T, and A for DNA and C, G, U, and A for RNA), such that the reagent modifies the one nucleotide without modifying the other three nucleotides. In one exemplary embodiment, such a reagent modifies an unmethylated selected nucleotide to produce a different nucleotide. In another exemplary embodiment, such a reagent can deaminate unmethylated cytosine nucleotides. An exemplary reagent is bisulfite.

As used herein, the term "bisulfite reagent" refers to a reagent comprising in some embodiments bisulfite, disulfite, hydrogen sulfite, or combinations thereof to distinguish between methylated and unmethylated cytidines, e.g., in CpG dinucleotide sequences.

The term "methylation assay" refers to any assay for determining the methylation state of one or more CpG dinucleotide sequences within a sequence of a nucleic acid.

The term "MS AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al. (1997) Cancer Research 57: 594-599.

The term "MethyLight^{™}" refers to the art-recognized fluorescence-based real-time PCR technique described by Eads et al. (1999) Cancer Res. 59: 2302-2306.

The term "HeavyMethyl^{™}" refers to an assay wherein methylation specific blocking probes (also referred to herein as blockers) covering CpG positions between, or covered by, the amplification primers enable methylation-specific selective amplification of a nucleic acid sample.

The term "HeavyMethyl^{™} MethyLight^{™}" assay refers to a HeavyMethyl^{™} MethyLight^{™} assay, which is a variation of the MethyLight^{™} assay, wherein the MethyLight^{™} assay is combined with methylation specific blocking probes covering CpG positions between the amplification primers.

The term "Ms-SNuPE" (Methylation-sensitive Single Nucleotide Primer Extension) refers to the art-recognized assay described by Gonzalgo & Jones (1997) Nucleic Acids Res. 25: 2529-2531.

The term "MSP" (Methylation-specific PCR) refers to the art-recognized methylation assay described by Herman et al. (1996) Proc. Natl. Acad. Sci. USA 93: 9821-9826, and by U.S. Pat. No. 5,786,146.

The term "COBRA" (Combined Bisulfite Restriction Analysis) refers to the art-recognized methylation assay described by Xiong & Laird (1997) Nucleic Acids Res. 25: 2532-2534.

The term "MCA" (Methylated CpG Island Amplification) refers to the methylation assay described by Toyota et al. (1999) Cancer Res. 59: 2307-12, and in WO 00/26401A1.

As used herein, a "selected nucleotide" refers to one nucleotide of the four typically occurring nucleotides in a nucleic acid molecule (C, G, T, and A for DNA and C, G, U, and A for RNA), and can include methylated derivatives of the typically occurring nucleotides (e.g., when C is the selected nucleotide, both methylated and unmethylated C are included within the meaning of a selected nucleotide), whereas a methylated selected nucleotide refers specifically to a methylated typically occurring nucleotide and an unmethylated selected nucleotides refers specifically to an unmethylated typically occurring nucleotide.

The terms "methylation-specific restriction enzyme" or "methylation-sensitive restriction enzyme" refers to an enzyme that selectively digests a nucleic acid dependent on the methylation state of its recognition site. In the case of a restriction enzyme that specifically cuts if the recognition site is not methylated or is hemimethylated, the cut will not take place or will take place with a significantly reduced efficiency if the recognition site is methylated. In the case of a restriction enzyme that specifically cuts if the recognition site is methylated, the cut will not take place or will take place with a significantly reduced efficiency if the recognition site is not methylated. Preferred are methylation-specific restriction enzymes, the recognition sequence of which contains a CG dinucleotide (for instance a recognition sequence such as CGCG or CCCGGG). Further preferred for some embodiments are restriction enzymes that do not cut if the cytosine in this dinucleotide is methylated at the carbon atom C5.

As used herein, a "different nucleotide" refers to a nucleotide that is chemically different from a selected nucleotide, typically such that the different nucleotide has Watson-Crick base-pairing properties that differ from the selected nucleotide, whereby the typically occurring nucleotide that is complementary to the selected nucleotide is not the same as the typically occurring nucleotide that is complementary to the different nucleotide. For example, when C is the selected nucleotide, U or T can be the different nucleotide, which is exemplified by the complementarity of C to G and the complementarity of U or T to A. As used herein, a nucleotide that is complementary to the selected nucleotide or that is complementary to the different nucleotide refers to a nucleotide that base-pairs, under high stringency conditions, with the selected nucleotide or different nucleotide with higher affinity than the complementary nucleotide's base-paring with three of the four typically occurring nucleotides. An example of complementarity is Watson-Crick base pairing in DNA (e.g., A-T and C-G) and RNA (e.g., A-U and C-G). Thus, for example, G base-pairs, under high stringency conditions, with higher affinity to C than G base-pairs to G, A, or T and, therefore, when C is the selected nucleotide, G is a nucleotide complementary to the selected nucleotide.

As used herein, the "sensitivity" of a given marker refers to the percentage of samples that report a DNA methylation value above a threshold value that distinguishes between neoplastic and non-neoplastic samples. In some embodiments, a positive is defined as a histology-confirmed neoplasia that reports a DNA methylation value above a threshold value (e.g., the range associated with disease), and a false negative is defined as a histology-confirmed neoplasia that reports a DNA methylation value below the threshold value (e.g., the range associated with no disease). The value of sensitivity, therefore, reflects the probability that a DNA methylation measurement for a given marker obtained from a known diseased sample will be in the range of disease-associated measurements. As defined here, the clinical relevance of the calculated sensitivity value represents an estimation of the probability that a given marker would detect the presence of a clinical condition when applied to a subject with that condition.

As used herein, the "specificity" of a given marker refers to the percentage of non-neoplastic samples that report a DNA methylation value below a threshold value that distinguishes between neoplastic and non-neoplastic samples. In some embodiments, a negative is defined as a histology-confirmed non-neoplastic sample that reports a DNA methylation value below the threshold value (e.g., the range associated with no disease) and a false positive is defined as a histology-confirmed non-neoplastic sample that reports a DNA methylation value above the threshold value (e.g., the range associated with disease). The value of specificity, therefore, reflects the probability that a DNA methylation measurement for a given marker obtained from a known non-neoplastic sample will be in the range of nondisease associated measurements. As defined here, the clinical relevance of the calculated specificity value represents an estimation of the probability that a given marker would detect the absence of a clinical condition when applied to a patient without that condition.

The term "AUC" as used herein is an abbreviation for the "area under a curve". In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better; the optimum is 1; a random test would have a ROC curve lying on the diagonal with an area of 0.5; for reference: J. P. Egan. (1975) Signal Detection Theory and ROC Analysis, Academic Press, New York).

As used herein, the term "neoplasm" refers to "an abnormal mass of tissue, the growth of which exceeds and is uncoordinated with that of the normal tissues" See, e.g., Willis RA, "The Spread of Tumors in the Human Body", London, Butterworth & Co, 1952.

As used herein, the term "adenoma" refers to a benign tumor of glandular origin. Although these growths are benign, over time they may progress to become malignant.

The term "pre-cancerous" or "pre-neoplastic" and equivalents thereof refer to any cellular proliferative disorder that is undergoing malignant transformation.

A "site" or "region" of a neoplasm, adenoma, cancer, etc. is the tissue, organ, cell type, anatomical area, body part, etc. in a subject's body where the neoplasm, adenoma, cancer, etc. is located.

As used herein, a "diagnostic" test application includes the detection or identification of a disease state or condition of a subject, determining the likelihood that a subject will contract a given disease or condition, determining the likelihood that a subject with a disease or condition will respond to therapy, determining the prognosis of a subject with a disease or condition (or its likely progression or regression), and determining the effect of a treatment on a subject with a disease or condition. For example, a diagnostic can be used for detecting the presence or likelihood of a subject contracting a neoplasm or the likelihood that such a subject will respond favorably to a compound (e.g., a pharmaceutical, e.g., a drug) or other treatment.

The term "marker", as used herein, refers to a substance (e.g., a nucleic acid or a region of a nucleic acid) that is able to diagnose a disorder (e.g., a non-cancerous disorder) (e.g., a cancerous disorder) by distinguishing disorder-associated cells (e.g., non-cancerous cells associated with the disorder) (e.g., cancerous cells associated with the disorder) from normal cells, e.g., based its methylation state.

The term "isolated" when used in relation to a nucleic acid, as in "an isolated oligonucleotide" refers to a nucleic acid sequence that is identified and separated from at least one contaminant nucleic acid with which it is ordinarily associated in its natural source. Isolated nucleic acid is present in a form or setting that is different from that in which it is found in nature. In contrast, non-isolated nucleic acids, such as DNA and RNA, are found in the state they exist in nature. Examples of non-isolated nucleic acids include: a given DNA sequence (e.g., a gene) found on the host cell chromosome in proximity to neighboring genes; RNA sequences, such as a specific mRNA sequence encoding a specific protein, found in the cell as a mixture with numerous other mRNAs which encode a multitude of proteins. However, isolated nucleic acid encoding a particular protein includes, by way of example, such nucleic acid in cells ordinarily expressing the protein, where the nucleic acid is in a chromosomal location different from that of natural cells, or is otherwise flanked by a different nucleic acid sequence than that found in nature. The isolated nucleic acid or oligonucleotide may be present in single-stranded or double-stranded form. When an isolated nucleic acid or oligonucleotide is to be utilized to express a protein, the oligonucleotide will contain at a minimum the sense or coding strand (i.e., the oligonucleotide may be single-stranded), but may contain both the sense and anti-sense strands (i.e., the oligonucleotide may be double-stranded). An isolated nucleic acid may, after isolation from its natural or typical environment, by be combined with other nucleic acids or molecules. For example, an isolated nucleic acid may be present in a host cell in which into which it has been placed, e.g., for heterologous expression.

The term "purified" refers to molecules, either nucleic acid or amino acid sequences that are removed from their natural environment, isolated, or separated. An "isolated nucleic acid sequence" may therefore be a purified nucleic acid sequence. "Substantially purified" molecules are at least 60% free, preferably at least 75% free, and more preferably at least 90% free from other components with which they are naturally associated. As used herein, the terms "purified" or "to purify" also refer to the removal of contaminants from a sample. The removal of contaminating proteins results in an increase in the percent of polypeptide or nucleic acid of interest in the sample. In another example, recombinant polypeptides are expressed in plant, bacterial, yeast, or mammalian host cells and the polypeptides are purified by the removal of host cell proteins; the percent of recombinant polypeptides is thereby increased in the sample.

The term "composition comprising" a given polynucleotide sequence or polypeptide refers broadly to any composition containing the given polynucleotide sequence or polypeptide. The composition may comprise an aqueous solution containing salts (e.g., NaCl), detergents (e.g., SDS), and other components (e.g., Denhardt's solution, dry milk, salmon sperm DNA, etc.).

The term "sample" is used in its broadest sense. In one sense it can refer to an animal cell or tissue. In another sense, it is meant to include a specimen or culture obtained from any source, as well as biological and environmental samples. Biological samples may be obtained from plants or animals (including humans) and encompass fluids, solids, tissues, and gases. Environmental samples include environmental material such as surface matter, soil, water, and industrial samples. These examples are not to be construed as limiting the sample types applicable to the present invention

As used herein, a "remote sample" as used in some contexts relates to a sample indirectly collected from a site that is not the cell, tissue, or organ source of the sample. For instance, when sample material originating from the pancreas is assessed in a stool sample (e.g., not from a sample taken directly from a pancreas), the sample is a remote sample.

As used herein, the terms "patient" or "subject" refer to organisms to be subject to various tests provided by the technology. The term "subject" includes animals, preferably mammals, including humans. In a preferred embodiment, the subject is a primate. In an even more preferred embodiment, the subject is a human.

As used herein, the term "kit" refers to any delivery system for delivering materials. In the context of reaction assays, such delivery systems include systems that allow for the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contain a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

### Embodiments of the technology

Provided herein is technology for HCC screening (e.g., surveilling) and particularly, but not exclusively, to methods, compositions, and related uses for detecting the presence of HCC in subjects.

Markers and/or panels of markers were identified (e.g., a chromosomal region having an annotation provided in Tables 1 and 4) capable of detecting HCC (see, Examples I, II, and III) (e.g., ACP1, BDH1, Chr12.133, CLEC11A, DAB2IP, DBNL, EMX1, EFNB2, HOXA1, LRRC4, SPINT2, TSPYL5, CCNJ 3707, CCNJ 3124, PFKP, SCRN1, and ECE1).

Although the disclosure herein refers to certain illustrated embodiments, it is to be understood that these embodiments are presented by way of example and not by way of limitation.

The methods comprise determining the methylation status of at least one methylation marker in a biological sample isolated from a subject, wherein a change in the methylation state of the marker is indicative of the presence, or class of HCC. Particular aspects relate to markers comprising a differentially methylated region (DMR, e.g., DMR 1-400 (from Tables 1 and 4)) that are used for diagnosis (e.g., screening) of HCC.

In addition to aspects wherein the methylation analysis of at least one marker, a region of a marker, or a base of a marker comprising a DMR (e.g., DMR 1-400) provided herein and listed in Tables 1 and 3 is analyzed, the technology also provides panels of markers comprising at least one marker, region of a marker, or base of a marker comprising a DMR with utility for the detection of HCC in a subject.

Some aspects of the technology are based upon the analysis of the CpG methylation status of at least one marker, region of a marker, or base of a marker comprising a DMR.

In some aspects, the present technology provides for the use of the bisulfite technique in combination with one or more methylation assays to determine the methylation status of CpG dinucleotide sequences within at least one marker comprising a DMR (e.g., as provided in Tables 1 and 4 (e.g., DMR 1-400)). Genomic CpG dinucleotides can be methylated or unmethylated (alternatively known as up- and down-methylated respectively). However the methods of the present invention are suitable for the analysis of biological samples of a heterogeneous nature, e.g., a low concentration of tumor cells, or biological materials therefrom, within a background of a remote sample (e.g., blood, organ effluent, or stool). Accordingly, when analyzing the methylation status of a CpG position within such a sample one may use a quantitative assay for determining the level (e.g., percent, fraction, ratio, proportion, or degree) of methylation at a particular CpG position.

According to the present technology, determination of the methylation status of CpG dinucleotide sequences in markers comprising a DMR has utility both in the diagnosis and characterization of HCC in subjects.

### Combinations of markers

In some aspects, the technology relates to assessing the methylation state of combinations of markers comprising two or more DMRs from Tables 1 and/or 4 (e.g., two or more DMRs from DMR Nos. 1-400). In some aspects, assessing the methylation state of more than one marker increases the specificity and/or sensitivity of a screen or diagnostic for identifying the presence of HCC in a subject.

Various cancers are predicted by various combinations of markers, e.g., as identified by statistical techniques related to specificity and sensitivity of prediction. The technology provides methods for identifying predictive combinations and validated predictive combinations for some cancers.

For example, markers and/or panels of markers were identified (e.g., a chromosomal region having an annotation provided in Tables 1 and 4) capable of detecting HCC (see, Examples I, II and III) (e.g., ACP1, BDH1, Chr12.133, CLEC11A, DAB2IP, DBNL, EMX1, EFNB2, HOXA1, LRRC4, SPINT2, TSPYL5, CCNJ 3707, CCNJ 3124, PFKP, SCRN1, and ECE1).

### Methods for assaying methylation state

The most frequently used method for analyzing a nucleic acid for the presence of 5-methylcytosine is based upon the bisulfite method described by Frommer, et al. for the detection of 5-methylcytosines in DNA (Frommer et al. (1992) Proc. Natl. Acad. Sci. USA 89: 1827-31) or variations thereof. The bisulfite method of mapping 5-methylcytosines is based on the observation that cytosine, but not 5-methylcytosine, reacts with hydrogen sulfite ion (also known as bisulfite). The reaction is usually performed according to the following steps: first, cytosine reacts with hydrogen sulfite to form a sulfonated cytosine. Next, spontaneous deamination of the sulfonated reaction intermediate results in a sulfonated uracil. Finally, the sulfonated uricil is desulfonated under alkaline conditions to form uracil. Detection is possible because uracil forms base pairs with adenine (thus behaving like thymine), whereas 5-methylcytosine base pairs with guanine (thus behaving like cytosine). This makes the discrimination of methylated cytosines from non-methylated cytosines possible by, e.g., bisulfite genomic sequencing (Grigg G, & Clark S, Bioessays (1994) 16: 431-36; Grigg G, DNA Seq. (1996) 6: 189-98) or methylation-specific PCR (MSP) as is disclosed, e.g., in U.S. Patent No. 5,786,146.

Some conventional technologies are related to methods comprising enclosing the DNA to be analyzed in an agarose matrix, thereby preventing the diffusion and renaturation of the DNA (bisulfite only reacts with single-stranded DNA), and replacing precipitation and purification steps with a fast dialysis (Olek A, et al. (1996) "A modified and improved method for bisulfite based cytosine methylation analysis" Nucleic Acids Res. 24: 5064-6). It is thus possible to analyze individual cells for methylation status, illustrating the utility and sensitivity of the method. An overview of conventional methods for detecting 5-methylcytosine is provided by Rein, T., et al. (1998) Nucleic Acids Res. 26: 2255.

The bisulfite technique typically involves amplifying short, specific fragments of a known nucleic acid subsequent to a bisulfite treatment, then either assaying the product by sequencing (Olek & Walter (1997) Nat. Genet. 17: 275-6) or a primer extension reaction (Gonzalgo & Jones (1997) Nucleic Acids Res. 25: 2529-31; WO 95/00669; U.S. Pat. No. 6,251,594) to analyze individual cytosine positions. Some methods use enzymatic digestion (Xiong & Laird (1997) Nucleic Acids Res. 25: 2532-4). Detection by hybridization has also been described in the art (Olek et al., WO 99/28498). Additionally, use of the bisulfite technique for methylation detection with respect to individual genes has been described (Grigg & Clark (1994) Bioessays 16: 431-6,; Zeschnigk et al. (1997) Hum Mol Genet. 6: 387-95; Feil et al. (1994) Nucleic Acids Res. 22: 695; Martin et al. (1995) Gene 157: 261-4; WO 9746705; WO 9515373).

Various methylation assay procedures are known in the art and can be used in conjunction with bisulfite treatment according to the present technology. These assays allow for determination of the methylation state of one or a plurality of CpG dinucleotides (e.g., CpG islands) within a nucleic acid sequence. Such assays involve, among other techniques, sequencing of bisulfite-treated nucleic acid, PCR (for sequence-specific amplification), Southern blot analysis, and use of methylation-sensitive restriction enzymes.

For example, genomic sequencing has been simplified for analysis of methylation patterns and 5-methylcytosine distributions by using bisulfite treatment (Frommer et al. (1992) Proc. Natl. Acad. Sci. USA 89: 1827-1831). Additionally, restriction enzyme digestion of PCR products amplified from bisulfite-converted DNA finds use in assessing methylation state, e.g., as described by Sadri & Hornsby (1997) Nucl. Acids Res. 24: 5058-5059 or as embodied in the method known as COBRA (Combined Bisulfite Restriction Analysis) (Xiong & Laird (1997) Nucleic Acids Res. 25: 2532-2534).

COBRA^{™} analysis is a quantitative methylation assay useful for determining DNA methylation levels at specific loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (e.g., as might be found in a typical COBRA^{™}-based kit) for COBRA^{™} analysis may include, but are not limited to: PCR primers for specific loci (e.g., specific genes, markers, DMR, regions of genes, regions of markers, bisulfite treated DNA sequence , CpG island, etc.); restriction enzyme and appropriate buffer; gene-hybridization oligonucleotide; control hybridization oligonucleotide; kinase labeling kit for oligonucleotide probe; and labeled nucleotides. Additionally, bisulfite conversion reagents may include: DNA denaturation buffer; sulfonation buffer; DNA recovery reagents or kits (e.g., precipitation, ultrafiltration, affinity column); desulfonation buffer; and DNA recovery components.

Preferably, assays such as "MethyLight^{™}" (a fluorescence-based real-time PCR technique) (Eads et al., Cancer Res. 59:2302-2306, 1999), Ms-SNuPE^{™} (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; U.S. Pat. No. 5,786,146), and methylated CpG island amplification ("MCA"; Toyota et al., Cancer Res. 59:2307-12, 1999) are used alone or in combination with one or more of these methods.

The "HeavyMethyl^{™}" assay, technique is a quantitative method for assessing methylation differences based on methylation-specific amplification of bisulfite-treated DNA. Methylation-specific blocking probes ("blockers") covering CpG positions between, or covered by, the amplification primers enable methylation-specific selective amplification of a nucleic acid sample.

The term "HeavyMethyl^{™} MethyLight^{™}" assay refers to a HeavyMethyl^{™} MethyLight^{™} assay, which is a variation of the MethyLight^{™} assay, wherein the MethyLight^{™} assay is combined with methylation specific blocking probes covering CpG positions between the amplification primers. The HeavyMethyl^{™} assay may also be used in combination with methylation specific amplification primers.

Typical reagents (e.g., as might be found in a typical MethyLight^{™}-based kit) for HeavyMethyl^{™} analysis may include, but are not limited to: PCR primers for specific loci (e.g., specific genes, markers, DMR, regions of genes, regions of markers, bisulfite treated DNA sequence , CpG island, or bisulfite treated DNA sequence or CpG island, etc.); blocking oligonucleotides; optimized PCR buffers and deoxynucleotides; and Taq polymerase.

MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; U.S. Pat. No. 5,786,146). Briefly, DNA is modified by sodium bisulfite, which converts unmethylated, but not methylated cytosines, to uracil, and the products are subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (e.g., as might be found in a typical MSP-based kit) for MSP analysis may include, but are not limited to: methylated and unmethylated PCR primers for specific loci (e.g., specific genes, markers, DMR, regions of genes, regions of markers, bisulfite treated DNA sequence , CpG island, etc.); optimized PCR buffers and deoxynucleotides, and specific probes.

The MethyLight^{™} assay is a high-throughput quantitative methylation assay that utilizes fluorescence-based real-time PCR (e.g., TaqMan^{®}) that requires no further manipulations after the PCR step (Eads et al., Cancer Res. 59:2302-2306, 1999). Briefly, the MethyLight^{™} process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed in a "biased" reaction, e.g., with PCR primers that overlap known CpG dinucleotides. Sequence discrimination occurs both at the level of the amplification process and at the level of the fluorescence detection process.

The MethyLight^{™} assay is used as a quantitative test for methylation patterns in a nucleic acid, e.g., a genomic DNA sample, wherein sequence discrimination occurs at the level of probe hybridization. In a quantitative version, the PCR reaction provides for a methylation specific amplification in the presence of a fluorescent probe that overlaps a particular putative methylation site. An unbiased control for the amount of input DNA is provided by a reaction in which neither the primers, nor the probe, overlie any CpG dinucleotides. Alternatively, a qualitative test for genomic methylation is achieved by probing the biased PCR pool with either control oligonucleotides that do not cover known methylation sites (e.g., a fluorescence-based version of the HeavyMethyl^{™} and MSP techniques) or with oligonucleotides covering potential methylation sites.

The MethyLight^{™} process is used with any suitable probe (e.g. a "TaqMan^{®}" probe, a Lightcycler^{®} probe, etc.) For example, in some applications double-stranded genomic DNA is treated with sodium bisulfite and subjected to one of two sets of PCR reactions using TaqMan^{®} probes, e.g., with MSP primers and/or HeavyMethyl blocker oligonucleotides and a TaqMan^{®} probe. The TaqMan^{®} probe is dual-labeled with fluorescent "reporter" and "quencher" molecules and is designed to be specific for a relatively high GC content region so that it melts at about a 10°C higher temperature in the PCR cycle than the forward or reverse primers. This allows the TaqMan^{®} probe to remain fully hybridized during the PCR annealing/extension step. As the Taq polymerase enzymatically synthesizes a new strand during PCR, it will eventually reach the annealed TaqMan^{®} probe. The Taq polymerase 5' to 3' endonuclease activity will then displace the TaqMan^{®} probe by digesting it to release the fluorescent reporter molecule for quantitative detection of its now unquenched signal using a real-time fluorescent detection system.

Typical reagents (e.g., as might be found in a typical MethyLight^{™}-based kit) for MethyLight^{™} analysis may include, but are not limited to: PCR primers for specific loci (e.g., specific genes, markers, DMR, regions of genes, regions of markers, bisulfite treated DNA sequence, CpG island, etc.); TaqMan^{®} or Lightcycler^{®} probes; optimized PCR buffers and deoxynucleotides; and Taq polymerase.

The QM^{™} (quantitative methylation) assay is an alternative quantitative test for methylation patterns in genomic DNA samples, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe, overlie any CpG dinucleotides. Alternatively, a qualitative test for genomic methylation is achieved by probing the biased PCR pool with either control oligonucleotides that do not cover known methylation sites (a fluorescence-based version of the HeavyMethyl^{™} and MSP techniques) or with oligonucleotides covering potential methylation sites.

The QM^{™} process can be used with any suitable probe, e.g., "TaqMan^{®}" probes, Lightcycler^{®} probes, in the amplification process. For example, double-stranded genomic DNA is treated with sodium bisulfite and subjected to unbiased primers and the TaqMan^{®} probe. The TaqMan^{®} probe is dual-labeled with fluorescent "reporter" and "quencher" molecules, and is designed to be specific for a relatively high GC content region so that it melts out at about a 10°C higher temperature in the PCR cycle than the forward or reverse primers. This allows the TaqMan^{®} probe to remain fully hybridized during the PCR annealing/extension step. As the Taq polymerase enzymatically synthesizes a new strand during PCR, it will eventually reach the annealed TaqMan^{®} probe. The Taq polymerase 5' to 3' endonuclease activity will then displace the TaqMan^{®} probe by digesting it to release the fluorescent reporter molecule for quantitative detection of its now unquenched signal using a real-time fluorescent detection system. Typical reagents (e.g., as might be found in a typical QM^{™}-based kit) for QM^{™} analysis may include, but are not limited to: PCR primers for specific loci (e.g., specific genes, markers, DMR, regions of genes, regions of markers, bisulfite treated DNA sequence, CpG island, etc.); TaqMan^{®} or Lightcycler^{®} probes; optimized PCR buffers and deoxynucleotides; and Taq polymerase.

The Ms-SNuPE^{™} technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site of interest. Small amounts of DNA can be analyzed (e.g., microdissected pathology sections) and it avoids utilization of restriction enzymes for determining the methylation status at CpG sites.

Typical reagents (e.g., as might be found in a typical Ms-SNuPE^{™}-based kit) for Ms-SNuPE^{™} analysis may include, but are not limited to: PCR primers for specific loci (e.g., specific genes, markers, DMR, regions of genes, regions of markers, bisulfite treated DNA sequence, CpG island, etc.); optimized PCR buffers and deoxynucleotides; gel extraction kit; positive control primers; Ms-SNuPE^{™} primers for specific loci; reaction buffer (for the Ms-SNuPE reaction); and labeled nucleotides. Additionally, bisulfite conversion reagents may include: DNA denaturation buffer; sulfonation buffer; DNA recovery reagents or kit (e.g., precipitation, ultrafiltration, affinity column); desulfonation buffer; and DNA recovery components.

Reduced Representation Bisulfite Sequencing (RRBS) begins with bisulfite treatment of nucleic acid to convert all unmethylated cytosines to uracil, followed by restriction enzyme digestion (e.g., by an enzyme that recognizes a site including a CG sequence such as MspI) and complete sequencing of fragments after coupling to an adapter ligand. The choice of restriction enzyme enriches the fragments for CpG dense regions, reducing the number of redundant sequences that may map to multiple gene positions during analysis. As such, RRBS reduces the complexity of the nucleic acid sample by selecting a subset (e.g., by size selection using preparative gel electrophoresis) of restriction fragments for sequencing. As opposed to whole-genome bisulfite sequencing, every fragment produced by the restriction enzyme digestion contains DNA methylation information for at least one CpG dinucleotide. As such, RRBS enriches the sample for promoters, CpG islands, and other genomic features with a high frequency of restriction enzyme cut sites in these regions and thus provides an assay to assess the methylation state of one or more genomic loci.

A typical protocol for RRBS comprises the steps of digesting a nucleic acid sample with a restriction enzyme such as MspI, filling in overhangs and A-tailing, ligating adaptors, bisulfite conversion, and PCR. See, e.g., et al. (2005) "Genome-scale DNA methylation mapping of clinical samples at single-nucleotide resolution" Nat Methods 7: 133-6; Meissner et al. (2005) "Reduced representation bisulfite sequencing for comparative high-resolution DNA methylation analysis" Nucleic Acids Res. 33: 5868-77.

In some embodiments, a quantitative allele-specific real-time target and signal amplification (QuARTS) assay is used to evaluate methylation state. Three reactions sequentially occur in each QuARTS assay, including amplification (reaction 1) and target probe cleavage (reaction 2) in the primary reaction; and FRET cleavage and fluorescent signal generation (reaction 3) in the secondary reaction. When target nucleic acid is amplified with specific primers, a specific detection probe with a flap sequence loosely binds to the amplicon. The presence of the specific invasive oligonucleotide at the target binding site causes cleavase to release the flap sequence by cutting between the detection probe and the flap sequence. The flap sequence is complementary to a nonhairpin portion of a corresponding FRET cassette. Accordingly, the flap sequence functions as an invasive oligonucleotide on the FRET cassette and effects a cleavage between the FRET cassette fluorophore and a quencher, which produces a fluorescent signal. The cleavage reaction can cut multiple probes per target and thus release multiple fluorophore per flap, providing exponential signal amplification. QuARTS can detect multiple targets in a single reaction well by using FRET cassettes with different dyes. See, e.g., in Zou et al. (2010) "Sensitive quantification of methylated markers with a novel methylation specific technology" Clin Chem 56: A199; U.S. Pat. Appl. Ser. Nos. 12/946,737, 12/946,745, 12/946,752, and 61/548,639.

The term "bisulfite reagent" refers to a reagent comprising bisulfite, disulfite, hydrogen sulfite, or combinations thereof, useful as disclosed herein to distinguish between methylated and unmethylated CpG dinucleotide sequences. Methods of said treatment are known in the art (e.g., PCT/EP2004/011715). It is preferred that the bisulfite treatment is conducted in the presence of denaturing solvents such as but not limited to n-alkylenglycol or diethylene glycol dimethyl ether (DME), or in the presence of dioxane or dioxane derivatives. In some embodiments the denaturing solvents are used in concentrations between 1% and 35% (v/v). In some embodiments, the bisulfite reaction is carried out in the presence of scavengers such as but not limited to chromane derivatives, e.g., 6-hydroxy-2,5,7,8,-tetramethylchromane 2-carboxylic acid or trihydroxybenzone acid and derivates thereof, e.g., Gallic acid (see: PCT/EP2004/011715). The bisulfite conversion is preferably carried out at a reaction temperature between 30°C and 70°C, whereby the temperature is increased to over 85°C for short times during the reaction (see: PCT/EP2004/011715). The bisulfite treated DNA is preferably purified prior to the quantification. This may be conducted by any means known in the art, such as but not limited to ultrafiltration, e.g., by means of Microcon^{™} columns (manufactured by Millipore^{™}). The purification is carried out according to a modified manufacturer's protocol (see, e.g., PCT/EP2004/011715).

In some embodiments, fragments of the treated DNA are amplified using sets of primer oligonucleotides according to the present invention (e.g., see Table 2) and an amplification enzyme. The amplification of several DNA segments can be carried out simultaneously in one and the same reaction vessel. Typically, the amplification is carried out using a polymerase chain reaction (PCR). Amplicons are typically 100 to 2000 base pairs in length.

In another embodiment of the method, the methylation status of CpG positions within or near a marker comprising a DMR (e.g., DMR 1-400; Tables 1 and 4) may be detected by use of methylation-specific primer oligonucleotides. This technique (MSP) has been described in U.S. Pat. No. 6,265,171 to Herman. The use of methylation status specific primers for the amplification of bisulfite treated DNA allows the differentiation between methylated and unmethylated nucleic acids. MSP primer pairs contain at least one primer that hybridizes to a bisulfite treated CpG dinucleotide. Therefore, the sequence of said primers comprises at least one CpG dinucleotide. MSP primers specific for non-methylated DNA contain a "T" at the position of the C position in the CpG.

The fragments obtained by means of the amplification can carry a directly or indirectly detectable label. In some embodiments, the labels are fluorescent labels, radionuclides, or detachable molecule fragments having a typical mass that can be detected in a mass spectrometer. Where said labels are mass labels, some embodiments provide that the labeled amplicons have a single positive or negative net charge, allowing for better delectability in the mass spectrometer. The detection may be carried out and visualized by means of, e.g., matrix assisted laser desorption/ionization mass spectrometry (MALDI) or using electron spray mass spectrometry (ESI).

Methods for isolating DNA suitable for these assay technologies are known in the art. In particular, some embodiments comprise isolation of nucleic acids as described in U.S. Pat. Appl. Ser. No. 13/470,251 ("Isolation of Nucleic Acids").

### Methods

In some embodiments the technology, methods are provided that comprise the following steps:
1) contacting a nucleic (e.g., genomic DNA, e.g., isolated from a body fluids such as a blood sample (e.g., a plasma sample), a stool sample, or a tissue sample) obtained from a subject with at least one reagent or series of reagents that distinguishes between methylated and non-methylated CpG dinucleotides within at least one marker comprising a DMR (e.g., DMR 1-400 (from Tables 1 and 4)) and
2) detecting a lack of HCC (e.g., afforded with a sensitivity of greater than or equal to 80% and a specificity of greater than or equal to 80%).

In some embodiments the technology, methods are provided that comprise the following steps:
1) contacting a nucleic acid (e.g., genomic DNA, e.g., isolated from a body fluids such as a blood sample (e.g., a plasma sample), a stool sample, or a tissue sample) obtained from a subject with at least one reagent or series of reagents that distinguishes between methylated and non-methylated CpG dinucleotides within at least one marker comprising a DMR (e.g., DMR 1-400 (from Tables 1 and 4)) and
2) classifying HCC (e.g., afforded with a sensitivity of greater than or equal to 80% and a specificity of greater than or equal to 80%).
Preferably, the sensitivity is from about 70% to about 100%, or from about 80% to about 90%, or from about 80% to about 85%. Preferably, the specificity is from about 70% to about 100%, or from about 80% to about 90%, or from about 80% to about 85%.

Genomic DNA may be isolated by any means, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction, or binding of the DNA to a solid phase support. The choice of method will be affected by several factors including time, expense, and required quantity of DNA. All clinical sample types comprising neoplastic matter or pre-neoplastic matter are suitable for use in the present method, e.g., cell lines, histological slides, biopsies, paraffin-embedded tissue, body fluids, stool, colonic effluent, urine, blood plasma, blood serum, whole blood, isolated blood cells, cells isolated from the blood, and combinations thereof.

The technology is not limited in the methods used to prepare the samples and provide a nucleic acid for testing. For example, in some embodiments, a DNA is isolated from a stool sample or from blood or from a plasma sample using direct gene capture, e.g., as detailed in U.S. Pat. Appl. Ser. No. 61/485386 or by a related method.

The genomic DNA sample is then treated with at least one reagent, or series of reagents, that distinguishes between methylated and non-methylated CpG dinucleotides within at least one marker comprising a DMR (e.g., DMR 1-400, e.g., as provided by Tables 1 and 4).

In some embodiments, the reagent converts cytosine bases which are unmethylated at the 5'-position to uracil, thymine, or another base which is dissimilar to cytosine in terms of hybridization behavior. However in some embodiments, the reagent may be a methylation sensitive restriction enzyme.

In some embodiments, the genomic DNA sample is treated in such a manner that cytosine bases that are unmethylated at the 5' position are converted to uracil, thymine, or another base that is dissimilar to cytosine in terms of hybridization behavior. In some embodiments, this treatment is carried out with bisulfate (hydrogen sulfite, disulfite) followed byt alkaline hydrolysis.

The treated nucleic acid is then analyzed to determine the methylation state of the target gene sequences (at least one gene, genomic sequence, or nucleotide from a marker comprising a DMR, e.g., at least one DMR chosen from DMR 1-400, e.g., as provided in Tables 1 and 4). The method of analysis may be selected from those known in the art, including those listed herein, e.g., QuARTS and MSP as described herein.

The technology relates to the analysis of any sample associated with HCC. For example, in some embodiments the sample comprises a plasma sample from a patient. In some embodiments, the sample comprises a tissue and/or biological fluid obtained from a patient. In some embodiments, the sample comprises liver tissue. In some embodiments, the sample comprises a secretion. In some embodiments, the sample comprises blood, plasma and/or serum. In some embodiments, the subject is human. These samples may originate from the upper gastrointestinal tract, the lower gastrointestinal tract, or comprise cells, tissues, and/or secretions from both the upper gastrointestinal tract and the lower gastrointestinal tract. The sample may include cells, secretions, or tissues from the liver, bile ducts, pancreas, stomach, colon, rectum, esophagus, small intestine, appendix, duodenum, polyps, gall bladder, anus, and/or peritoneum. In some embodiments, the sample comprises cellular fluid, ascites, urine, feces, pancreatic fluid, fluid obtained during endoscopy, blood, mucus, or saliva. In some embodiments, the sample is a stool sample.

Such samples can be obtained by any number of means known in the art, such as will be apparent to the skilled person. For instance, urine and fecal samples are easily attainable, while blood, ascites, serum, or pancreatic fluid samples can be obtained parenterally by using a needle and syringe, for instance. Cell free or substantially cell free samples can be obtained by subjecting the sample to various techniques known to those of skill in the art which include, but are not limited to, centrifugation and filtration. Although it is generally preferred that no invasive techniques are used to obtain the sample, it still may be preferable to obtain samples such as tissue homogenates, tissue sections, and biopsy specimens.

In some embodiments of the technology, a method for diagnosing HCC in a subject is provided. The terms "diagnosing" and "diagnosis" as used herein refer to methods by which the skilled artisan can estimate and even determine whether or not a subject is suffering from a given disease or condition or may develop a given disease or condition in the future. The skilled artisan often makes a diagnosis on the basis of one or more diagnostic indicators, such as for example a biomarker (e.g., a DMR as disclosed herein), the methylation state of which is indicative of the presence, severity, or absence of the condition.

Along with diagnosis, clinical cancer prognosis (e.g., for HCC) relates to determining the aggressiveness of the cancer and the likelihood of tumor recurrence to plan the most effective therapy. If a more accurate prognosis can be made or even a potential risk for developing the cancer can be assessed, appropriate therapy, and in some instances less severe therapy for the patient can be chosen. Assessment (e.g., determining methylation state) of cancer biomarkers is useful to separate subjects with good prognosis and/or low risk of developing cancer who will need no therapy or limited therapy from those more likely to develop cancer or suffer a recurrence of cancer who might benefit from more intensive treatments.

As such, "making a diagnosis" or "diagnosing", as used herein, is further inclusive of making determining a risk of developing cancer or determining a prognosis, which can provide for predicting a clinical outcome (with or without medical treatment), selecting an appropriate treatment (or whether treatment would be effective), or monitoring a current treatment and potentially changing the treatment, based on the measure of the diagnostic biomarkers (e.g., DMR) disclosed herein. Further, in some embodiments of the presently disclosed subject matter, multiple determinations of the biomarkers over time can be made to facilitate diagnosis and/or prognosis. A temporal change in the biomarker can be used to predict a clinical outcome, monitor the progression of HCC, and/or monitor the efficacy of appropriate therapies directed against the cancer. In such an embodiment for example, one might expect to see a change in the methylation state of one or more biomarkers (e.g., DMR) disclosed herein (and potentially one or more additional biomarker(s), if monitored) in a biological sample over time during the course of an effective therapy.

The presently disclosed subject matter further provides in some embodiments a method for determining whether to initiate or continue prophylaxis or treatment of HCC in a subject. In some embodiments, the method comprises providing a series of biological samples over a time period from the subject; analyzing the series of biological samples to determine a methylation state of at least one biomarker disclosed herein in each of the biological samples; and comparing any measurable change in the methylation states of one or more of the biomarkers in each of the biological samples. Any changes in the methylation states of biomarkers over the time period can be used to predict risk of developing HCC, predict clinical outcome, determine whether to initiate or continue the prophylaxis or therapy of the cancer, and whether a current therapy is effectively treating the HCC. For example, a first time point can be selected prior to initiation of a treatment and a second time point can be selected at some time after initiation of the treatment. Methylation states can be measured in each of the samples taken from different time points and qualitative and/or quantitative differences noted. A change in the methylation states of the biomarker levels from the different samples can be correlated with risk (e.g., risk of HCC), prognosis, determining treatment efficacy, and/or progression of the disorder in the subject.

In preferred embodiments, the methods and compositions of the invention are for treatment or diagnosis of disease at an early stage, for example, before symptoms of the disease appear. In some embodiments, the methods and compositions of the invention are for treatment or diagnosis of disease at a clinical stage.

As noted, in some embodiments, multiple determinations of one or more diagnostic or prognostic biomarkers can be made, and a temporal change in the marker can be used to determine a diagnosis or prognosis. For example, a diagnostic marker can be determined at an initial time, and again at a second time. In such embodiments, an increase in the marker from the initial time to the second time can be diagnostic of a particular type or severity of the disorder, or a given prognosis. Likewise, a decrease in the marker from the initial time to the second time can be indicative of a particular type or severity of a disorder, or a given prognosis. Furthermore, the degree of change of one or more markers can be related to the severity of the disorder and future adverse events. The skilled artisan will understand that, while in certain embodiments comparative measurements can be made of the same biomarker at multiple time points, one can also measure a given biomarker at one time point, and a second biomarker at a second time point, and a comparison of these markers can provide diagnostic information.

As used herein, the phrase "determining the prognosis" refers to methods by which the skilled artisan can predict the course or outcome of a condition in a subject. The term "prognosis" does not refer to the ability to predict the course or outcome of a condition with 100% accuracy, or even that a given course or outcome is predictably more or less likely to occur based on the methylation state of a biomarker (e.g., a DMR). Instead, the skilled artisan will understand that the term "prognosis" refers to an increased probability that a certain course or outcome will occur; that is, that a course or outcome is more likely to occur in a subject exhibiting a given condition, when compared to those individuals not exhibiting the condition. For example, in individuals not exhibiting the condition (e.g., having a normal methylation state of one or more DMR), the chance of a given outcome may be very low.

In some embodiments, a statistical analysis associates a prognostic indicator with a predisposition to an adverse outcome. For example, in some embodiments, a methylation state different from that in a normal control sample obtained from a patient who does not have a disorder can signal that a subject is more likely to suffer from a disorder than subjects with a level that is more similar to the methylation state in the control sample, as determined by a level of statistical significance. Additionally, a change in methylation state from a baseline (e.g., "normal") level can be reflective of subject prognosis, and the degree of change in methylation state can be related to the severity of adverse events. Statistical significance is often determined by comparing two or more populations and determining a confidence interval and/or a p value (see, e.g., Dowdy and Wearden, Statistics for Research, John Wiley & Sons, New York, 1983). Exemplary confidence intervals of the present subject matter are 90%, 95%, 97.5%, 98%, 99%, 99.5%, 99.9% and 99.99%, while exemplary p values are 0.1, 0.05, 0.025, 0.02, 0.01, 0.005, 0.001, and 0.0001.

In other embodiments, a threshold degree of change in the methylation state of a prognostic or diagnostic biomarker disclosed herein (e.g., a DMR) can be established, and the degree of change in the methylation state of the biomarker in a biological sample is simply compared to the threshold degree of change in the methylation state. A preferred threshold change in the methylation state for biomarkers provided herein is about 5%, about 10%, about 15%, about 20%, about 25%, about 30%, about 50%, about 75%, about 100%, and about 150%. In yet other embodiments, a "nomogram" can be established, by which a methylation state of a prognostic or diagnostic indicator (biomarker or combination of biomarkers) is directly related to an associated disposition towards a given outcome. The skilled artisan is acquainted with the use of such nomograms to relate two numeric values with the understanding that the uncertainty in this measurement is the same as the uncertainty in the marker concentration because individual sample measurements are referenced, not population averages.

In some embodiments, a control sample is analyzed concurrently with the biological sample, such that the results obtained from the biological sample can be compared to the results obtained from the control sample. Additionally, it is contemplated that standard curves can be provided, with which assay results for the biological sample may be compared. Such standard curves present methylation states of a biomarker as a function of assay units, e.g., fluorescent signal intensity, if a fluorescent label is used. Using samples taken from multiple donors, standard curves can be provided for control methylation states of the one or more biomarkers in normal tissue, as well as for "at-risk" levels of the one or more biomarkers in tissue taken from donors with metaplasia or from donors with a disorder (e.g., HCC). In certain embodiments of the method, a subject is identified as having HCC upon identifying an aberrant methylation state of one or more DMR provided herein in a biological sample obtained from the subject. In other embodiments of the method, the detection of an aberrant methylation state of one or more of such biomarkers in a biological sample obtained from the subject results in the subject being identified as having HCC.

The analysis of markers can be carried out separately or simultaneously with additional markers within one test sample. For example, several markers can be combined into one test for efficient processing of a multiple of samples and for potentially providing greater diagnostic and/or prognostic accuracy. In addition, one skilled in the art would recognize the value of testing multiple samples (for example, at successive time points) from the same subject. Such testing of serial samples can allow the identification of changes in marker methylation states over time. Changes in methylation state, as well as the absence of change in methylation state, can provide useful information about the disease status that includes, but is not limited to, identifying the approximate time from onset of the event, the presence and amount of salvageable tissue, the appropriateness of drug therapies, the effectiveness of various therapies, and identification of the subject's outcome, including risk of future events.

The analysis of biomarkers can be carried out in a variety of physical formats. For example, the use of microtiter plates or automation can be used to facilitate the processing of large numbers of test samples. Alternatively, single sample formats could be developed to facilitate immediate treatment and diagnosis in a timely fashion, for example, in ambulatory transport or emergency room settings.

In some embodiments, the subject is diagnosed as having HCC if, when compared to a control methylation state, there is a measurable difference in the methylation state of at least one biomarker in the sample. Conversely, when no change in methylation state is identified in the biological sample, the subject can be identified as not HCC, not being at risk for HCC, or as having a low risk of HCC. In this regard, subjects having HCC or risk thereof can be differentiated from subjects having low to substantially no HCC or risk thereof. Those subjects having a risk of developing HCC can be placed on a more intensive and/or regular screening schedule.

As mentioned above, depending on the embodiment of the method of the present technology, detecting a change in methylation state of the one or more biomarkers can be a qualitative determination or it can be a quantitative determination. As such, the step of diagnosing a subject as having, or at risk of developing, HCC indicates that certain threshold measurements are made, e.g., the methylation state of the one or more biomarkers in the biological sample varies from a predetermined control methylation state. In some embodiments of the method, the control methylation state is any detectable methylation state of the biomarker. In other embodiments of the method where a control sample is tested concurrently with the biological sample, the predetermined methylation state is the methylation state in the control sample. In other embodiments of the method, the predetermined methylation state is based upon and/or identified by a standard curve. In other embodiments of the method, the predetermined methylation state is a specifically state or range of state. As such, the predetermined methylation state can be chosen, within acceptable limits that will be apparent to those skilled in the art, based in part on the embodiment of the method being practiced and the desired specificity, etc.

Further with respect to diagnostic methods, a preferred subject is a vertebrate subject. A preferred vertebrate is warm-blooded; a preferred warm-blooded vertebrate is a mammal. A preferred mammal is most preferably a human. As used herein, the term "subject' includes both human and animal subjects. Thus, veterinary therapeutic uses are provided herein. As such, the present technology provides for the diagnosis of mammals such as humans, as well as those mammals of importance due to being endangered, such as Siberian tigers; of economic importance, such as animals raised on farms for consumption by humans; and/or animals of social importance to humans, such as animals kept as pets or in zoos. Examples of such animals include but are not limited to: carnivores such as cats and dogs; swine, including pigs, hogs, and wild boars; ruminants and/or ungulates such as cattle, oxen, sheep, giraffes, deer, goats, bison, and camels; and horses. Thus, also provided is the diagnosis and treatment of livestock, including, but not limited to, domesticated swine, ruminants, ungulates, horses (including race horses), and the like. The presently-disclosed subject matter further includes a system for diagnosing HCC in a subject. The system can be provided, for example, as a commercial kit that can be used to screen for a risk of such a disorder in a subject from whom a biological sample has been collected. An exemplary system provided in accordance with the present technology includes assessing the methylation state of a DMR as provided in Tables 1 and/or 4.

### EXAMPLES

### Example I.

This example describes the identification of 311 differentially methylated regions (DMRs) for discriminating HCC DNA samples from DNA derived from normal controls (e.g., non-HCC individuals with or without liver cirrhosis).

Experiments were conducted in four phases.

First, DNA methylation marker discovery was performed using RRBS on DNA extracted from frozen tumor HCC tissues (with and without cirrhosis) and from frozen normal liver tissues (with and without cirrhosis) and buffy coat samples from healthy volunteers. Discriminant differentially methylated regions (DMRs) were identified by strict filtration criteria and re-assayed on either the same or expanded specimens to ensure reproducibility of results using a real-time methylation specific quantitative PCR assay (qMSP) (technical validation).

Second, candidate markers were selected by further ranking criteria for biological validation by blinded qMSP assays on DNA extracted from independent archival case and control tissues.

Third, the sequencing results for the candidate markers were compared across a pan-GI RRBS sequencing set to determine the level of methylation specificity.

Fourth, a separate decision model was applied to select a small set of HCC markers which would best perform in a blood-based environment where the majority of DNA is from non-liver source. Selected markers were then tested in blinded independent plasma samples to assess HCC detection in a clinical medium.

Fig. 1 summarizes these four phases.

### Results

Un-biased whole methylome sequencing was performed on DNA extracted from 18 HCC and 35 control (9 cirrhosis, 26 normal liver) tissues. Through tissue validation, the best DMRs were confirmed in DNA extracted from independent tissues from 75 HCCs and 29 controls (16 cirrhosis, 13 normal liver) using methylation specific PCR. Blinded quantitative allele-specific real time target and signal amplification assays targeting top DMRs were then performed on plasma DNA from independent patient sets comprising 21 HCC cases (9 BCLC [Barcelona Clinic Liver Cancer staging] stage A, 6 stage B, 6 stage C) and 33 cirrhotic controls. Recursive partitioning decision analysis was used to identify best DMR combinations. Initial sequencing identified 311 DMRs with AUCs greater than 0.75. Following biological validation, the top 12 DMRs (ACP1, BDH1, Chr12.133, CLEC11A, DAB2IP, DBNL, EMX1, EFNB2, HOXA1, LRRC4, SPINT2, TSPYL5, CCNJ_3707, CCNJ_3124, PFKP, SCRN1, and ECE1) were selected to carry forward to plasma testing. The most discriminant marker in plasma, EMX1, alone had an AUC of 0.89. A complementary 3-marker combination (EMX1, LRRC4 and BDH1) identified 20/21 HCC and 32/33 controls in plasma; 1 HCC had low levels of BDH1 and 1 control had elevated LRRC4. The panel was 95% sensitive (95% CI, 74%-100%) for HCC at 97% specificity (95% CI, 82%-100%) and achieved an AUC of 0.98 (see, Fig. 2). Area under the receiver operating characteristic curve information for ACP1, Chr12.133, CLEC11A, DAB2IP, DBNL, EMX1, HOXA1, LRRC4, SPINT2, and TSPYL5 from the biological tissue validation phase is provided at Fig. 3A-I.

Fig. 4A-CC provide box plots (log scale) of 27 gastric cancer markers (29 with fit point analyses added) from biological tissue validation data. Samples are arranged with normal liver on the far left, followed by HCC w/o cirrhosis, HCC w/ cirrhosis, and cirrhosis controls (Inflammatory). The vertical axis is fractional methylation (normalized to β-actin strands).

Fig. 5 shows the performance of 27 HCC cancer markers in 75 HCC tissue samples and 29 controls (16 cirrhosis, 13 normal liver) in a matrix format at 95% normal specificity. Markers are listed vertically and samples horizontally. Samples are arranged with normal liver (NI) on the far left, followed by HCC w/o cirrhosis (HN), HCC w/ cirrhosis (HC), and cirrhosis controls (In). Positive hits are in light grey and misses in dark grey. This plot allows markers to be assessed in a complementary fashion. Note: 2 markers, TBX15 and EGR2, were analyzed a second time using a fit points method on the qMSP data and are included here.

Table 1 provides information for DMRs distinguishing HCC from normal controls.

Table 2 provides primer information for selected DMRs for from Table 1.

Table 3 provides AUC and fold-change information for specific DMRs in the comparison between HCC versus normal liver, wherein fold-change is the ratio of the fractional methylation of the cases over the fractional methylation of the controls.

**Table 1.**

| **DMR No.** | **Gene Annotation** | **Chromosome No.** | **DMR Start and End Position** |
|---|---|---|---|
| 1 | Septin9 | chr17 | 75368812-75368887 |
| 2 | Septin9 | chr17 | 75369228-75369313 |
| 3 | Septin9 | chr17 | 75368948-75369052 |
| 4 | ACP1 | chr2 | 264204-264250 |
| 5 | ACP1 | chr2 | 264087-264151 |
| 6 | ACTG1 | chr17 | 79481554-79481612 |
| 7 | AKR1B1 | chr7 | 134143298-134143341 |
| 8 | AKR1B1 | chr7 | 134143650-134143684 |
| 9 | AKR1B1 | chr7 | 134143461-134143500 |
| 10 | ALDH1A3 | chr15 | 101419689-101419707 |
| 11 | ALDH1A3 | chr15 | 101419988-101420013 |
| 12 | ANKRD33B | chr5 | 10564710-10564793 |
| 13 | ANKRD34B | chr5 | 79866119-79866169 |
| 14 | ARHGEF19 | chr1 | 16533241-16533318 |
| 15 | ARL4C | chr2 | 235404686-235404738 |
| 16 | ATL1 | chr14 | 51027344-51027417 |
| 17 | B3GNT4 | chr12 | 122688498-122688504 |
| 18 | B3GNT4 | chr12 | 122688516-122688554 |
| 19 | B3GNT5 | chr3 | 182971710-182971817 |
| 20 | B3GNT5 | chr3 | 182971523-182971580 |
| 21 | BAALC | chr8 | 104153072-104153134 |
| 22 | BACE2 | chr21 | 42539722-42539743 |
| 23 | BACE2 | chr21 | 42539915-42539974 |
| 24 | BLVRB | chr19 | 40973164-40973202 |
| 25 | BLVRB | chr19 | 40973019-40973041 |
| 26 | BMP6 | chr6 | 7727949-7728013 |
| 27 | BMP6 | chr6 | 7726324-7726384 |
| 28 | BVES | chr6 | 105584689-105584756 |
| 29 | BVES | chr6 | 105584565-105584590 |
| 30 | BVES | chr6 | 105584763-105584789 |
| 31 | C6orf126 | chr6 | 35744417-35744467 |
| 32 | C6orf174 | chr6 | 127837271-127837331 |
| 33 | C7orf51 | chr7 | 100091317-100091392 |
| 34 | C7orf57 | chr7 | 48075209-48075261 |
| 35 | CATSPERG | chr19 | 38852416-38852441 |
| 36 | CATSPERG | chr19 | 38853253-38853336 |
| 37 | CCDC48 | chr3 | 128745984-128746119 |
| 38 | CCNI2 | chr5 | 132082945-132083024 |
| 39 | CCNJ | chr10 | 97803226-97803325 |
| 40 | CCNJ | chr10 | 97803156-97803203 |
| 41 | CCNJ | chr10 | 97803124-97803152 |
| 42 | CCNJ | chr10 | 97803707-97803733 |
| 43 | CDKN2A | chr9 | 21974872-21974890 |
| 44 | CDKN2A | chr9 | 21974943-21975018 |
| 45 | CELF2 | chr10 | 11059845-11059861 |
| 46 | CELF2 | chr10 | 11059991-11060017 |
| 47 | CELSR3 | chr3 | 48693898-48693974 |
| 48 | CHST2 | chr3 | 142839074-142839107 |
| 49 | CHST2 | chr3 | 142839408-142839457 |
| 50 | CHST2 | chr3 | 142838525-142838580 |
| 51 | CHST2 | chr3 | 142838038-142838164 |
| 52 | CHST2 | chr3 | 142838701-142838753 |
| 53 | CHST2 | chr3 | 142838686-142838696 |
| 54 | CHST2 | chr3 | 142839245-142839275 |
| 55 | CHST2 | chr3 | 142838916-142838930 |
| 56 | CLIP4 | chr2 | 29338189-29338335 |
| 57 | CORO2B | chr15 | 68871554-68871567 |
| 58 | CORO2B | chr15 | 68871572-68871581 |
| 59 | CORO2B | chr15 | 68871623-68871639 |
| 60 | CORO2B | chr15 | 68871190-68871224 |
| 61 | CRHR2 | chr7 | 30722095-30722147 |
| 62 | DCLK2 | chr4 | 150999533-150999622 |
| 63 | DCLK2 | chr4 | 150999775-150999832 |
| 64 | DGCR14 | chr22 | 19137109-19137145 |
| 65 | DGCR14 | chr22 | 19137067-19137086 |
| 66 | DKFZp686O24166 | chr11 | 17373279-17373346 |
| 67 | DNAJB6 | chr7 | 157129265-157129325 |
| 68 | DUSP4 | chr8 | 29207072-29207112 |
| 69 | DUSP4 | chr8 | 29207421-29207440 |
| 70 | ECE1 | chr1 | 21616801-21616877 |
| 71 | EFNB2 | chr13 | 107188064-107188108 |
| 72 | EFNB2 | chr13 | 107188196-107188212 |
| 73 | EFNB2 | chr13 | 107187748-107187766 |
| 74 | EFNB2 | chr13 | 107187768-107187785 |
| 75 | EFNB2 | chr13 | 107188435-107188478 |
| 76 | EFNB2 | chr13 | 107188935-107189019 |
| 77 | EFNB2 | chr13 | 107188790-107188904 |
| 78 | EFNB2 | chr13 | 107187702-107187716 |
| 79 | EFNB2 | chr13 | 107188503-107188558 |
| 80 | EGR2 | chr10 | 64575092-64575141 |
| 81 | EHD3 | chr2 | 31456843-31456921 |
| 82 | EHD3 | chr2 | 31457043-31457061 |
| 83 | EIF4E3 | chr3 | 71803308-71803340 |
| 84 | EIF4E3 | chr3 | 71803236-71803290 |
| 85 | EMILIN2 | chr18 | 2847067-2847090 |
| 86 | EMILIN2 | chr18 | 2847092-2847100 |
| 87 | EMILIN2 | chr18 | 2847233-2847274 |
| 88 | FAHD2B | chr2 | 97760723-97760782 |
| 89 | FAM105A | chr5 | 14582008-14582040 |
| 90 | FAM105A | chr5 | 14582046-14582064 |
| 91 | FAM105A | chr5 | 14582380-14582417 |
| 92 | FAM55C | chr3 | 101498249-101498337 |
| 93 | FBXL19 | chr16 | 30935481-30935570 |
| 94 | FCHSD1 | chr5 | 141031071-141031122 |
| 95 | FCHSD1 | chr5 | 141031057-141031069 |
| 96 | FHOD1 | chr16 | 67281136-67281198 |
| 97 | FHOD1 | chr16 | 67281037-67281085 |
| 98 | FIBCD1 | chr9 | 133815197-133815238 |
| 99 | FIBCD1 | chr9 | 133814914-133814942 |
| 100 | FOXD1 | chr5 | 72743623-72743647 |
| 101 | FOXD1 | chr5 | 72743444-72743467 |
| 102 | FOXD1 | chr5 | 72743469-72743495 |
| 103 | FUT4 | chr11 | 94277618-94277669 |
| 104 | FYN | chr6 | 112194124-112194158 |
| 105 | FYN | chr6 | 112194611-112194633 |
| 106 | GALNT12 | chr9 | 101569940-101569975 |
| 107 | GALNT12 | chr9 | 101570002-101570029 |
| 108 | GNG4 | chr1 | 235813128-235813161 |
| 109 | GNG4 | chr1 | 235813683-235813724 |
| 110 | GPX7 | chr1 | 53068129-53068171 |
| 111 | GPX7 | chr1 | 53068016-53068063 |
| 112 | GPX7 | chr1 | 53068089-53068108 |
| 113 | GRID2IP | chr7 | 6543224-6543246 |
| 114 | GRID2IP | chr7 | 6570807-6570820 |
| 115 | GRID2IP | chr7 | 6543285-6543348 |
| 116 | GRID2IP | chr7 | 6570602-6570650 |
| 117 | HDGFRP3 | chr15 | 83876476-83876501 |
| 118 | HDGFRP3 | chr15 | 83876552-83876612 |
| 119 | HDGFRP3 | chr15 | 83876614-83876641 |
| 120 | HK1 | chr10 | 71078304-71078341 |
| 121 | HK1 | chr10 | 71078661-71078739 |
| 122 | HOXA13 | chr7 | 27239880-27239914 |
| 123 | HOXA13 | chr7 | 27239850-27239876 |
| 124 | HPDL | chr1 | 45792443-45792512 |
| 125 | IKZF1 | chr7 | 50343562-50343603 |
| 126 | IKZF1 | chr7 | 50343493-50343512 |
| 127 | IKZF1 | chr7 | 50343859-50343873 |
| 128 | IKZF1 | chr7 | 50344046-50344106 |
| 129 | IKZF1 | chr7 | 50343990-50344044 |
| 130 | ITPR3 | chr6 | 33601477-33601537 |
| 131 | KCNQ3 | chr8 | 133493278-133493345 |
| 132 | KCNS2 | chr8 | 99439489-99439524 |
| 133 | KCNS2 | chr8 | 99439451-99439464 |
| 134 | KCTD12 | chr13 | 77460548-77460628 |
| 135 | KCTD12 | chr13 | 77460089-77460122 |
| 136 | KCTD12 | chr13 | 77459810-77459868 |
| 137 | KIAA1614 | chr1 | 180881686-180881721 |
| 138 | KIAA1614 | chr1 | 180881734-180881776 |
| 139 | LCNL1 | chr9 | 139872612-139872667 |
| 140 | LGALS3 | chr14 | 55596202-55596277 |
| 141 | LIMD2 | chr17 | 61777597-61777647 |
| 142 | LMO2 | chr11 | 33891092-33891223 |
| 143 | LOC440461 | chr17 | 66195307-66195331 |
| 144 | LOC440461 | chr17 | 66195475-66195486 |
| 145 | LOC440461 | chr17 | 66195649-66195686 |
| 146 | LOC441617 | chr11 | 74953198-74953215 |
| 147 | LOC441617 | chr11 | 74952822-74952901 |
| 148 | LOC441617 | chr11 | 74953185-74953195 |
| 149 | LPAR2 | chr19 | 19739243-19739256 |
| 150 | LPAR2 | chr19 | 19739094-19739141 |
| 151 | LRRC10B | chr11 | 61276689-61276765 |
| 152 | LRRC10B | chr11 | 61276161-61276167 |
| 153 | LRRC34 | chr3 | 169530174-169530220 |
| 154 | LRRC34 | chr3 | 169530317-169530418 |
| 155 | LRRK1 | chr15 | 101459830-101459847 |
| 156 | LRRK1 | chr15 | 101459790-101459821 |
| 157 | LRRN1 | chr3 | 3841285-3841390 |
| 158 | MATK | chr19 | 3785888-3785923 |
| 159 | MATK | chr19 | 3785942-3785986 |
| 160 | MAX.chr1.1535351-1535441 | chr1 | 1535351-1535441 |
| 161 | MAX.chr1.161582155-161582236 | chr1 | 161582155-161582236 |
| 162 | MAX.chr1.227976136-227976180 | chr1 | 227976136-227976180 |
| 163 | MAX.chr1.227976189-227976206 | chr1 | 227976189-227976206 |
| 164 | MAX.chr1.41847970-41848023 | chr1 | 41847970-41848023 |
| 165 | MAX.chr11.1357749-1357833 | chr11 | 1357749-1357833 |
| 166 | MAX.chr11.45377012-45377082 | chr11 | 45377012-45377082 |
| 167 | MAX.chr11.75947388-75947438 | chr11 | 75947388-75947438 |
| 168 | MAX.chr17.1132739-1132794 | chr17 | 1132739-1132794 |
| 169 | MAX.chr19.37288291-37288390 | chr19 | 37288291-37288390 |
| 170 | MAX.chr19.37464150-37464218 | chr19 | 37464150-37464218 |
| 171 | MAX.chr19.9896775-9896833 | chr19 | 9896775-9896833 |
| 172 | MAX.chr2.144694740-144694873 | chr2 | 144694740-144694873 |
| 173 | MAX.chr2.219773668-219773754 | chr2 | 219773668-219773754 |
| 174 | MAX.chr20.62461671-62461721 | chr20 | 62461671-62461721 |
| 175 | MAX.chr3.184243258-184243328 | chr3 | 184243258-184243328 |
| 176 | MAX.chr4.56915281-56915399 | chr4 | 56915281-56915399 |
| 177 | MAX.chr5.178957711-178957760 | chr5 | 178957711-178957760 |
| 178 | MAX.chr6.130686855-130686917 | chr6 | 130686855-130686917 |
| 179 | MAX.chr6.155316859-155316913 | chr6 | 155316859-155316913 |
| 180 | MAX.chr7.139930482-139930532 | chr7 | 139930482-139930532 |
| 181 | MAX.chr9.140024004-140024057 | chr9 | 140024004-140024057 |
| 182 | MAX.chr9.99983940-99983992 | chr9 | 99983940-99983992 |
| 183 | MECOM | chr3 | 169380257-169380286 |
| 184 | MECOM | chr3 | 169380412-169380427 |
| 185 | MEX3B | chr15 | 82339734-82339758 |
| 186 | MEX3B | chr15 | 82339894-82339967 |
| 187 | MGC16703 | chr22 | 21368623-21368674 |
| 188 | MIR3132 | chr2 | 220417386-220417427 |
| 189 | MIR3132 | chr2 | 220417233-220417279 |
| 190 | MIR92B | chr1 | 155164705-155164747 |
| 191 | MIR92B | chr1 | 155164749-155164784 |
| 192 | MNT | chr17 | 2297347-2297397 |
| 193 | N4BP3 | chr5 | 177540980-177541072 |
| 194 | NCRNA00085 | chr19 | 52207589-52207731 |
| 195 | NCRNA00085 | chr19 | 52207475-52207527 |
| 196 | OBSCN | chr1 | 228401498-228401559 |
| 197 | OBSCN | chr1 | 228463603-228463619 |
| 198 | ODF2L | chr1 | 86861515-86861590 |
| 199 | OVOL1 | chr11 | 65554351-65554410 |
| 200 | OXT | chr20 | 3052753-3052809 |
| 201 | PAQR8 | chr6 | 52227453-52227521 |
| 202 | PDE4D | chr5 | 58335632-58335685 |
| 203 | PFKP | chr10 | 3110620-3110694 |
| 204 | PFKP | chr10 | 3110554-3110609 |
| 205 | PFKP | chr10 | 3111174-3111244 |
| 206 | PFKP | chr10 | 3110913-3110978 |
| 207 | PLEKHO1 | chr1 | 150122743-150122798 |
| 208 | PMAIP1 | chr18 | 57567061-57567089 |
| 209 | PMAIP1 | chr18 | 57567094-57567115 |
| 210 | PPP2R5C | chr14 | 102248080-102248126 |
| 211 | PPP2R5C | chr14 | 102248158-102248201 |
| 212 | PRDM13 | chr6 | 100061630-100061691 |
| 213 | PRDM5 | chr4 | 121843466-121843518 |
| 214 | PSMG2 | chr18 | 12658195-12658238 |
| 215 | PSMG2 | chr18 | 12658247-12658264 |
| 216 | RANGRF | chr17 | 8192319-8192372 |
| 217 | RANGRF | chr17 | 8192381-8192390 |
| 218 | RASL11B | chr4 | 53728280-53728354 |
| 219 | RASSF2 | chr20 | 4803295-4803348 |
| 220 | RASSF2 | chr20 | 4803983-4804072 |
| 221 | RECK | chr9 | 36037020-36037061 |
| 222 | RECK | chr9 | 36037093-36037184 |
| 223 | RGS10 | chr10 | 121302301-121302309 |
| 224 | RGS10 | chr10 | 121302871-121302922 |
| 225 | RGS10 | chr10 | 121302655-121302672 |
| 226 | RGS20 | chr8 | 54793664-54793736 |
| 227 | RPP25 | chr15 | 75248658-75248693 |
| 228 | RPP25 | chr15 | 75249650-75249686 |
| 229 | SCRN1 | chr7 | 30029222-30029267 |
| 230 | SCRN1 | chr7 | 30029115-30029160 |
| 231 | SCRN1 | chr7 | 30029187-30029209 |
| 232 | SDK1 | chr7 | 3340834-3340842 |
| 233 | SDK1 | chr7 | 3340543-3340635 |
| 234 | SDK1 | chr7 | 3340737-3340813 |
| 235 | SDK1 | chr7 | 3340869-3340900 |
| 236 | SLC16A3 | chr17 | 80186851-80186883 |
| 237 | SLC16A3 | chr17 | 80186820-80186835 |
| 238 | SLC35F1 | chr6 | 118228406-118228451 |
| 239 | SLC35F1 | chr6 | 118228566-118228578 |
| 240 | SLC35F1 | chr6 | 118228886-118228910 |
| 241 | SLC6A20 | chr3 | 45838034-45838084 |
| 242 | SLC6A6 | chr3 | 14443943-14444020 |
| 243 | SLC7A5 | chr16 | 87902822-87902863 |
| 244 | SLC7A5 | chr16 | 87903100-87903127 |
| 245 | SPNS2 | chr17 | 4403134-4403184 |
| 246 | ST8SIA1 | chr12 | 22487540-22487711 |
| 247 | ST8SIA6 | chr10 | 17496576-17496610 |
| 248 | ST8SIA6 | chr10 | 17496378-17496406 |
| 249 | STK32C | chr10 | 134121022-134121044 |
| 250 | STK32C | chr10 | 134121172-134121183 |
| 251 | STK32C | chr10 | 134122104-134122151 |
| 252 | STK32C | chr10 | 134122249-134122269 |
| 253 | STXBP1 | chr9 | 130370839-130370874 |
| 254 | STXBP1 | chr9 | 130370883-130370925 |
| 255 | SYCE1L | chr16 | 77246378-77246432 |
| 256 | TAF4B | chr18 | 23806638-23806671 |
| 257 | TAF4B | chr18 | 23806718-23806723 |
| 258 | TCF24 | chr8 | 67875000-67875071 |
| 259 | TMEM143 | chr19 | 48837211-48837295 |
| 260 | TMEM163 | chr2 | 135476181-135476192 |
| 261 | TMEM163 | chr2 | 135476038-135476121 |
| 262 | TMEM163 | chr2 | 135476286-135476322 |
| 263 | TRIM17 | chr1 | 228604397-228604446 |
| 264 | TRPC3 | chr4 | 122872963-122873035 |
| 265 | TSC22D1 | chr13 | 45150818-45150847 |
| 266 | TSC22D1 | chr13 | 45150763-45150815 |
| 267 | UAP1L1 | chr9 | 139972155-139972218 |
| 268 | VASH1 | chr14 | 77228066-77228133 |
| 269 | VASH2 | chr1 | 213124546-213124598 |
| 270 | WDR66 | chr12 | 122356382-122356450 |
| 271 | WNT7A | chr3 | 13921575-13921631 |
| 272 | ZC3HAV1L | chr7 | 138720665-138720718 |
| 273 | ZEB2 | chr2 | 145274897-145275040 |
| 274 | ZNF160 | chr19 | 53606314-53606402 |
| 275 | ZNF160 | chr19 | 53606256-53606282 |
| 276 | ZNF160 | chr19 | 53606572-53606659 |
| 277 | ZNF354C | chr5 | 178487210-178487287 |
| 278 | ZNF468 | chr19 | 53360825-53360874 |
| 279 | ZNF506 | chr19 | 19932506-19932566 |
| 280 | ZNF510 | chr9 | 99540238-99540295 |
| 281 | ZNF549 | chr19 | 58038905-58038971 |
| 282 | ZNF568 | chr19 | 37407242-37407355 |
| 283 | ZNF607 | chr19 | 38210327-38210438 |
| 284 | ZNF611 | chr19 | 53238186-53238286 |
| 285 | ZNF671 | chr19 | 58238870-58238942 |
| 286 | ZNF816 | chr19 | 53466145-53466206 |
| 287 | ZNF816 | chr19 | 53466048-53466114 |
| 288 | SPINT2 | chr19 | 38755130-38755164 |
| 289 | DBNL | chr7 | 44080227-44080310 |
| 290 | PRKAG2 | chr7 | 151329671-151329753 |
| 291 | TDH | chr8 | 11205044-11205092 |
| 292 | COTL1 | chr16 | 84651245-84651314 |
| 293 | PHF21B | chr22 | 45403120-45403172 |
| 294 | PHF21B | chr22 | 45404843-45404858 |
| 295 | PHF21B | chr22 | 45405759-45405766 |
| 296 | MCF2L2 | chr3 | 183146162-183146204 |
| 297 | MCF2L2 | chr3 | 183146290-183146355 |
| 298 | MCF2L2 | chr3 | 182897154-182897207 |
| 299 | AMN | chr14 | 103394822-103394849 |
| 300 | MARK1 | chr1 | 220701604-220701619 |
| 301 | MARK1 | chr1 | 220701582-220701599 |
| 302 | MARK1 | chr1 | 220701657-220701686 |
| 303 | EMX1 | chr2 | 73147710-73147772 |
| 304 | TSPYL5 | chr8 | 98289858-98290220 |
| 305 | DAB2IP | chr9 | 124461305-124461420 |
| 306 | CLEC11A | chr19 | 51228217-51228732 |
| 307 | HOXA1 | chr7 | 27136145-27136425 |
| 308 | ADCY1 | chr7 | 45613877-45614572 |
| 309 | DMRTA2 | chr1 | 50884349-50884499 |
| 310 | TBX15 | chr1 | 119527066-119527655 |
| 311 | AK055957 | chr12 | 133484978-133485739 |

**Table 2.**

| **MARKER** | **OLIGO TYPE** | **SEQUENCE** |
|---|---|---|
| TSPYL5 | Primer | GCGCGGGAGGATTTTCG (SEQ ID NO: 1) |
| | Primer | CCGCCACCATAAACGACC (SEQ ID NO: 2) |
| | Probe | CCACGGACG CGAAATCGAAAT/3C6/ (SEQ ID NO: 3) |
| DAB2IP | Primer | CGTTCGTTACGTCGTTTTCGT (SEQ ID NO: 4) |
| | Primer | GATCGACGCGACTCGAC (SEQ ID NO: 5) |
| | Probe | CCACGGACGCTCGACGTCGCC/3C6/ (SEQ ID NO: 6) |
| CLEC11A | Primer | GCGGGAGTTTGGCGTAG (SEQ ID NO: 7) |
| | Primer | CGCGCAAATACCGAATAAACG (SEQ ID NO: 8) |
| | Probe | CCACGGACGGTCGGTAGATCG/3C6/ (SEQ ID NO: 9) |
| ACP1 | Primer | GCGCGTTGTTTCGTTTCG (SEQ ID NO: 10) |
| | Primer | CGTCACCTACCGCAAATACG (SEQ ID NO: 11) |
| | Probe | CCACGGACGGCGGATAAGGAG/3C6/ (SEQ ID NO: 12) |
| BDH1 | Primer | AGTACGTAAGTAGAGCGCG (SEQ ID NO: 13) |
| | Primer | CTAAAATTAACTACGCCGCCGT (SEQ ID NO: 14) |
| | Probe | CCACGGACGGAGAACGTTCGA/3C6/ (SEQ ID NO: 15) |
| EMX1 | Primer | GGCGTCGCGTTTTTTAGAGAA (SEQ ID NO: 16) |
| | Primer | TTCCTTTTCGTTCGTATAAAATTTCGTT (SEQ ID NO: 17) |
| | Probe | CCACGGACGATCGGGTTTTAG/3C6/ (SEQ ID NO: 18) |
| ZF_RASSF1 | Primer | TGCGTATGGTGGGCG AG (SEQ ID NO: 19) |
| | Primer | CCTAATTTACACGTCAACCAATCGAA (SEQ ID NO: 20) |
| | Probe | CCACGGACGGCGCGTGCGTTT/3C6/ (SEQ ID NO: 21) |
| BTACT | Primer | TTTGTTTTTTTGATTAGGTGTTTAAGA (SEQ ID NO: 22) |
| | Primer | CACCAACCTCATAACCTTATC (SEQ ID NO: 23) |
| | Probe | GACGCGGAGATAGTGTTGTGG /3C6/ (SEQ ID NO: 24) |
| AK055957 (chr12.133) | Primer | GCGTTTTAGTTAGATAGGGCGG (SEQ ID NO: 25) |
| | Primer | GAAAACCCCTTCCCCGAAAC (SEQ ID NO: 26) |
| | Probe | CGCCGAGGCGCACGCCTAAA/3C6/ (SEQ ID NO: 27) |
| HOXA1 | Primer | AGTCGTTTTTTTAGGTAGTTTAGGCG (SEQ ID NO: 28) |
| | Primer | CGACCTTTACAATCGCCGC (SEQ ID NO: 29) |
| | Probe | CGCCGAGGGGCGGTAGTTGT/3C6/ (SEQ ID NO: 30) |
| SPINT2 | Primer | GGGAGCGGTCGCGTAG (SEQ ID NO: 31) |
| | Primer | GCACCTAACTAAACAAAACGAACTAAAC (SEQ ID NO: 32) |
| | Probe | CGCCGAGGCGCAAACGCAAA/3C6/ (SEQ ID NO: 33) |
| DBNL | Primer | AGGTGGCGCGTATTACG (SEQ ID NO: 34) |
| | Primer | CCTACTAAACGCGCTCAACC (SEQ ID NO: 35) |
| | Probe | CGCCGAGGCGCTCGATTCCC/3C6/ (SEQ ID NO: 36) |
| EFNB2 | Primer | TTCGATATTGGGTGTCGCG (SEQ ID NO: 37) |
| | Primer | CGCGAAAACCAAAAACGAAAC (SEQ ID NO: 38) |
| | Probe | CGCCGAGG GAGGCGGGGTTC/3C6/ (SEQ ID NO: 39) |
| LRRC4 | Primer | GCGTTAATTTCGCGAGGTA (SEQ ID NO: 40) |
| | Primer | ACAATACTCTTATATATTAACGCCGCTC (SEQ ID NO: 41) |
| | Probe | CGCCGAGGAGGCGACGGAGG/3C6/ (SEQ ID NO: 42) |

**Table 3.**

| **Gene Annotation** | **Area Under Curve** | **Fold-Change (vs normal liver)** | **Fold-Change (vs normal buffy)** |
|---|---|---|---|
| DAB2IP | 0.94 | 55.04 | 403.81 |
| TSPYL5 | 0.94 | 159.16 | 648.30 |
| AK055957 (chr12.133) | 0.94 | 166.22 | 483.57 |
| EMX1 | 0.90 | 578.89 | 154.47 |
| X57SPINT2 | 0.89 | 15.73 | 83.12 |
| X61ACP1 | 0.89 | 378.07 | 17.96 |
| TBX15.fit | 0.86 | 10.02 | N/A |
| X44SCRN1 | 0.85 | 5.68 | 12.94 |
| TBX15 | 0.85 | 8.57 | 41.50 |
| X53HDGFRP3 | 0.83 | 2.85 | 74.06 |
| OPLAH | 0.83 | 2.16 | 900.80 |
| X49ST8SIA6 | 0.82 | 5405378.50 | 90.32 |
| X52HDGFRP3 | 0.82 | 285.68 | 42.86 |
| CLEC11A | 0.82 | 30.73 | 51.09 |
| X63DBNL | 0.81 | 3.04 | 32.20 |
| X65EGR2.fit | 0.80 | 9.83 | N/A |
| HOXA1 | 0.80 | 9.13 | 2746.00 |
| X50OVOL1 | 0.79 | 17.01 | 66.53 |
| X65EGR2 | 0.78 | 23.71 | 12.57 |
| X71COTL1 | 0.77 | 2407.68 | 10613.37 |
| X72RASSF2 | 0.77 | 4.35 | 6960.58 |
| X47TDH | 0.76 | 8.55 | 13.52 |
| X74GALNT12 | 0.75 | 16.31 | 42.20 |
| X45PRKAG2 | 0.72 | 20.02 | 923.17 |
| ST8SIA1 | 0.71 | 1.66 | 56.25 |
| LRRC4 | 0.71 | 1.47 | 433.42 |
| C13orf18 | 0.68 | 1.99 | 27.00 |
| X39ECE1 | 0.65 | 3.36 | 163.46 |
| X67chr4.56915281 | 0.64 | 6.39 | 102.50 |

Such experiments additionally resulted in the identification of 89 liver epithelial DMRs which are methylated in liver (cancer and normal) but not in normal leukocyte DNA samples.

Table 4 provides information for liver epithelial DMRs which are methylated in liver (cancer and normal) but not in normal leukocyte DNA samples.

**Table 4.**

| **DMR No.** | **Gene Annotation** | **DMR Chr. No and Start End Position** |
|---|---|---|
| 312 | ABHD8 | chr19:17403265-17403457 |
| 313 | ABTB1 | chr3:127391081-127391163 |
| 314 | ADAM8 | chr10:135090174-135090246 |
| 315 | AGAP3 | chr7:150812289-150812467 |
| 316 | AGAP3 | chr7:150812127-150812178 |
| 317 | AMIGO3 | chr3:49757070-49757161 |
| 318 | ANXA2 | chr15:60690852-60690949 |
| 319 | APBB2 | chr4:40859188-40859260 |
| 320 | ATP2B4 | chr1:203598701-203598782 |
| 321 | B3GALT4 | chr6:33245156-33245191 |
| 322 | B3GALT4 | chr6:33244921-33245067 |
| 323 | BDH1 | chr3:197282831-197282922 |
| 324 | BDH1 | chr3:197281722-197281827 |
| 325 | BMP2 | chr20:6750738-6750803 |
| 326 | C17orf64 | chr17:58499118-58499182 |
| 327 | C6orf223 | chr6:43970506-43970593 |
| 328 | CELF6 | chr15:72612688-72612773 |
| 329 | DAP2IP | chr9:124461326-124461415 |
| 330 | DLEC1 | chr3:38080680-38080710 |
| 331 | EPS8L2 | chr11:726256-726367 |
| 332 | ESPNP | chr1:17027786-17027840 |
| 333 | F12 | chr5:176830943-176831003 |
| 334 | F12 | chr5:176830740-176830823 |
| 335 | FIGNL2 | chr12:52214703-52214931 |
| 336 | FLJ45983 | chr10:8097592-8097679 |
| 337 | GAL3ST2 | chr2:242743108-242743318 |
| 338 | GRIN2D | chr19:48901811-48901852 |
| 339 | GSTO2 | chr10:106028898-106028963 |
| 340 | IL17C | chr16:88701236-88701393 |
| 341 | IL4I1 | chr19:50393413-50493491 |
| 342 | IRF4 | chr6:393636-393768 |
| 343 | JARID2 | chr6:15244974-15245009 |
| 344 | KCNQ4 | chr1:41249959-41250042 |
| 345 | KDM2B | chr12:121904281-121904436 |
| 346 | LFNG | chr7:2559582-2559607 |
| 347 | LGALS3 | chr14:55595740-55595831 |
| 348 | LIMD2 | chr17:61778042-61778108 |
| 349 | LOC389333 | chr5:138728888-138728935 |
| 350 | LOC389333 | chr5:138728233-138728342 |
| 351 | LRRC4 | chr7:127671917-127672169 |
| 352 | LRRC4 | chr7:127672388-127672445 |
| 353 | LRRFIP1 | chr2:238600061-238600124 |
| 354 | LTBP4 | chr19:41119803-41119889 |
| 355 | LYL1 | chr19:13215369-13215437 |
| 356 | LYL1 | chr19:13210345-13210498 |
| 357 | MACROD1 | chr11:63828351-63828427 |
| 358 | MARVELD1 | chr10:99474326-99474382 |
| 359 | MAX.chr10:22765154-22765214 | chr10:22765154-22765214 |
| 360 | MAX.chr1 1:518982-519057 | chr11:518982-519057 |
| 361 | MAX.chr14:107253126-107253203 | chr14:107253126-107253203 |
| 362 | MAX.chr20:1784481-1784547 | chr20:1784481-1784547 |
| 363 | MAX.chr6:167763903-167763975 | chr6:167763903-167763975 |
| 364 | MAX.chr8:142215988-142216025 | chr8:142215988-142216025 |
| 365 | MAX.chr8:145104176-145104352 | chr8:145104176-145104352 |
| 366 | MBOAT2 | chr2:9144074-9144113 |
| 367 | MTHFD1L | chr6:151187945-151188021 |
| 368 | MTHFD2 | chr2:74425838-74425898 |
| 369 | MYPOP | chr19:46405010-46405058 |
| 370 | NGEF | chr2:2337929980233793066 |
| 371 | NR2F6 | chr19:17346368-17346464 |
| 372 | NR2F6 | chr19:17346575-17346695 |
| 373 | NTN1 | chr17:9143556-9143609 |
| 374 | NTRK1 | chr1:156786617-156786674 |
| 375 | PDZD7 | chr10:102792180-102792249 |
| 376 | PTK2B | chr8:27183159-27183229 |
| 377 | PTPRE | chr10:129845681-129845740 |
| 378 | RASSF1 | chr3:50378497-50378540 |
| 379 | RUNX3 | chr1:25256236-25256294 |
| 380 | S1PR4 | chr19:3179884-3179960 |
| 381 | SBNO2 | chr19:1131812-1131869 |
| 382 | SH3PXD2A | chr10:105453034-105453075 |
| 383 | SHH | chr7:155597905-155597937 |
| 384 | SLC25A36 | chr3:140661249-140661281 |
| 385 | SOCS3 | chr17:76355477-76355512 |
| 386 | SP9 | chr2:175202178-175202322 |
| 387 | SPDYA | chr2:29033684-29033774 |
| 388 | TFR2 | chr7:100230996-100231029 |
| 389 | TIAM1 | chr21:32930248-32930318 |
| 390 | TIAM1 | chr21:32931595-32931681 |
| 391 | TIAM1 | chr21:32931280-32931363 |
| 392 | UCP2 | chr11:73693845-73693912 |
| 393 | VIM | chr10:17271919-17271971 |
| 394 | WNT1 | chr12:49375039-49375117 |
| 395 | WNT11 | chr11:75917494-75917626 |
| 396 | ZFYVE28 | chr4:2415181-2415265 |
| 397 | ZMIZ1 | chr10:81003086-81003157 |
| 398 | ZMIZ1 | chr10:81002084-81002169 |
| 399 | ZMIZ1 | chr10:81002889-81002992 |
| 400 | ZNF703 | chr8:37554906-37554971 |

### Study subjects and samples

The study was approved by the Mayo Clinic Institutional Review Board (Rochester, MN). Fresh frozen (FF) tissues, plasma, and buffy coat samples were provided by IRB-approved patient biobanks. Tumor tissue sections were re-reviewed by an expert GI pathologist to confirm diagnosis and estimate neoplastic cellularity. Sections were then macro-dissected. Genomic DNA was purified using the QiaAmp Mini kit (Qiagen, Valencia CA) and subsequently re-purified with the AMPure XP kit (Beckman Coulter, Brea CA).

### Preparation of Reduced Representation Bisulfite Sequencing Libraries

Sequencing libraries were prepared using a modified version of a previously published method. Genomic DNA (300 ng) was digested overnight with 10 U of MspI. All enzymes used for this and subsequent steps were provided by New England Biolabs (NEB) unless otherwise specified. Fragments were end-repaired and A-tailed with 5 U of Klenow fragment (3'-5' exo-), and ligated overnight to TruSeq adapters (Illumina) containing barcode sequences and universally methylated cytosines. SYBR Green qPCR (LightCycler 480 - Roche) was used to gauge ligation efficiency and fragment quality. Samples were bisulfite treated and purified (twice) using a modified EpiTect protocol (Qiagen) and then put through a final AMPure XP clean up. qPCR was used to determine the optimal PCR cycles for library enrichment. The following conditions were used for the enrichment PCR: Each 50uL reaction contained 5uL of 10X buffer, 1.25uL of 10 mM each deoxyribonucleotide triphosphate (dNTP), 5uL primer cocktail (~5uM), 15uL sample, 1uL PfuTurbo Cx hotstart and 22.75 µL water; temperatures and times were 95C-5min; 98C-30sec; 12 to 16 cycles of 98C-10sec, 65C-30sec, 72C-30sec, 72C-5min and 4C hold, respectively. Samples were quantified by PicoGreen assay (Molecular Probes), combined into randomized 4-plex libraries, and tested with the Bioanalyzer 2100 (Agilent) for size verification. Additional rounds of AMPure XP purification/size selection were performed at empirically determined buffer concentrations to minimize adaptor dimer contamination and remove inserts larger than 350 bp. A final library assessment was accomplished by qPCR using phiX control standards (Illumina) and adaptor-specific primers.

### Massively Parallel Sequencing and Bioinformatics

Samples were loaded onto flow cells according to a randomized lane assignment with additional lanes reserved for internal assay controls. Sequencing was performed by the Next Generation Sequencing Core at the Mayo Clinic Medical Genome Facility on the Illumina HiSeq 2000. Reads were unidirectional for 101 cycles. Each flow cell lane generated 100-120 million reads, sufficient for a median coverage of 30-50 fold sequencing depth for aligned sequences. Standard Illumina pipeline software called bases and generated reads in the fastq format. SAAP-RRBS (streamlined analysis and annotation pipeline for reduced representation bisulfite sequencing) was used for sequence read assessment and clean-up, alignment to reference genome, methylation status extraction, and CpG reporting and annotation. CpGs with low coverage (≤ 10) were excluded. Tertiary analysis consisted of removing non-informative or low sample coverage CpGs, and identifying methylated CpG regions with low background and dense clusters within sliding 100 bp windows. Read-depth criteria were based on the desired statistical power to detect a 10% difference in the %-methylation between cases and controls. Statistical significance was determined by logistic regression of the methylation percentage per DMR, based on read counts. To account for varying read depths across individual subjects, an over-dispersed logistic regression model was used, where dispersion parameter was estimated using the Pearson Chi-square statistic of the residuals from fitted model. DMRs, ranked according to their significance level, were further considered if %-methylation in control groups was ≤1% and ≥10% in cancers. In most organ sites, this resulted in hundreds of potential candidates. Additional filters utilized were area under the receiver operating characteristic curve, signal to background % methylation ratios (fold-change), and positive sample to sample co-methylation of CpGs throughout the DMR (and lack thereof in controls).

### Technical and Biological Tissue Validation

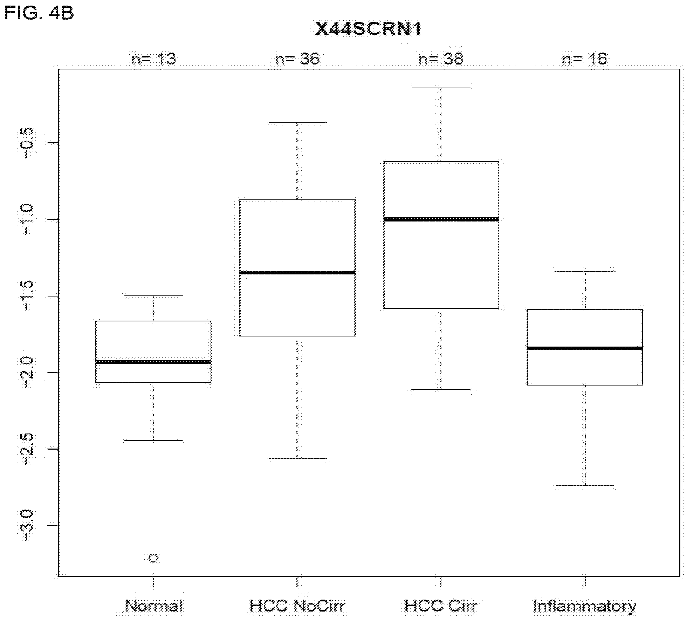

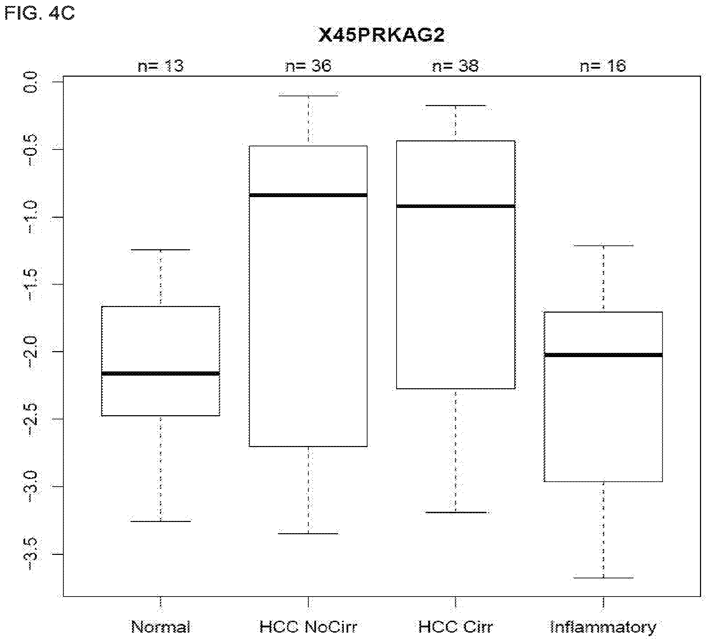

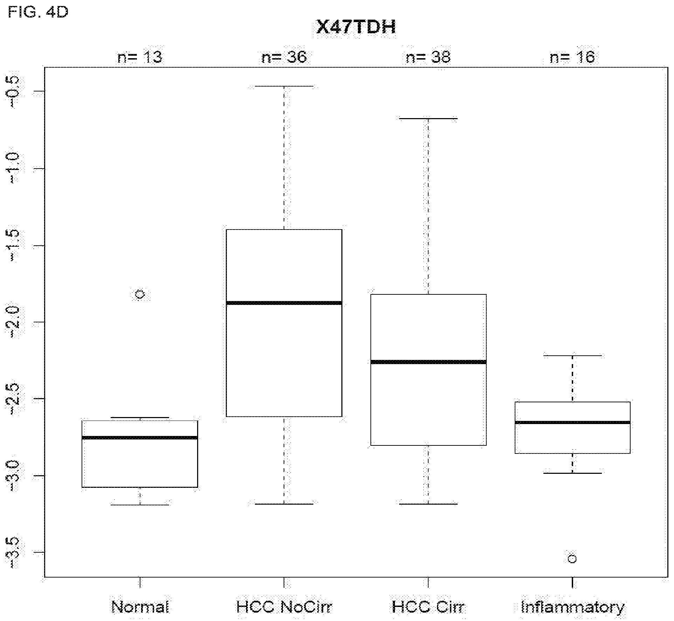

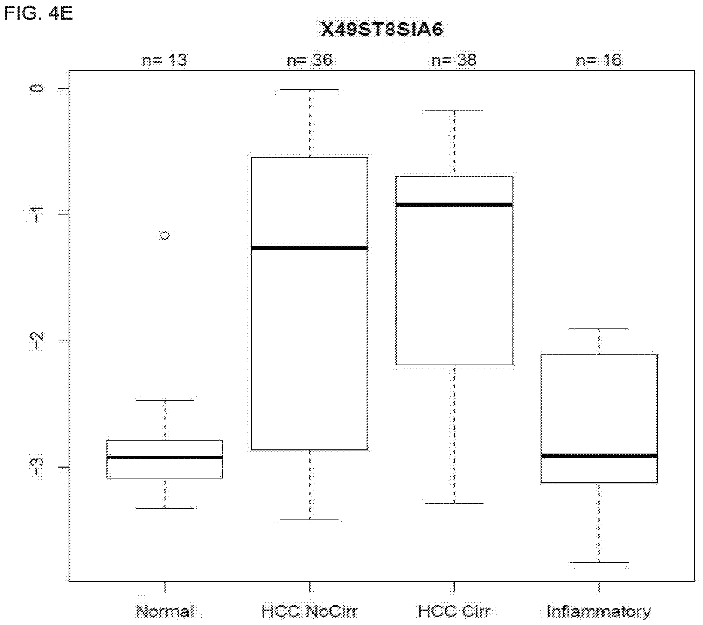

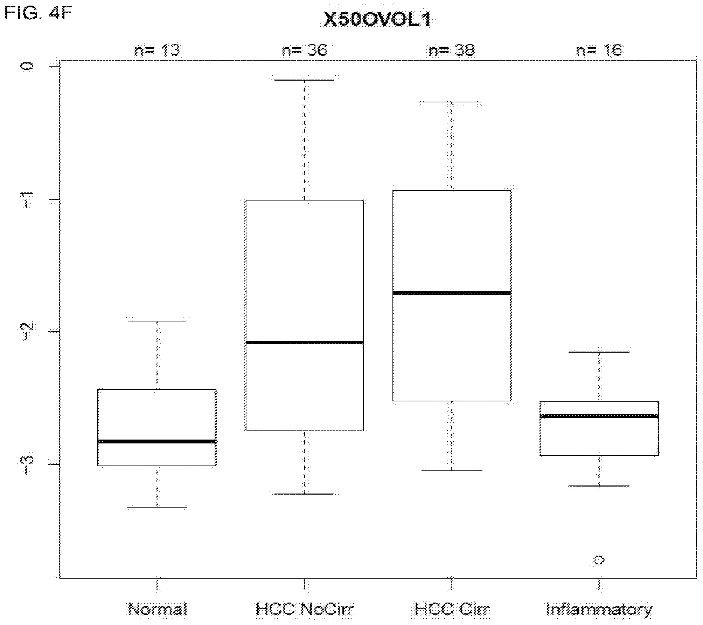

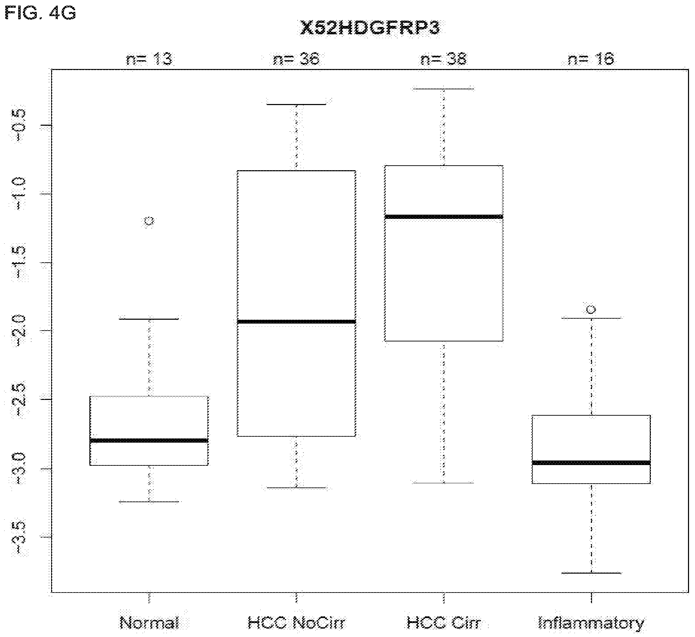

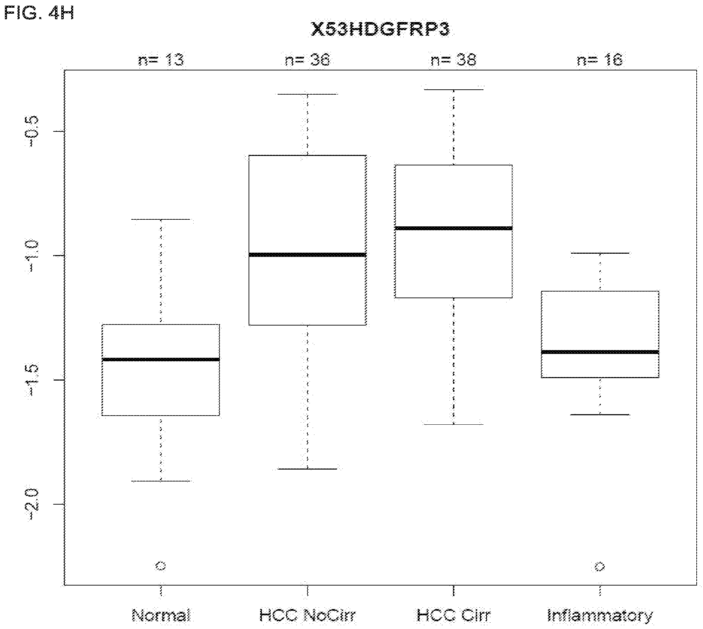

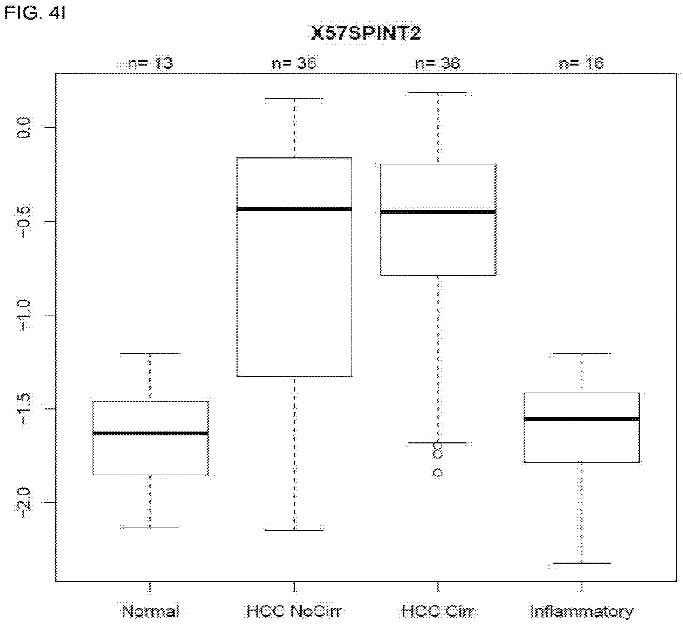

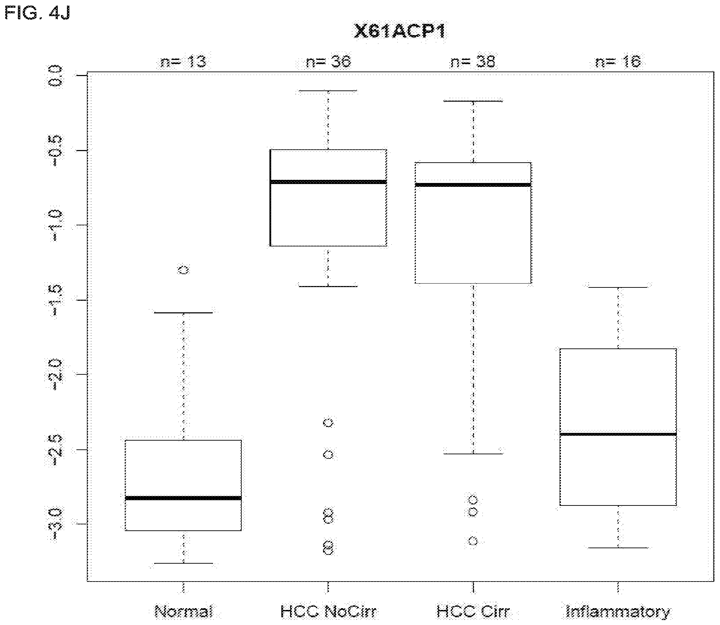

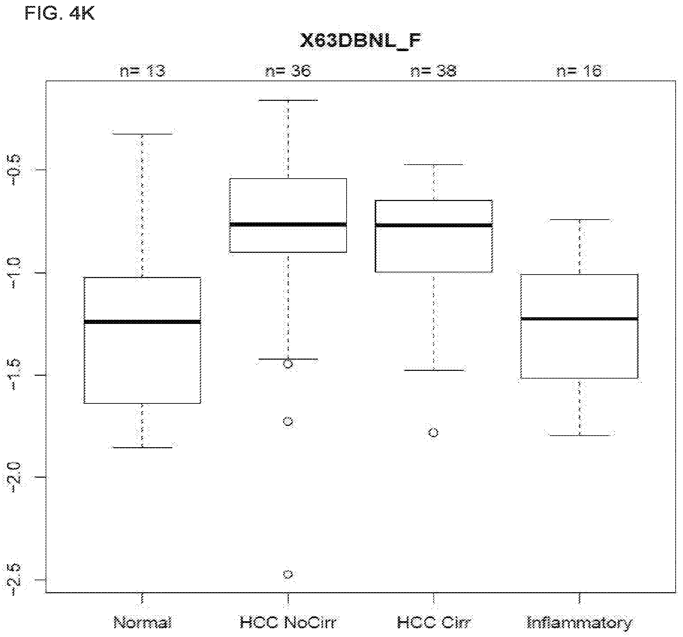

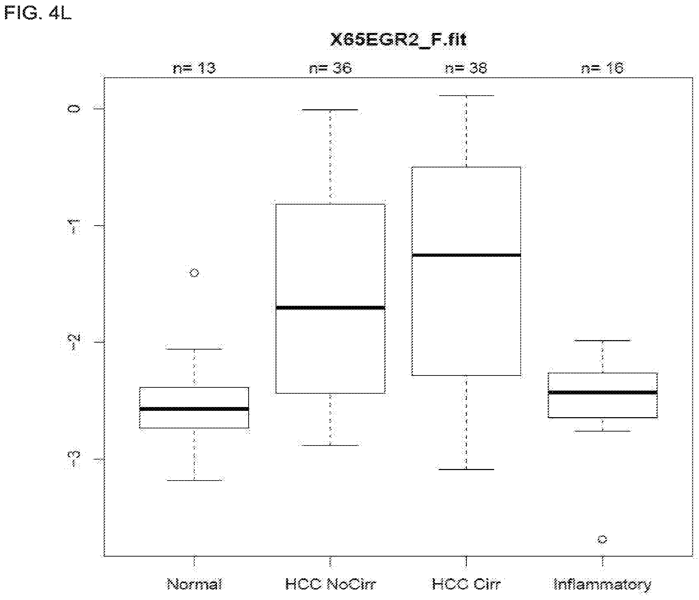

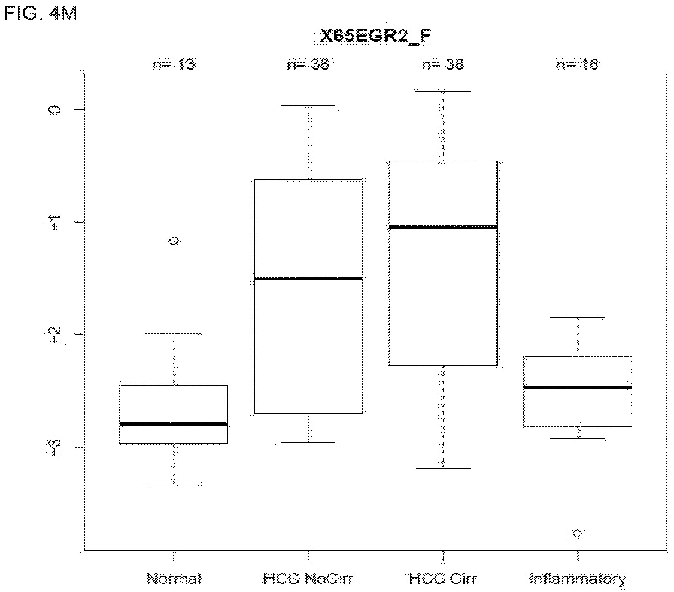

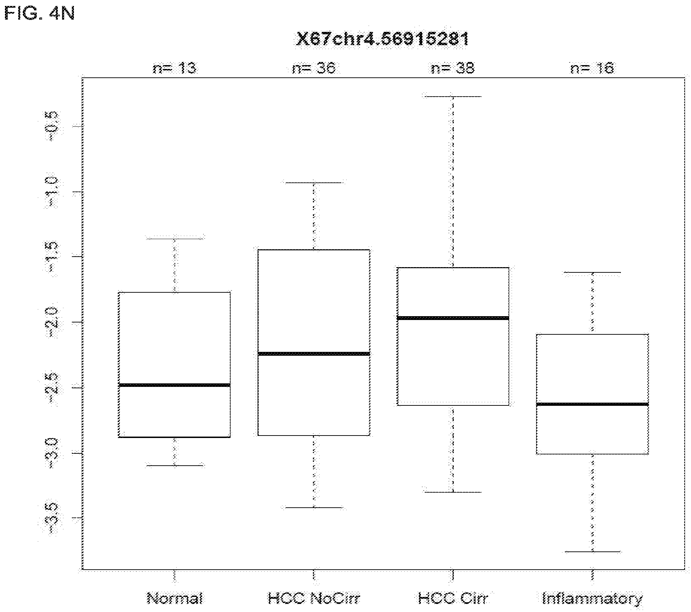

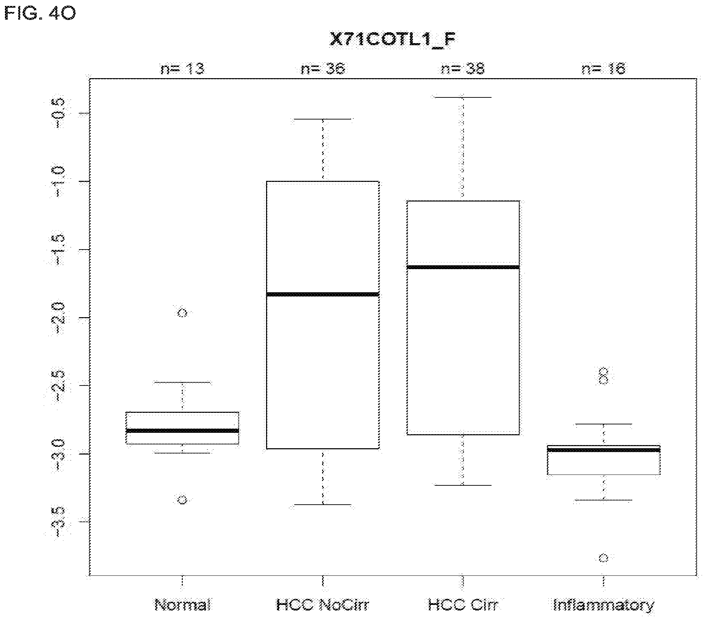

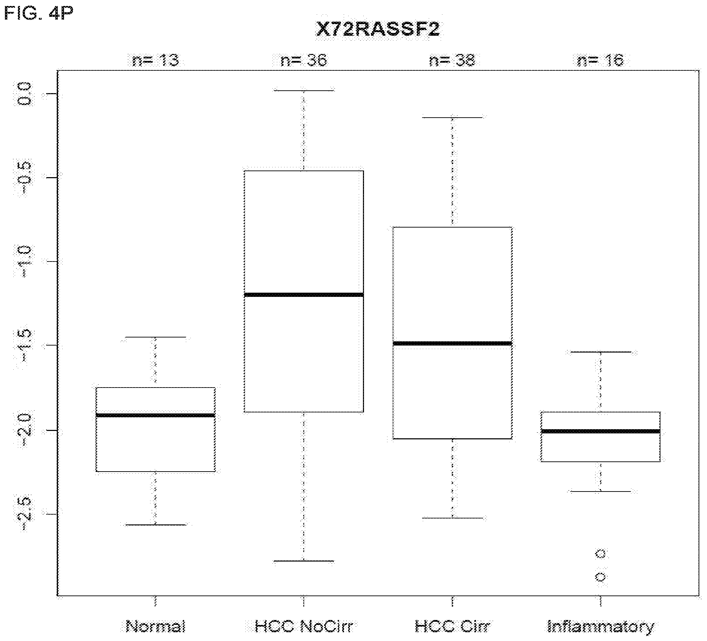

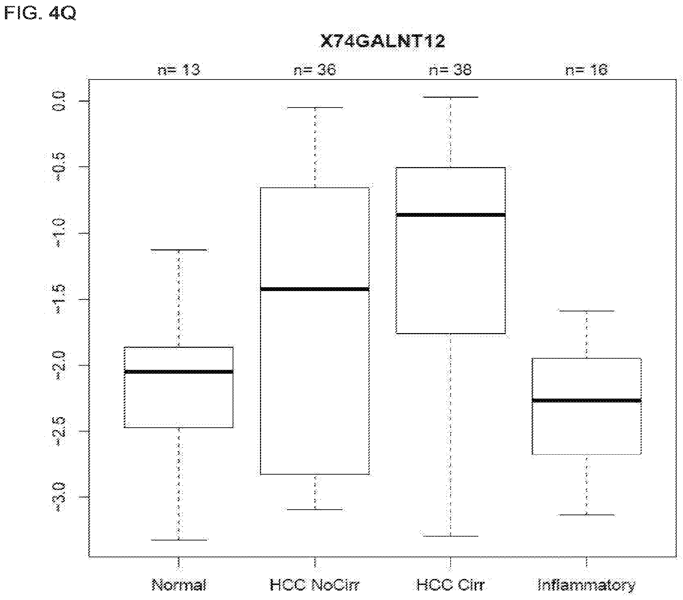

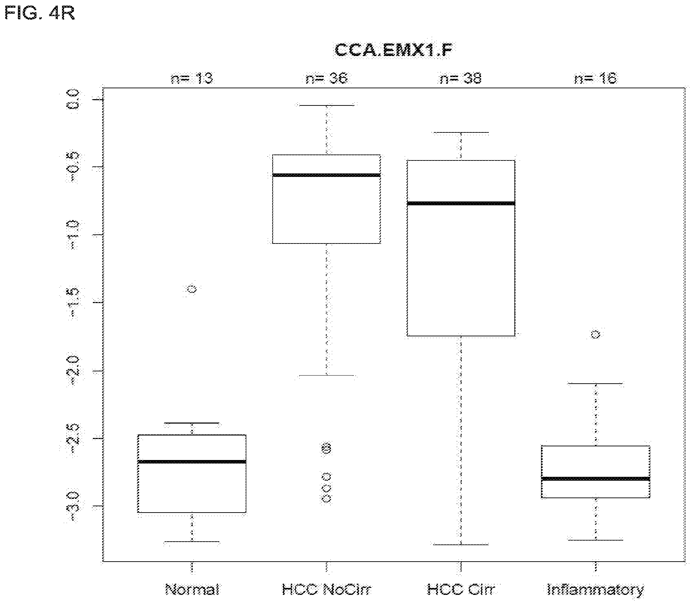

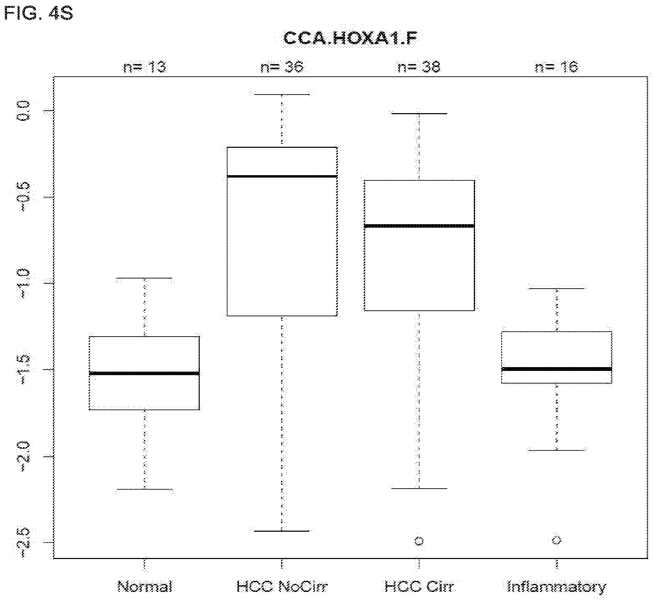

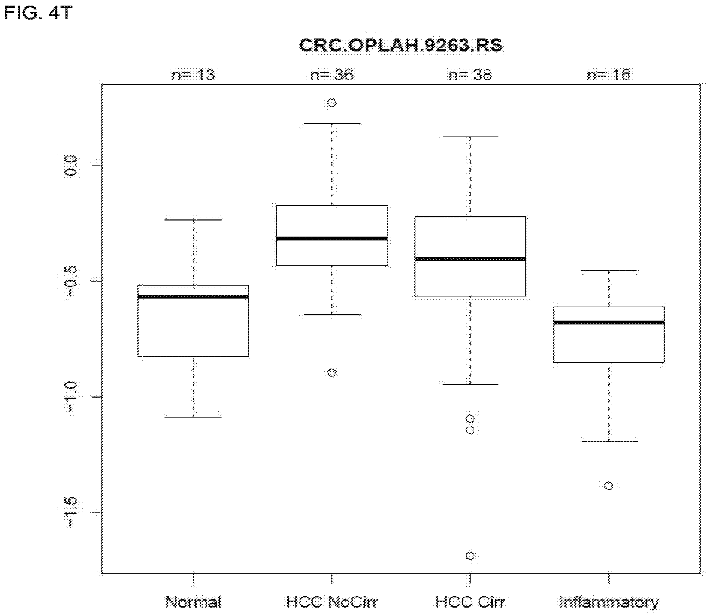

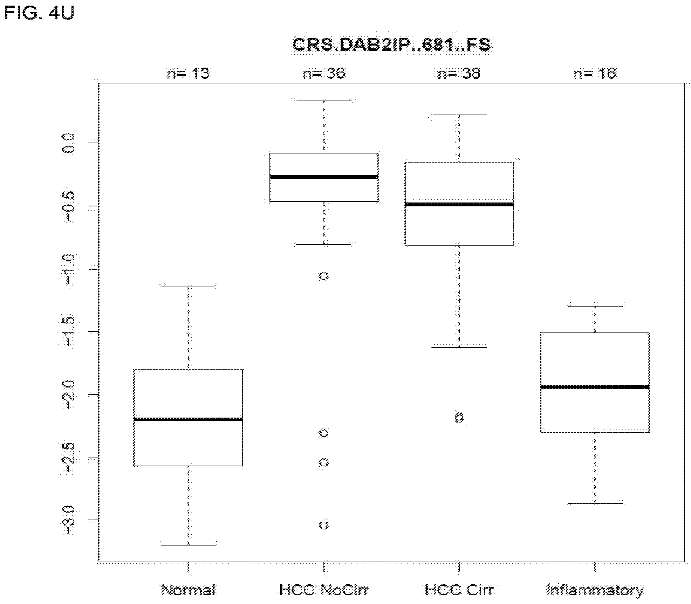

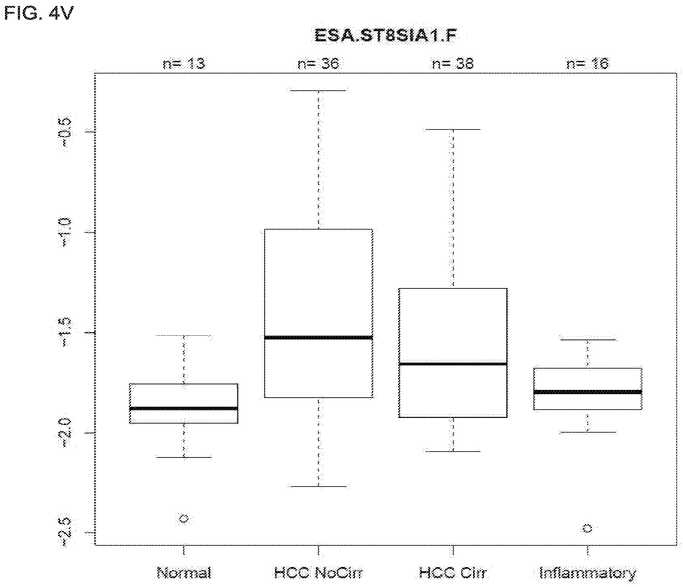

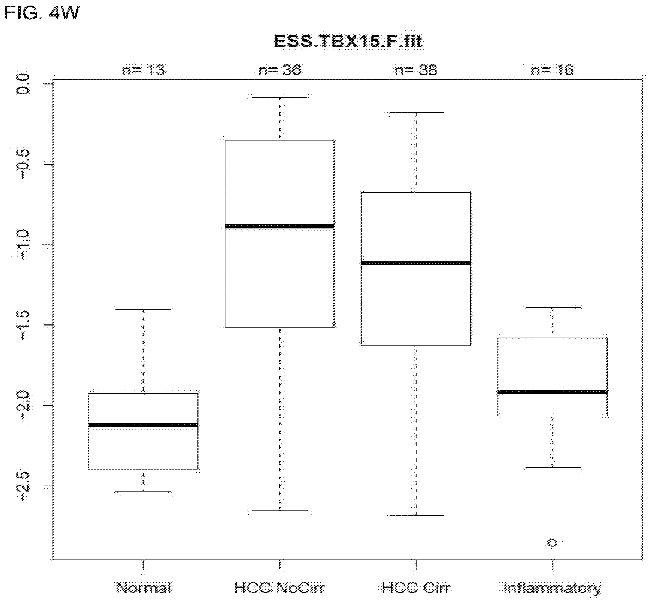

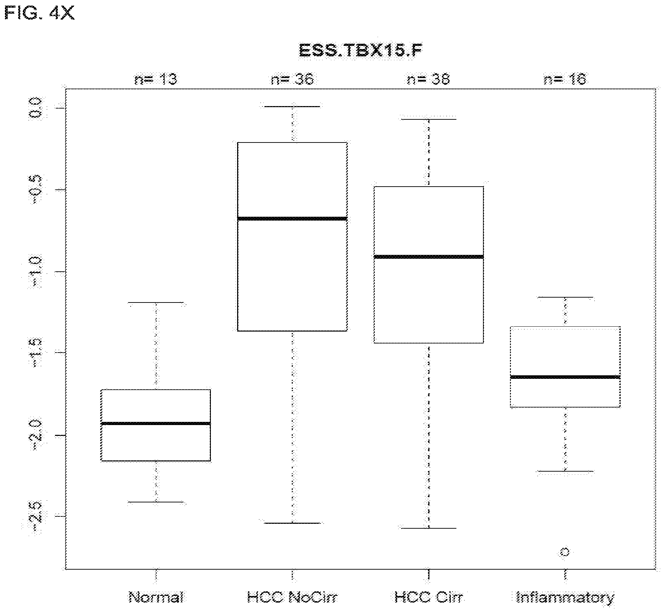

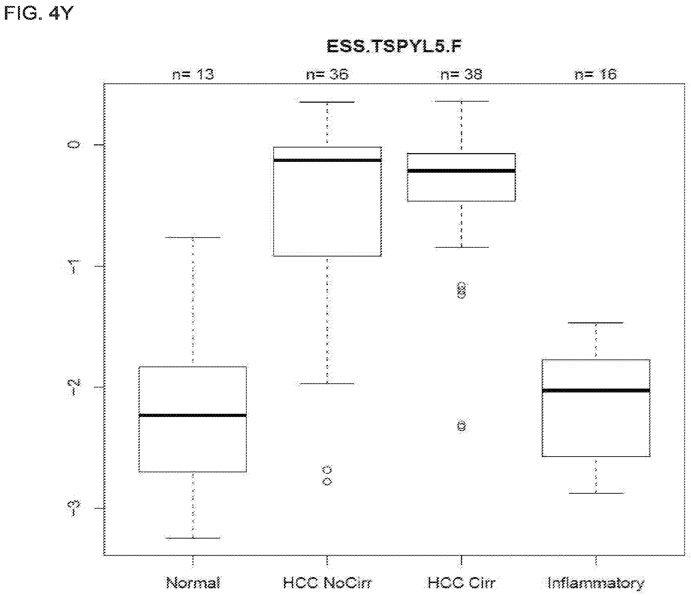

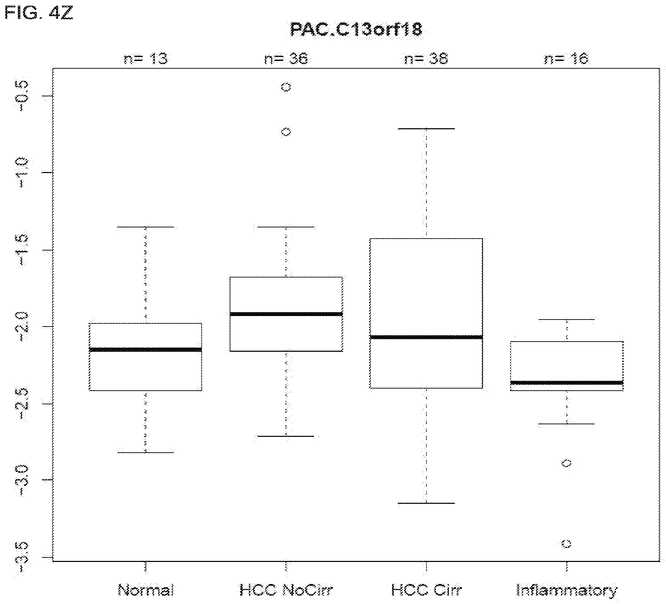

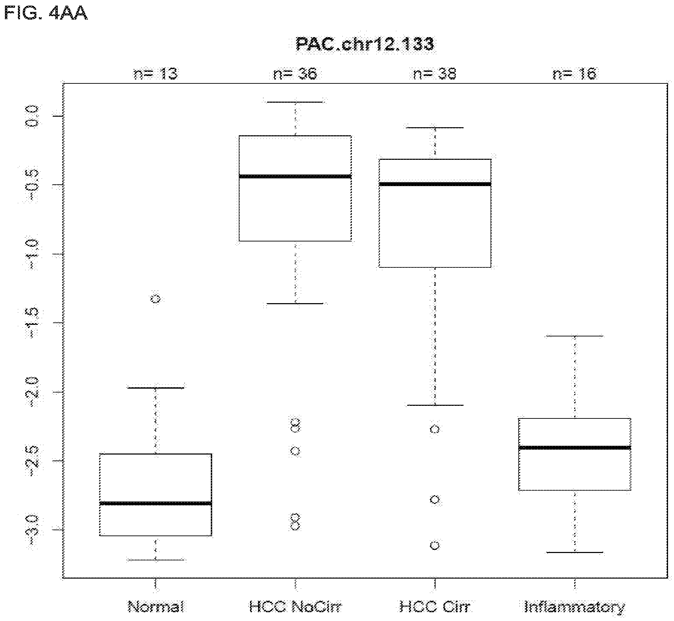

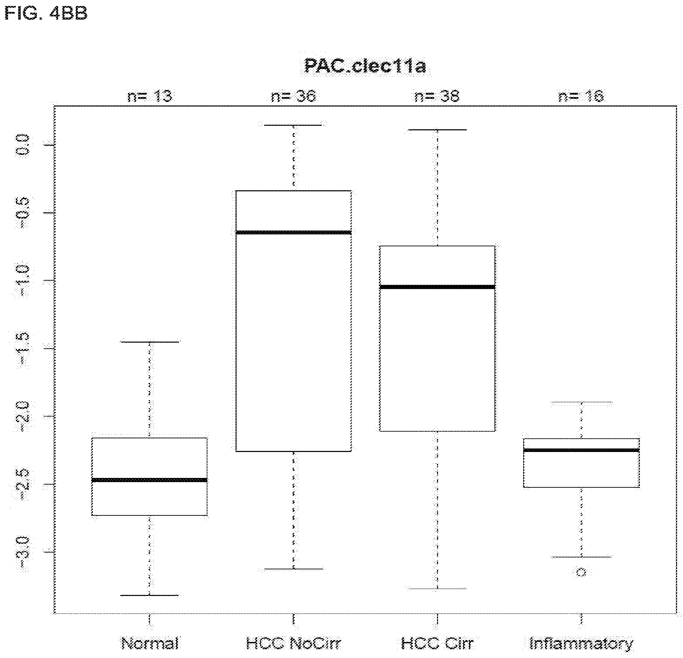

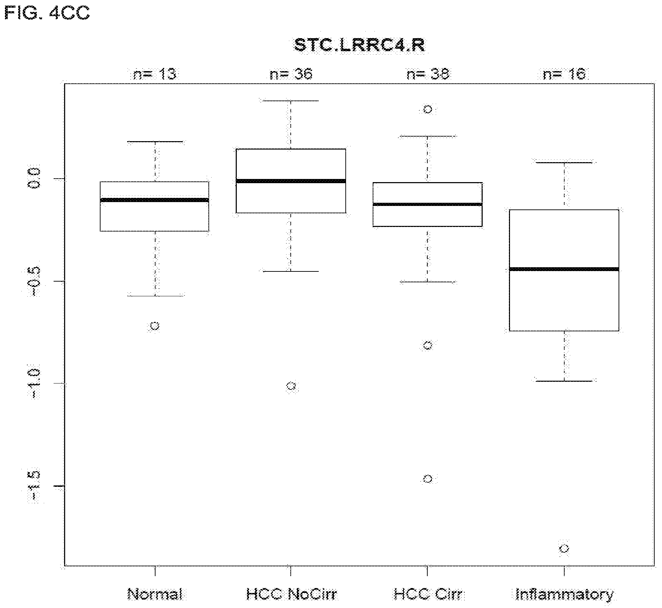

Methylation specific PCR (MSP) marker assays were developed for 30 of the most promising DMRs from the liver discovery dataset - as determined by the criteria listed above. Primers were designed either by software (Methprimer - University of California, San Francisco CA; MSP Primer - Johns Hopkins University, Baltimore, MD) or by hand. Assays were rigorously tested and optimized by SYBR Green qPCR on bisulfite converted (methylated and unmethylated genomic DNA), unconverted, and non-template controls. Assays which cross-reacted with negative controls were either redesigned or discarded. In addition, melting curve analysis was performed to ensure specific amplification was occurring. For the technical validation phase, the same samples used for the RRBS discovery were retested by qMSP. A β-actin assay designed to be methylation blind was used as a denominator representing total DNA copies. The data were analyzed by logistic regression and the AUC and signal to background results compared to the discovery values. Slightly less than half of the markers underperformed and were eliminated. The remainder (N=16) were tested by qMSP on an expanded set of 104 independent tissue samples. In addition, experiments included 11 methylation cancer markers which were identified and validated in earlier sequencing studies on other GI cancers (colon, esopagus, pancreas, bile duct) and which are strong multi-organ cancer markers. The outcome metrics were AUC and fold change ratio (Table 3). Box plots and a complementarity matrix for the assayed markers are portrayed in figures 4 and 5, respectively.

### Across Organ Validation

To assess how the best methylation markers performed outside of the liver, experiments constructed a comparitive CpG %methylation matrix using the sequencing reads for the validation DMRs across the HCC samples and other major GI cancers that were sequenced earlier - colon, pancreatic, esophageal, and stomach. A final panel of markers was chosen to test in plasma based on 1) overall performance in the biological tissue validation phase and 2) the site specific characteristics of the markers across other cancers. To best detect HCC in blood, given the excess of non-HCC DNA, a robust 12 marker panel was chosen that would exhibit both universal and liver specific cancer signals. 10 of the markers were from the tissue validation; 2 additional markers which demonstrated extraordinary liver site specificity, EFNB2 and BDH1, were directly designed and ulilized from the RRBS data without subsequent tissue validation.

### Plasma Validation

Plasma DNA was extracted from 2mL fractions by an automated silica bead method developed at Exact Sciences.

| | |
|---|---|
| 1 | 2ml of Te buffer (1mM Tris 0.1mM EDTA) |
| 2 | 100µl of 120 cp/ul Zebrafish DNA in 0.4 ng/µl of Fish DNA diluent |
| 3 | 7ml of plasma Lysis buffer (4.3M GTC 10% IGEPAL) |
| 4 | 2ml of plasma |
| 5 | incubate at 55°C for 1 hour |
| 6 | Add 200µl binding beads |
| 7 | Add 2.8ml of 100% Isopropanol |
| 8 | Incubate at 30°C for 30 minutes. |
| 9 | magnatize the beads and remove the supernatent |
| 10 | Add 750µl 3M GuHCl 56.8% EtOH to resuspend binding beads |
| 11 | shake at 400 RPM for 2 minute. |
| 12 | Bind beads and aspirate supernatant to waste |
| 13 | 1000µl wash 1 (80%ETOH), incubate at 30°C for 3 minutes, Bind beads and aspirate supernatant to waste |
| 14 | 500µl wash 1 (80%ETOH), incubate at 30°C for 3 minutes, Bind beads and aspirate supernatant to waste |
| 15 | 250µl wash 1 (80%ETOH), incubate at 30°C for 3 minutes, Bind beads and aspirate supernatant to waste |
| 16 | 250µl wash 1 (80%ETOH), incubate at 30°C for 3 minutes, Bind beads and aspirate supernatant to waste |
| 17 | Dry at 70°C, 15 minutes, with shaking. |
| 18 | Add 125ul of Te buffer (1mM Tris 0. ImM EDTA) incubate 65°c for 25 minutes with shaking |
| 19 | Bind beads and transfer supernatent containing DNA to clean tubes |
| 20 | Store at -20°C until use. |

The DNA was then bisulfite converted and purified, using the proprietary method outlined below.

| | |
|---|---|
| 1 | 5ul 0.36% BSA |
| 2 | 70ul of Sample |
| 3 | 5ul 1.6N NaOH |
| 4 | Incubate (denaturation) 20' @ 42°C |
| 5 | cool for 8' |
| 6 | add 120ul of the Ammonium Bisulfite |
| 7 | Incubate (conversion) 75' @ 65°C (shake 3') |
| 8 | add 750ul of the 7M GuHCl |
| 9 | add 50ul binding beads |
| 10 | Incubate for 30' @ 30°C with shaking |
| 11 | Bind the beads |
| 12 | Aspirate supernatent to waste |
| 13 | add 1000ul 80% ETOH |
| 14 | Incubate for 3' @ 30°C with shaking |
| 15 | Bind the beads |
| 16 | Aspirate supernatent to waste |
| 17 | Dispense 200ul Desulphonation solution |
| 18 | Incubate for 7' @ 30°C with shaking |
| 19 | Bind the beads |
| 20 | Aspirate supernatent to waste |
| 21 | add 250ul 80% ETOH |
| 22 | Incubate for 3' @ 30°C with shaking |
| 23 | Bind the beads |
| 24 | Aspirate supernatent to waste |
| 25 | Dry beads for 15' @ 70°C with shaking |
| 26 | Add 80ul of Te buffer (1mM Tris 0.1mM EDTA) |
| 27 | incubate 65°c for 25 minutes with shaking |
| 28 | Bind beads and transfer supernatent containing DNA to clean tubes |
| 29 | Store at -20°C until use. |

Samples were run on a real-time PCR instrument (Roche LC480) in the QuARTs format (see, U.S. Patent No. 8,361,720) using primers and probes developed from the DMR sequence (see, Table 2), GoTaq DNA Polymerase (Promega), Cleavase II (Hologic), and fluorescence resonance energy transfer reporter cassettes (FRETs) containing FAM, HEX, and Quasar 670 dyes (Biosearch Technologies).

Fig. 6 provides the oligonucleotide sequences for the FRET cassettes, used in the detection of methylated DNA signatures by QuARTs (quantitative allele-specific real-time target and signal amplification) assay. Each FRET sequence includes a fluorophore and quencher which can be multiplexed together into 3 separate assays.

Plasmids containing the marker sequence of interest were obtained from Genscript and diluted in IX QuARTs reagents to a nominal concentration of 1 copy per 15 ul reaction. The reaction mix was distributed to each of 384 wells, cycled for 45 cycles on a LightCycler, and data collected. Wells were given a call of either containing or not containing sample. The Poisson random variable was set at 1 and average rate of success values were entered by trial and error and used to calculate the cumulative probability for that value. When the cumulative probability equals the percent of wells with signal, the correct average rate of success, in this case copy number, has been found. These plasmids were diluted and used as assay standards.

QuARTs-X (see, U.S. Provisional Patent No. 62/249,097) is performed by first creating a pre-amplification plate of samples performed with primers for up to 12 targets that undergo 11 cycles of amplification. This product is then diluted 1:9 and used as template for subsequent QuARTs reactions that contain only three targets in a triplex reaction. Standards used to calculate strand counts do not go through the pre-amplification. By pre-amplifying the samples but not the standards the sensitivity of the assays are increased.

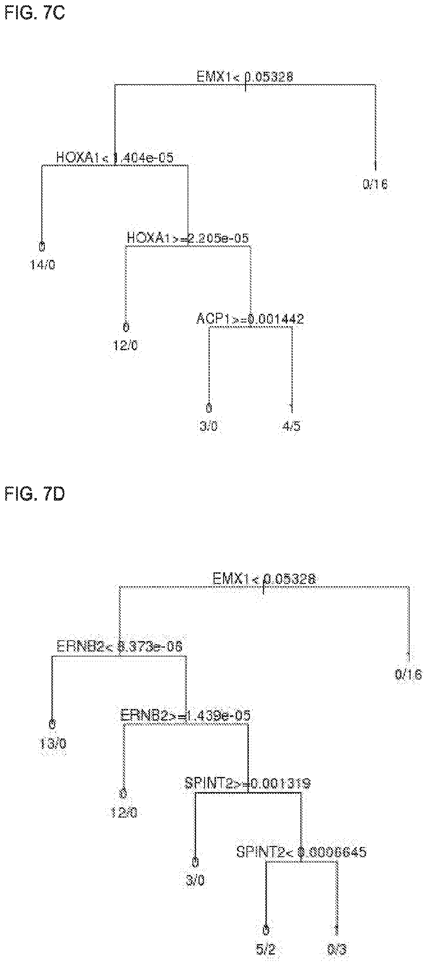

Results were analyzed by regressive partitioning (rPart). Using logistic regression to combine multiple methylation markers into a single risk score is a standard technique. However, it is difficult to discover and/or model high order interactions between markers within the logistic model. This limits the prediction capabilities of our panel of markers when such effects exist. Regression partitioning trees (rPart) is a decision tree approach that is able to discover high order interactions between the markers in such a way as to maximize the predictive accuracy of a panel of markers. With rPart, the sensitivity and specificity of HCC blood samples for the top 3 marker combination (EMX1, BDH1, LRRC4) was 97% and 95%, respectively. (Fig. 7A) Three alternative marker combinations were modelled:
Alternative #1 (EMX1, DAB2IP, TSPYL5): Specificity=100% Sensitivity=90% (Fig. 7B)
Alternative #2 (EMX1, HOXA1, ACPI): Specificity=88% Sensitivity=100% (Fig. 7C)
Alternative #3 (EMX1, EFNB2, SPINT2): Specificity=100% Sensitivity=90% (Fig. 7D)

The best performing single marker in plasma, EMX1, had an AUC of 0.89 with 77% sensitivity at 100% specificity. Signals for EMX1 showed higher beta actin normalized signal with increasing stage. (Fig. 8).

### Example II.

Tissue samples (75 HCC, 20 cirrhosis, and 30 normal) were run on a real-time PCR instrument (Roche LC480) in the QuARTs format (see, U.S. Patent No. 8,361,720) using primers and probes developed from the DMR sequence (see, Table 5), GoTaq DNA Polymerase (Promega), Cleavase 2.0 (Hologic), and fluorescence resonance energy transfer reporter cassettes (FRETs) containing FAM, HEX, and Quasar 670 dyes (Biosearch Technologies). Table 6 shows the ability of each marker to discriminate HCC from cirrhosis and normal at 100% sensitivity.

**Table 5.**

| **Gene** | **Forward QuARTS primer (5'-3')** | **Reverse QuARTS primer (5'-3')** | **Probe** |
|---|---|---|---|
| TSPYL5 | TTTGTTTCGGTTTTTGG CG (SEQ ID NO: 43) | ACCATAAACGACCGAAAT CGA (SEQ ID NO:44) | CCACGGACG GCGGGAGGAT TT /3C6/ (SEQ ID NO: 45) |
| DAB2IP | CGTTCGTTACGTCGTT TTCGT (SEQ ID NO: 46) | GATCGACGCGACTCGAC (SEQ ID NO: 47) | CCACGGACG CTCGACGTCGC C/3C6/  (SEQ ID NO: 48) |
| Chr12_ 133_V2 | GCGTTTTAGTTAGATA GGGCGG (SEQ ID NO: 49) | GAAAACCCCTTCCCCGAA AC (SEQ ID NO: 50) | CGCCGAGG CGCACGCCTA AA/3C6/ (SEQ ID NO: 51) |
| CLEC11A | GCGGGAGTTTGGCGTA G (SEQ ID NO: 52) | CGCGCAAATACCGAATAA ACG (SEQ ID NO: 53) | CCACGGACG- GTCGGTAGAT CG/3C6/ (SEQ ID NO: 54) |
| SPINT2 | GGGAGCGGTCGCGTA G (SEQ ID NO: 55) | GCACCTAACTAAACAAAA CGAACTAAAC (SEQ ID NO: 56) | CGCCGAGG CGCAAACGCA AA/3C6/ (SEQ ID NO: 57) |
| ACP1 | GCGCGTTGTTTCGTTT CG (SEQ ID NO: 58) | CGTCACCTACCGCAAATA CG  (SEQ ID NO: 59) | CCACGGACG GCGGATAAGG AG/3C6/ (SEQ ID NO: 60) |
| BDH1 | AGTACGTAAGTAGAG CGCG  (SEQ ID NO: 61) | CTAAAATTAACTACGCCG CCGT (SEQ ID NO: 62) | CCACGGACG GAGAACGTTC GA/3C6/ (SEQ ID NO: 63) |
| EFNB2 | CGTTCGTTCGTTATTTT TTTTCGA  (SEQ ID NO: 64) | GCCCGCGAAAACCAAAA AC  (SEQ ID NO: 65) | CGCCGAGG CGAAACTCAC CT/3C6/  (SEQ ID NO: 66) |
| CCNJ_3707 | GCGTTTTTTTTTAGCG GGGTTA (SEQ ID NO: 67) | CCGAAACTAAAATTCTCC CGC  (SEQ ID NO: 68) | CGCCGAGG ATGAGCGTGTT A\3C6\  (SEQ ID NO: 69) |
| CCNJ_3124 | CGGTTTTCGTTTGGGT ACG  (SEQ ID NO: 70) | CCAACCCAAACCACGCC (SEQ ID NO: 71) | CCACGGACG CGCGCCGTAC GA\3C6\ (SEQ ID NO: 72) |
| PFKP | GGAGGTTGGCGGGGA G  (SEQ ID NO: 73) | CATATACTATCGCCTTCC GACTC  (SEQ ID NO: 74) | CGCCGAGG CGTAACAAAA AC\3C6\ (SEQ ID NO: 75) |
| SCRN1 | TCGTTTTAGGTGAGTC GCG  (SEQ ID NO: 76) | AAATAAAACCGCCGAAA AAACAAC (SEQ ID NO: 77) | CCACGGACG CGCTACAAAC GC\3C6\ (SEQ ID NO: 78) |
| ECE1 | GGAGGGGTTTCGTTCG T (SEQ ID NO: 79) | CTACTATCGACGCTAAAA ATAAAACGAAC  (SEQ ID NO: 80) | CGCCGAGG CGCGACCTAA AA/3C6/ (SEQ ID NO: 81) |
| DBNL | AGGTGGCGCGTATTAC G  (SEQ ID NO: 82) | CCTACTAAACGCGCTCAA CC (SEQ ID NO: 83) | CGCCGAGG CGCTCGATTCC C/3C6/  (SEQ ID NO: 84) |
| LRRC4 | GCGTTAATTTCGCGAG GTA (SEQ ID NO: 85) | ACAATACTCTTATATATT AACGCCGCTC  (SEQ ID NO: 86) | CGCCGAGG AGGCGACGGA GG/3C6/ (SEQ ID NO: 87) |
| HOXA1 | AGTCGTTTTTTAGGT AGTTTAGGCG (SEQ ID NO: 88) | CGACCTTTACAATCGCCG C  (SEQ ID NO: 89) | CGCCGAGG GGCGGTAGTT GT/3C6/ (SEQ ID NO: 90) |
| EMX1 | GGCGTCGCGTTTTTA GAGAA (SEQ ID NO: 91) | TTCCTTTTCGTTCGTATAA AATTTCGT (SEQ ID NO: 92) | CCACGGACG ATCGGGTTTTA G/3C6/ (SEQ ID NO: 93) Or CGCCGAGG ATCGGGTTTTA G/3C6/ (SEQ ID NO: 94) |

**Table 6.**

| **Gene** | **Sensitivity for Discriminating HCC from cirrhosis or norm at 100% specificity** |
|---|---|
| TSPYL5 | 79% |
| DAB2IP | 91% |
| Chr12_133_V2 | 91% |
| CLEC11A | 69% |
| SPINT2 | 68% |
| ACP1 | 84% |
| BDH1 | 8% |
| EFNB2 | 45% |
| CCNJ_3707 | 53% |
| CCNJ_3124 | 49% |
| PFKP | 57% |
| SCRN1 | 51% |
| ECE1 | 49% |
| EMX1 | 67% |
| LRRC4 | 20% |
| HOXA1 | 52% |

### Example III.

A primary aim of this was to determine a panel of markers that are predictive of hepatocellar carcinoma (HCC). Plasma from 244 subjects (95 hepatocellular carcinoma and 149 controls) were adjusted to 2 mL and extracted. The 149 controls consisted of 51 cirrhotic and 98 normal patients.

Fig. 9 depicts the relative importance of each of the methylation markers considered in this analysis. The cross validated estimate of sensitivity and specificity for the entire panel of markers was 75% and 96%, respectively.

At 88.6% specificity of controls versus normal, the following marker panel (Chr12.133, CLEC11A, EMX1, HOXA1, CCNJ_3707) resulted in 85.3% sensitivity for HCC (Table 7).

**Table 7.**

| | **Total patients** | **Prediction** | | **Sensitivity** |
|---|---|---|---|---|
| | | **Negative** | **Positive** | |
| HCC | 95 | 14 | 81 | 85.3% |
| controls | 149 | 132 | 17 | |

The same panel (Chr12.133, CLEC11A, EMX1, HOXA1, CCNJ_3707) resulted in the following breakdown of specificities for normal and cirrhotic (control group) patients (Table 8).

**Table 8.**

| | **Total patients** | **Prediction** | | |
|---|---|---|---|---|
| **Patient Group** | | **Negative** | **Positive** | **Specificity** |
| Cirrhotic | 51 | 41 | 10 | 80.4% |
| Normal | 98 | 91 | 7 | 92.9% |
| Total | 149 | 132 | 17 | 88.6% |

An exemplary procedure for isolating DNA from a 4 mL sample of plasma would be conducted as follows:
- To a 2 mL sample of plasma, 300 µL of proteinase K (20mg/mL) is added and mixed. When samples are less than 2 mL of plasma, adjust to 2 mL by adding 10 mM Tris-HCl, 0.1 mM EDTA solution
- Add 6 mL of plasma lysis buffer 1 to plasma and mix at room temperature Plasma lysis buffer is:
   - 4.3M guanidine thiocyanate
   - 10% IGEPAL CA-630 (Octylphenoxy poly(ethyleneoxy)ethanol, branched) (5.3g of IGEPAL CA-630 combined with 45 mL of 4.8 M guanidine thiocyanate)
- Add 200 µL magnetic silica binding beads [16 µg of beads/µL] and mix again.
- Add 7 mL of lysis buffer 2 to tubes.

Plasma lysis buffer 2 is prepared by mixing a ratio of 60% of lysis buffer 1 with 40% ispropanol.
- Mix samples with lysis buffer 2 for 60 minutes
- Place tubes on magnet and let the beads collect for 10 minutes. Aspirate and discard the supernatant.
- Add 1000 µL wash buffer (10 mM Tris HCl, 80% EtOH) to the beads, and incubate at 30°C for 3 minutes with shaking.
- Place tubes on magnet and let the beads collect. Aspirate and discard the supernatant.
- Add 500 µL wash buffer to the beads and incubate at 30°C for 3 minutes with shaking.
- Place tubes on magnet and let the beads collect. Aspirate and discard the supernatant.
- Add 250 µL wash buffer and incubate at 30°C for 3 minutes with shaking.
- Place tubes on magnet and let the beads collect. Aspirate and discard the remaining buffer.
- Add 250 µL wash buffer and incubate at 30°C for 3 minutes with shaking.
- Place tubes on magnet and let the beads collect. Aspirate and discard the remaining buffer.
- Dry the beads at 70°C for 15 minutes, with shaking.
- Add 125 µL elution buffer (10 mM Tris HCl, pH 8.0, 0.1 mM EDTA) to the beads and incubate at 65°C for 25 minutes with shaking.
- Place tubes on magnet and let the beads collect for 10 minutes.
- Aspirate and transfer the supernatant containing the DNA to a new vessel or tube.

### Bisulfite conversion

### I. Sulfonation of DNA using ammonium hydrogen sulfite

1. In each tube, combine 64 µL DNA, 7 µL 1 N NaOH, and 9 µL of carrier solution containing 0.2 mg/mL BSA and 0.25 mg/mL of fish DNA.
2. Incubate at 42°C for 20 minutes.
3. Add 120 µL of 45% ammonium hydrogen sulfite and incubate at 66° for 75 minutes.
4. Incubate at 4°C for 10 minutes.

### II. Desulfonation using magnetic beads

### Materials

Magnetic beads (Promega MagneSil Paramagnetic Particles, Promega catalogue number AS1050, 16 µg/µL).

Binding buffer: 6.5-7 M guanidine hydrochoride.

Post-conversion Wash buffer: 80% ethanol with 10 mM Tris HCl (pH 8.0).

Desulfonation buffer: 70% isopropyl alcohol, 0.1 N NaOH was selected for the desulfonation buffer.

Samples are mixed using any appropriate device or technology to mix or incubate samples at the temperatures and mixing speeds essentially as described below. For example, a Thermomixer (Eppendorf) can be used for the mixing or incubation of samples. An exemplary desulfonation is as follows:
1. Mix bead stock thoroughly by vortexing bottle for 1 minute.
2. Aliquot 50 µL of beads into a 2.0 mL tube (e.g., from USA Scientific).
3. Add 750 µL of binding buffer to the beads.
4. Add 150 µL of sulfonated DNA from step I.
5. Mix (e.g., 1000 RPM at 30°C for 30 minutes).
6. Place tube on the magnet stand and leave in place for 5 minutes. With the tubes on the stand, remove and discard the supernatant.
7. Add 1,000 µL of wash buffer. Mix (e.g., 1000 RPM at 30°C for 3 minutes).
8. Place tube on the magnet stand and leave in place for 5 minutes. With the tubes on the stand, remove and discard the supernatant.
9. Add 250 µL of wash buffer. Mix (e.g., 1000 RPM at 30°C for 3 minutes).
10. Place tube on magnetic rack; remove and discard supernatant after 1 minute.
11. Add 200 µL of desulfonation buffer. Mix (e.g., 1000 RPM at 30°C for 5 minutes).
12. Place tube on magnetic rack; remove and discard supernatant after 1 minute.
13. Add 250 µL of wash buffer. Mix (e.g., 1000 RPM at 30°C for 3 minutes).
14. Place tube on magnetic rack; remove and discard supernatant after 1 minute.
15. Add 250 µL of wash buffer to the tube. Mix (e.g., 1000 RPM at 30°C for 3 minutes).
16. Place tube on magnetic rack; remove and discard supernatant after 1 minute.
17. Incubate all tubes at 30°C with the lid open for 15 minutes.
18. Remove tube from magnetic rack and add 70 µL of elution buffer directly to the beads.
19. Incubate the beads with elution-buffer (e.g., 1000 RPM at 40°C for 45 minutes).
20. Place tubes on magnetic rack for about one minute; remove and save the supernatant.

The converted DNA is then used in pre-amplification and/or flap endonuclease assays.

## Claims

1. A method for characterizing a biological sample comprising:
measuring a methylation level of a CpG site for the gene HOXA1 and one or more genes selected from either
EMX1, DAB2IP, TSPYL5, ACP1, BDH1, Chr12.133, CLEC11A, DBNL, EFNB2, LRRC4, SPINT2, CCNJ_3707, CCNJ_3124, PFKP, SCRN1, and ECE1 in a biological sample of a human individual through treating genomic DNA in the biological sample with bisulfite; amplifying the bisulfite-treated genomic DNA using a set of primers for the genes; and
determining the methylation level of the CpG site by methylation-specific PCR, quantitative methylation-specific PCR, methylation-sensitive DNA restriction enzyme analysis, quantitative bisulfite pyrosequencing, or bisulfite genomic sequencing PCR, and comparing the methylation level to a methylation level of a corresponding set of genes in control samples without HCC; and
determining that the individual has HCC when the methylation level measured in the genes is higher than the methylation level measured in the respective control samples.

2. The method of claim 1 wherein the following set of primers for the genes are used if the biological sample is a tissue a plasma sample:
for EMX1 a set of primers consisting of SEQ ID NOS: 16 and 17 or SEQ ID NOS: 91 and 92,
for DAB2IP a set of primers consisting of SEQ ID NOS: 4 and 5 or SEQ ID NOS: 46 and 47,
for TSPYL5 a set of primers consisting of SEQ ID NOS: 1 and 2 or SEQ ID NOS: 43 and 44,
for HOXA1 a set of primers consisting of SEQ ID NOS: 28 and 29 or SEQ ID NOS: 88 and 89,
for ACP1 a set of primers consisting of SEQ ID NOS: 10 and 11 or SEQ ID NOS: 58 and 59,
for BDH1 a set of primers consisting of SEQ ID NOS: 13 and 14 or SEQ ID NOS: 61 and 62,
for Chr12.133 a set of primers consisting of SEQ ID NOS: 25 and 26 or SEQ ID NOS: 49 and 50,
for CLEC11A a set of primers consisting of SEQ ID NOS: 7 and 8 or SEQ ID NOS: 52 and 53,
for DBNL a set of primers consisting of SEQ ID NOS: 34 and 35 or SEQ ID NOS: 82 and 83,
for EFNB2 a set of primers consisting of SEQ ID NOS: 37 and 38 or SEQ ID NOS: 64 and 65,
for LRRC4 a set of primers consisting of SEQ ID NOS: 40 and 41 or SEQ ID NOS: 85 and 86,
for SPINT2 a set of primers consisting of SEQ ID NOS: 31 and 32 or SEQ ID NOS: 55 and 56,
for CCNJ_3707 a set of primers consisting of SEQ ID NOS: 67 and 68,
for CCNJ_3124 a set of primers consisting of SEQ ID NOS: 70 and 71,
for PFKP a set of primers consisting of SEQ ID NOS: 73 and 74,
for SCRN1 a set of primers consisting of SEQ ID NOS: 76 and 77, and
for ECE1 a set of primers consisting of SEQ ID NOS: 79 and 80.

3. The method of claim 1, wherein the biological sample is a plasma sample or a tissue sample.

4. The method of claim 1, wherein said CpG site is present in a coding region or a regulatory region.

5. The method of claim 1, wherein said measuring the methylation level a CpG site for two or more genes comprises a determination selected from the group consisting of determining the methylation score of said CpG site and determining the methylation frequency of said CpG site.

6. The method according to claim 2, wherein the biological sample is a plasma sample

7. The method of claim 6, wherein said CpG site is present in a coding region or a regulatory region.

8. The method of claim 6, wherein said measuring the methylation level a CpG site for the genes comprises a determination selected from the group consisting of determining the methylation score of said CpG site and determining the methylation frequency of said CpG site.

## Patentansprüche

1. Verfahren zum Charakterisieren einer biologischen Probe, umfassend:
Messen eines Methylierungsgrads einer CpG-Stelle für das Gen HOXA1 und eines oder mehrerer Gene, die ausgewählt sind aus entweder
EMX1, DAB2IP, TSPYL5, ACP1, BDH1, Chr12.133, CLEC11A, DBNL, EFNB2, LRRC4, SPINT2, CCNJ_3707, CCNJ_3124, PFKP, SCRN1 und ECE1 in einer biologischen Probe eines menschlichen Individuums durch
Behandeln von genomischer DNA in der biologischen Probe mit Bisulfit;
Amplifizieren der bisulfitbehandelten genomischen DNA unter Verwendung eines Satzes von Primern für die Gene; und
Bestimmen des Methylierungsgrads der CpG-Stelle durch methylierungsspezifische PCR, quantitative methylierungsspezifische PCR, methylierungsempfindliche DNA-Restriktionsenzymanalyse, quantitative Bisulfit-Pyrosequenzierung oder Bisulfit-genomische Sequenzierungs-PCR und Vergleichen des Methylierungsgrads mit einem Methylierungsgrad eines entsprechenden Satzes von Genen in Kontrollproben ohne HCC; und
Bestimmen, dass das Individuum HCC aufweist, wenn der Methylierungsgrad, der in den Genen gemessen wird, höher als der Methylierungsgrad ist, der in den jeweiligen Kontrollproben gemessen wird.

2. Verfahren nach Anspruch 1, wobei der folgende Satz von Primern für die Gene verwendet wird, falls die biologische Probe ein Gewebe oder eine Plasmaprobe ist:
für EX1 ein Satz von Primern, bestehend aus SEQ ID NOS: 16 und 17 oder SEQ ID NOS: 91 und 92,
für DAB2IP ein Satz von Primern, bestehend aus SEQ ID NOS: 4 und 5 oder SEQ ID NOS: 46 und 47,
für TSPYL5 ein Satz von Primern, bestehend aus SEQ ID NOS: 1 und 2 oder SEQ ID NOS: 43 und 44,
für HOXA1 ein Satz von Primern, bestehend aus SEQ ID NOS: 28 und 29 oder SEQ ID NOS: 88 und 89,
für ACP1 ein Satz von Primern, bestehend aus SEQ ID NOS: 10 und 11 oder SEQ ID NOS: 58 und 59,
für BDH1 ein Satz von Primern, bestehend aus SEQ ID NOS: 13 und 14 oder SEQ ID NOS: 61 und 62,
für Chr12.133 ein Satz von Primern, bestehend aus SEQ ID NOS: 25 und 26 oder SEQ ID NOS: 49 und 50,
für CLEC11A ein Satz von Primern, bestehend aus SEQ ID NOS: 7 und 8 oder SEQ ID NOS: 52 und 53,
für DBNL einen Satz von Primern, bestehend aus SEQ ID NOS: 34 und 35 oder SEQ ID NOS: 82 und 83,
für EFNB2 ein Satz von Primern, bestehend aus SEQ ID NOS: 37 und 38 oder SEQ ID NOS: 64 und 65,
für LRRC4 ein Satz von Primern, bestehend aus SEQ ID NOS: 40 und 41 oder SEQ ID NOS: 85 und 86,
für SPINT2 ein Satz von Primern, bestehend aus SEQ ID NOS: 31 und 32 oder SEQ ID NOS: 55 und 56,
für CCNJ_3707 einen Satz von Primern, bestehend aus SEQ ID NOS: 67 und 68,
für CCNJ_3124 ein Satz von Primern, bestehend aus SEQ ID NOS: 70 und 71,
für PFKP einen Satz von Primern, bestehend aus SEQ ID NOS: 73 und 74,
für SCRN1 ein Satz von Primern, bestehend aus SEQ ID NOS: 76 und 77, und
für ECE1 ein Satz von Primern, bestehend aus SEQ ID NOS: 79 und 80.

3. Verfahren nach Anspruch 1, wobei die biologische Probe eine Plasmaprobe oder eine Gewebeprobe ist.

4. Verfahren nach Anspruch 1, wobei die CpG-Stelle in einer Codierungsregion oder einer regulatorischen Region vorhanden ist.

5. Verfahren nach Anspruch 1, wobei das Messen des Methylierungsgrads eine CpG-Stelle für zwei oder mehr Gene eine Bestimmung umfasst, die aus der Gruppe ausgewählt ist, bestehend aus Bestimmen des Methylierungswerts der CpG-Stelle, und Bestimmen der Methylierungsfrequenz der CpG-Stelle.

6. Verfahren nach Anspruch 2, wobei die biologische Probe eine Plasmaprobe ist

7. Verfahren nach Anspruch 6, wobei die CpG-Stelle in einer Codierungsregion oder einer regulatorischen Region vorhanden ist.

8. Verfahren nach Anspruch 6, wobei das Messen des Methylierungsgrads eine CpG-Stelle für die Gene eine Bestimmung umfasst, die aus der Gruppe ausgewählt ist, bestehend aus Bestimmen des Methylierungswerts der CpG-Stelle, und Bestimmen der Methylierungsfrequenz der CpG-Stelle.

## Revendications

1. Procédé de caractérisation d'un échantillon biologique comprenant :
la mesure d'un niveau de méthylation d'un site CpG pour le gène HOXA1 et un ou plusieurs gènes choisis parmi soit
EMX1, DAB2IP, TSPYL5, ACP1, BDH1, Chr12.133, CLEC11A, DBNL, EFNB2, LRRC4, SPINT2, CCNJ_3707, CCNJ_3124, PFKP, SCRN1 et ECE1 dans un échantillon biologique d'un individu humain par
le traitement de l'ADN génomique dans l'échantillon biologique avec du bisulfite ;
l'amplification de l'ADN génomique traité au bisulfite à l'aide d'un ensemble d'amorces pour les gènes ; et
la détermination du niveau de méthylation du site CpG par ACP spécifique à la méthylation, ACP quantitative en méthylation, analyse enzymatique de restriction de l'ADN sensible à la méthylation, pyroséquençage quantitatif au bisulfite ou ACP de séquençage génomique au bisulfite, et la comparaison du niveau de méthylation à un niveau de méthylation d'un ensemble correspondant de gènes dans des échantillons témoins sans HCC ; et
la détermination que l'individu a HCC lorsque le niveau de méthylation mesuré dans les gènes est supérieur au niveau de méthylation mesuré dans les échantillons témoins respectifs.

2. Procédé selon la revendication 1, dans lequel l'ensemble d'amorces suivantes pour les gènes sont utilisés si l'échantillon biologique est un échantillon de tissu ou de plasma :
pour EMX1, un ensemble d'amorces constitué de SEQ ID N° : 16 et 17 ou SEQ ID N° : 91 et 92,
pour DAB2IP, un ensemble d'amorces constitué de SEQ ID N° : 4 et 5 ou SEQ ID N° : 46 et 47,
pour TSPYL5, un ensemble d'amorces constitué de SEQ ID N° : 1 et 2 ou SEQ ID N° : 43 et 44,
pour HOXA1, un ensemble d'amorces constitué de SEQ ID N° : 28 et 29 ou SEQ ID N° : 88 et 89,
pour ACP1, un ensemble d'amorces constitué de SEQ ID N° : 10 et 11 ou SEQ ID N° : 58 et 59,
pour BDH1, un ensemble d'amorces constitué de SEQ ID N° : 13 et 14 ou SEQ ID N° : 61 et 62,
pour Chr12.133, un ensemble d'amorces constitué de SEQ ID N° : 25 et 26 ou SEQ ID N° : 49 et 50,
pour CLEC11A, un ensemble d'amorces constitué de SEQ ID N° : 7 et 8 ou SEQ ID N° : 52 et 53,
pour DBNL un ensemble d'amorces constitué de SEQ ID N° : 34 et 35 ou SEQ ID N° : 82 et 83,
pour EFNB2, un ensemble d'amorces constitué de SEQ ID N° : 37 et 38 ou SEQ ID N° : 64 et 65,
pour LRRC4, un ensemble d'amorces constitué de SEQ ID N° : 40 et 41 est SEQ ID N° : 85 et 86,
pour SPINT2, un ensemble d'amorces constitué de SEQ ID N° : 31 et 32 est SEQ ID N° : 55 et 56,
pour CCNJ_3707, un ensemble d'amorces constitué de SEQ ID N° : 67 et 68,
pour CCNJ_3124, un ensemble d'amorces constitué de SEQ ID N° : 70 et 71,
pour PFKP un ensemble d'amorces constitué de SEQ ID N° : 73 et 74,
pour SCRN1, un ensemble d'amorces constitué de SEQ ID N° : 76 et 77, et
pour ECE1, un ensemble d'amorces constitué de SEQ ID N° : 79 et 80.

3. Procédé selon la revendication 1, dans lequel l'échantillon biologique est un échantillon de plasma ou un échantillon de tissu.

4. Procédé selon la revendication 1, dans lequel ledit site CpG est présent dans une région codante ou une région régulatrice.

5. Procédé selon la revendication 1, dans lequel ladite mesure du niveau de méthylation d'un site CpG pour deux gènes ou plus comprend une détermination choisie dans le groupe consistant en la détermination du score de méthylation dudit site CpG et la détermination de la fréquence de méthylation dudit site CpG.

6. Procédé selon la revendication 2, dans lequel l'échantillon biologique est un échantillon de plasma.

7. Procédé selon la revendication 6, dans lequel ledit site CpG est présent dans une région codante ou une région régulatrice.

8. Procédé selon la revendication 6, dans lequel ladite mesure de la méthylation au niveau d'un site CpG pour les gènes comprend une détermination choisie dans le groupe consistant en la détermination du score de méthylation dudit site CpG et la détermination de la fréquence de méthylation dudit site CpG.
